(19) Europäisches Patentamt
European Patent Office
Office européen des brevets

(11) **EP 2 939 694 B1**

(12) **EUROPEAN PATENT SPECIFICATION**

(45) Date of publication and mention
of the grant of the patent:
**14.07.2021 Bulletin 2021/28**

(51) Int Cl.:
***A61K 47/02*** *(2006.01)*    ***A61K 9/16*** *(2006.01)*
***A61M 1/14*** *(2006.01)*

(21) Application number: **13867925.3**

(22) Date of filing: **26.12.2013**

(86) International application number:
**PCT/JP2013/084967**

(87) International publication number:
**WO 2014/104230 (03.07.2014 Gazette 2014/27)**

(54) **DIALYSIS AGENT, AND METHOD FOR PRODUCING DIALYSIS AGENT**

DIALYSEMITTEL UND VERFAHREN ZUR HERSTELLUNG DES DIALYSEMITTELS

AGENT DE DIALYSE, ET PROCÉDÉ DE PRODUCTION D'AGENT DE DIALYSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2012 JP 2012284548
27.12.2012 JP 2012284549
27.12.2012 JP 2012284550
17.12.2013 JP 2013259796
17.12.2013 JP 2013259797
17.12.2013 JP 2013259798**

(43) Date of publication of application:
**04.11.2015 Bulletin 2015/45**

(73) Proprietor: **Nikkiso Co., Ltd.
Tokyo 150-6022 (JP)**

(72) Inventors:
• **HARUTA, Yoshihisa
Kanazawa-shi
Ishikawa 920-0177 (JP)**

• **TSUNEMOTO, Tetsuya
Kanazawa-shi
Ishikawa 920-0177 (JP)**
• **KOMATSU, Daisuke
Kanazawa-shi
Ishikawa 920-0177 (JP)**

(74) Representative: **Klunker IP
Patentanwälte PartG mbB
Destouchesstraße 68
80796 München (DE)**

(56) References cited:
**WO-A1-2013/084922       JP-A- H1 087 478
JP-A- 2001 340 448       JP-A- 2010 029 654
JP-A- 2013 048 894       JP-A- 2013 048 895
US-A1- 2002 061 334       US-A1- 2004 060 865**

• **None**

Remarks:
The file contains technical information submitted after
the application was filed and not included in this
specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a method for providing the dialysis agent.

BACKGROUND ART

Dialysis agents are chemical agents used for preparing dialysis fluids. Dialysis fluids are used in hemodialysis, hemofiltration and peritoneal dialysis and the like to replace the function usually performed by the kidneys of removing waste products from body fluids, and are also used in some cases to supplement the blood with required components. These dialysis fluids are aqueous solutions having an electrolyte composition similar to body fluids. Dialysis fluids that use sodium hydrogen carbonate (sodium bicarbonate) as an alkaline component are preferable from a physiological perspective, and are in widespread use. The dialysis agents used for obtaining these types of dialysis fluids typically include sodium bicarbonate, as well as anhydrous glucose as a carbohydrate component, sodium chloride, potassium chloride, calcium chloride hydrate and magnesium chloride hydrate and the like as electrolytes, and an organic acid or organic acid salt or the like as a pH modifying component. Dialysis agents have conventionally been liquids, but liquids occupy a large volume, are heavy, and are inconvenient in terms of handling during storage, transport or use, and therefore there has been a shift to solid agents in recent years.

Current dialysis agents are generally two-part compositions composed of an "A agent" comprising electrolytes containing calcium ions and magnesium ions and the like, anhydrous glucose and a pH modifying component, and a "B agent" comprising sodium bicarbonate. The ranges currently used for the electrolyte concentration and the concentration of the anhydrous glucose are, for example, as shown below (a pH modifying component is also included).

$Na^+$: 103.0 to 143.0 mEq/L
$K^+$: 1.0 to 4.0 mEq/L
$Ca^{++}$: 1.25 to 3.50 mEq/L
$Mg^{++}$: 0.25 to 1.50 mEq/L
$Cl^-$: 107.0 to 115.5 mEq/L
anhydrous glucose: 0 to 250 mg/dL

One technique used for preparing a solid dialysis agent is granulation, and examples of the method used for granulating the A agent include mainly a dry granulation method, an extrusion granulation method, a rolling agitation fluidized bed granulation method and an agitation granulation method.

The dry granulation method is a granulation method in which, for example, various electrolyte compounds such as sodium chloride, potassium chloride, calcium chloride hydrate, magnesium chloride hydrate, and an organic acid salt such as sodium acetate are mixed, compressed, crushed and then granulated inside a dry granulator to obtain the A agent.

The extrusion granulation method is a granulation method in which, for example, an aqueous solution of various electrolytes other than sodium chloride is added and kneaded into sodium chloride powder, and the resulting granulated mixture is forced through the screen surface of an extrusion granulator to obtain granules of the A agent.

The rolling agitation fluidized bed granulation method is a granulation method in which, for example, sodium chloride is subjected to rolling and fluidization inside a rolling agitation fluidized bed granulator, and an aqueous solution of a mixture containing potassium chloride, calcium chloride hydrate, magnesium chloride hydrate, and an organic acid salt such as sodium acetate is sprayed onto the rolling fluidized bed of sodium chloride, thereby covering the surfaces of the rolling fluidized particles of sodium chloride substantially uniformly with microparticles of the mixture containing potassium chloride, calcium chloride hydrate, magnesium chloride hydrate and the organic acid salt such as sodium acetate to obtain the A agent.

The agitation granulation method is a granulation method in which, for example, a suspension of potassium chloride, calcium chloride hydrate and magnesium chloride hydrate is added to and mixed with sodium chloride inside an agitation granulator, an organic acid salt such as sodium acetate is mixed into the resulting mixture, and the thus obtained mixture is then dried to obtain the A agent.

However, forming the agent into granules results in more complex unit operations, leading to a loss in productivity, and an increase in unit operations increases the chances of foreign matter contamination. Further, forming the agent into granules means that production conditions must be established for each variety of agent when the variety is altered (alteration of the concentration of each component). Moreover, when the variety of agent is altered (alteration of the concentration of each component), raw material remaining in the production equipment must be removed, resulting in product loss and time loss, and causing a deterioration in the productivity.

On the other hand, examples of methods that have been investigated for providing a dialysis agent without forming the agent into granules include the following methods. For example, Patent Document 1 discloses a single pack chemical agent used in the preparation of a dialysis fluid in which in order to prevent reactions such as the precipitation of carbonate salts as a result of a chemical reaction between sodium bicarbonate and calcium chloride hydrate or magnesium chloride hydrate, a layer of chemically stable sodium chloride is used as a buffer layer between materials which would undergo a chemical reaction if brought into direct contact.

Patent Document 2 discloses a powder dialysis agent containing glucose and sodium bicarbonate, in which by drying the entire powder dialysis agent, or at least one component among the glucose, sodium chloride and sodium bicarbonate, coloration during storage can be suppressed, and the agent can be stored stably for long periods.

Patent Document 3 discloses a dialysis acid precursor composition for use during preparation of a dialysis acid concentrate solution and for mixing with water and a bicarbonate-containing concentrate to form a ready-for-use dialysis fluid, wherein the dialysis acid precursor composition is composed of powder components comprising sodium chloride, at least one dry acid and at least one magnesium salt, and optionally comprising a potassium salt, a calcium salt and glucose, the at least one magnesium salt and the optional glucose exist as anhydrous components in the dialysis acid precursor composition, and the dialysis acid precursor composition is sealed in a moisture-resistant container with a water vapor transmission rate of less than 0.3 $g/m^2/day$ at 38°C/90% RH.

In the method of Patent Document 1, because the chemical agent is placed in a container with no particular countermeasure being taken against the water having a high degree of freedom, consolidation or aggregation of the chemical agent tends to occur, meaning rapid dissolution of the agent becomes difficult.

When the entire powder dialysis agent is dried, as in the method disclosed in Patent Document 2, achieving drying without degradation of the glucose requires that the drying is conducted over a long period in a large apparatus by reduced-pressure drying (for example, drying conditions including vacuum drying at about 25°C for about 165 hours) or freeze drying (for example, drying conditions including preliminary freezing at about -45°C for about 12 hours, primary drying at about 0°C for about 72 hours, and then secondary drying at about 25°C for about 12 hours), and therefore the productivity is poor and the method is impractical. Moreover, under the type of low-temperature drying conditions described above, the water of crystallization of magnesium chloride hexahydrate cannot be dried. Further, when the glucose, sodium chloride and sodium bicarbonate that represent the main components of the dialysis agent are dried, the weight of components requiring drying within a single formulation is much higher than when only the minor component magnesium chloride hydrate is dried, and therefore the productivity is poor and the method is impractical.

In the composition of Patent Document 3, anhydrous magnesium chloride is used as an anhydrous magnesium salt, but anhydrous magnesium chloride cannot be produced simply be drying by heating, and providing a dialysis agent using anhydrous magnesium chloride is problematic from a cost perspective. Further, anhydrous magnesium chloride has a rapid water absorption rate, meaning transporting and storing a large amount of anhydrous magnesium chloride while maintaining the water content is difficult. Moreover, if a magnesium chloride hydrate with a stable water content cannot be maintained, then the precision of the magnesium content of the dialysis agent becomes unstable. Patent Document 4 discloses a method for the production of a dialysis agent by granulation followed by mixing different granules containing sodium chloride, potassium chloride, calcium chloride, magnesium chloride, and sodium acetate or sodium citrate as the components.

PRIOR ART DOCUMENTS

PATENT DOCUMENTS

Patent Document 1: JP H08-080345 A
Patent Document 2: JP 2001-340448 A
Patent Document 3: WO 2011/161055 pamphlet Patent Document 4: JP2010029654 A.

SUMMARY OF THE INVENTION

PROBLEMS TO BE SOLVED BY THE INVENTION

It is an object of the present invention to provide a method according to claim 1 for providing a dialysis agent which comprises at least magnesium chloride hydrate as electrolytes and in which each of the constituent components is placed in a container as a single solid component, the method enabling a dialysis agent for which consolidation and aggregation of the chemical agent can be suppressed to be obtained at low cost.

Moreover, yet another object of the present disclosure is to provide a method for providing a dialysis agent which comprises at least magnesium chloride hydrate and calcium chloride hydrate as electrolytes and in which each of the constituent components is placed in a container as a single solid component, the method enabling a dialysis agent for which consolidation and aggregation of the chemical agent can be suppressed to be obtained at low cost.

MEANS FOR SOLVING THE PROBLEMS

The present invention is defined in the appended claims. Described but not part of the present invention is a dialysis agent comprising electrolytes containing at least magnesium chloride hydrate, and a pH modifier, wherein the ratio between the water content of the entire dialysis agent and the amount of water removed from the magnesium chloride hydrate is less than 118% but at least 7%, the water content of the magnesium chloride hydrate is within a range from 43% to 51%, and each of the constituent components is placed in a container as a single solid component.

Further, in the above dialysis agent, the ratio between the water content of the entire dialysis agent and the amount of water removed from the magnesium chloride hydrate is preferably less than 97% but at least 7%.

Furthermore, in the above dialysis agent, the water content of the magnesium chloride hydrate is preferably within a range from 43% to 45%.

Further described but not part of the present invention is a dialysis agent comprising electrolytes containing at least magnesium chloride hydrate and calcium chloride hydrate, and a pH modifier, wherein the ratio between the water content of the entire dialysis agent and the sum of the amount of water removed from the magnesium chloride hydrate and the amount of water removed from the calcium chloride hydrate is not more than 210% but at least 2%, the water content of the magnesium chloride hydrate is within a range from 43% to 51%, the water content of the calcium chloride hydrate is within a range from 1% to 22%, and each of the constituent components is placed in a container as a single solid component.

Further, in the above dialysis agent, the ratio between the water content of the entire dialysis agent and the sum of the amount of water removed from the magnesium chloride hydrate and the amount of water removed from the calcium chloride hydrate is preferably less than 167% but at least 2%.

Furthermore, in the above dialysis agent, the water content of the magnesium chloride hydrate is preferably within a range from 43% to 45%, the water content of the calcium chloride hydrate is preferably within a range from 14% to 22%, and the water content of the calcium chloride hydrate is more preferably within a range from 14% to 16%.

Furthermore, in the above dialysis agents, the pH modifier is preferably at least one of an acetic acid mixture composed of a mixture of sodium acetate and glacial acetic acid, and a combination of this acetic acid mixture and sodium acetate.

Moreover, in the above dialysis agent, the molar ratio between sodium acetate and glacial acetic acid in the acetic acid mixture is preferably within a range from 1:1 to 5:1, and the molar ratio is more preferably within a range from 3:1 to 4:1.

The present invention provides a method for providing a dialysis agent comprising electrolytes containing at least magnesium chloride hydrate, and a pH modifier, the method comprising a drying treatment step of drying the magnesium chloride hydrate so that the ratio between the water content of the entire dialysis agent and the amount of water removed from the magnesium chloride hydrate becomes less than 118% but at least 7%, and the water content of the magnesium chloride hydrate becomes a value within a range from 43% to 51%, and a filling step of placing each of the constituent components in a container as a single solid component.

Further, in the drying treatment step of the method for providing a dialysis agent described above, the magnesium chloride hydrate is preferably dried so that that the ratio between the water content of the entire dialysis agent and the amount of water removed from the magnesium chloride hydrate becomes less than 97% but at least 7%.

Furthermore, in the drying treatment step of the method for providing a dialysis agent described above, the magnesium chloride hydrate is preferably dried so that the water content of the magnesium chloride hydrate becomes a value within a range from 43% to 45%.

Further described but not part of the present invention is a method for providing a dialysis agent comprising electrolytes containing at least magnesium chloride hydrate and calcium chloride hydrate, and a pH modifier, the method comprising a drying treatment step of drying the magnesium chloride hydrate and the calcium chloride hydrate so that the ratio between the water content of the entire dialysis agent and the sum of the amount of water removed from the magnesium chloride hydrate and the amount of water removed from the calcium chloride hydrate becomes not more than 210% but at least 2%, the water content of the magnesium chloride hydrate becomes a value within a range from 43% to 51%, and the water content of the calcium chloride hydrate becomes a value within a range from 1% to 22%, and a filling step of placing each of the constituent components in a container as a single solid component.

Further, in the drying treatment step of the method for providing a dialysis agent described above, the magnesium chloride hydrate and the calcium chloride hydrate are preferably dried so that the ratio between the water content of the entire dialysis agent and the sum of the amount of water removed from the magnesium chloride hydrate and the amount of water removed from the calcium chloride hydrate becomes less than 167% but at least 2%.

Furthermore, in the drying treatment step of the method for providing a dialysis agent described above, the magnesium chloride hydrate and the calcium chloride hydrate are preferably dried so that the water content of the magnesium chloride hydrate becomes a value within a range from 43% to 45%, and the water content of the calcium chloride hydrate becomes a value within a range from 14% to 22%, and the magnesium chloride hydrate and the calcium chloride hydrate are more preferably dried so that the water content of the calcium chloride hydrate becomes a value within a range from 14% to 16%.

Furthermore, in the above methods for providing dialysis agents, the pH modifier is preferably at least one of an acetic acid mixture composed of a mixture of sodium acetate and glacial acetic acid, and a combination of this acetic acid mixture and sodium acetate.

Moreover, in the above method for providing a dialysis agent, the molar ratio between sodium acetate and glacial acetic acid in the acetic acid mixture is preferably within a range from 1:1 to 5:1, and the molar ratio is more preferably within a range from 3:1 to 4:1.

EFFECTS OF THE INVENTION

In the dialysis agent comprising at least magnesium chloride hydrate as electrolytes, and a pH modifier, wherein each of the constituent components is placed in a container as a single solid component, by ensuring that the ratio between the water content of the entire dialysis agent and the amount of water removed from the magnesium chloride hydrate is less than 118% but at least 7%, and the water content of the magnesium chloride hydrate is within a range from 43% to 51%, a dialysis agent is provided which can be obtained at low cost, and in which consolidation and aggregation of the chemical agent can be suppressed.

In the dialysis agent comprising at least magnesium chloride hydrate and calcium chloride hydrate as electrolytes, and a pH modifier, wherein each of the constituent components is placed in a container as a single solid component, by ensuring that the ratio between the water content of the entire dialysis agent and the sum of the amount of water removed from the magnesium chloride hydrate and the amount of water removed from the calcium chloride hydrate is not more than 210% but at least 2%, the water content of the magnesium chloride hydrate is within a range from 43% to 51%, and the water content of the calcium chloride hydrate is within a range from 1% to 22%, a dialysis agent is provided which can be obtained at low cost, and in which consolidation and aggregation of the chemical agent can be suppressed.

Furthermore, in the present invention, in the method for providing a dialysis agent comprising at least magnesium chloride hydrate as electrolytes, and a pH modifier, wherein each of the constituent components is placed in a container as a single solid component, by drying the magnesium chloride hydrate so that the ratio between the water content of the entire dialysis agent and the amount of water removed from the magnesium chloride hydrate becomes less than 118% but at least 7%, and the water content of the magnesium chloride hydrate becomes a value within a range from 43% to 51%, a dialysis agent which can suppress consolidation and aggregation of the chemical agent can be obtained at low cost.

In the method for providing a dialysis agent comprising at least magnesium chloride hydrate and calcium chloride hydrate as electrolytes, and a pH modifier, wherein each of the constituent components is placed in a container as a single solid component, by drying the magnesium chloride hydrate and the calcium chloride hydrate so that the ratio between the water content of the entire dialysis agent and the sum of the amount of water removed from the magnesium chloride hydrate and the amount of water removed from the calcium chloride hydrate becomes not more than 210% but at least 2%, the water content of the magnesium chloride hydrate becomes a value within a range from 43% to 51%, and the water content of the calcium chloride hydrate becomes a value within a range from 1% to 22%, a dialysis agent which can suppress consolidation and aggregation of the chemical agent can be obtained at low cost.

BRIEF DESCRIPTION OF THE DRAWINGS

FIG. 1 is a diagram illustrating the relationship between a ratio M and the consolidation rate in the examples.
FIG. 2 is a diagram illustrating the relationship between the ratio M and the 5-HMF absorbance in the examples.
FIG. 3 is a diagram illustrating the relationship between a ratio CM and the consolidation rate in the examples.
FIG. 4 is a diagram illustrating the relationship between the ratio CM and the 5-HMF absorbance in the examples.
FIG. 5 is a diagram illustrating the relationship between a ratio C and the consolidation rate in the examples.
FIG. 6 is a diagram illustrating the relationship between the ratio C and the 5-HMF absorbance in the examples.
FIG. 7 is a diagram illustrating the change in water content when magnesium chloride hexahydrate is dried at 100°C.
FIG. 8 is a diagram illustrating the change in water content when calcium chloride dihydrate is dried at 100°C.

EMBODIMENTS FOR CARRYING OUT THE INVENTION

Embodiments of the present invention are described below. These embodiments are merely examples of implementing the present invention, and the present invention is in no way limited by these embodiments. Herein, the first embodiment does not fall under the scope of the present invention.

<First Embodiment of Dialysis Agent>

A dialysis agent according to a first embodiment is a dialysis agent comprising electrolytes containing at least magnesium chloride hydrate, and a pH modifier, wherein each of the constituent components is placed in a container as a single solid component. In the dialysis agent according to the first embodiment, the ratio between the water content of the entire dialysis agent and the amount of water removed from the magnesium chloride hydrate is less than 118% but at least 7%, and the water content of the magnesium chloride hydrate is within a range from 43% to 51%. In the present description, the expression "each of the constituent components is placed in a container as a single solid component" means that the plurality of components contained within the dialysis agent are not formed together as granules, and includes not only the configuration in which each component is placed in the container as a "single crystalline component", but also the configuration in which each component is placed in the container as a "single-component granulated material", and the configuration in which each component is placed in the container as a "single-component ground material". Here, a "single component" refers to a material having a purity of at least 95%, and components which contain not more than about 5% of impurities are also deemed to be single components. Further, in the present description, a "single component" may include the aforementioned acetic acid mixture such as sodium diacetate. Moreover, the expression that "each of the constituent components is placed in a container as a single solid component" also includes the case in which before each "single component" is placed in the container, a plurality of the "single components" are mixed together, such as a production method in which each "single component" is put in a container, the components are mixed together, and a prescribed amount of the resulting mixture is then placed in a container in a single operation.

Consolidation and aggregation, which are one major cause of deterioration in the solubility of dialysis agents, are thought to occur when aggregation, caused when the water having a high degree of freedom inside an airtight container forms liquid bridging to the crystals of each of the components of the dialysis agent, and drying of the resulting aggregates occur repeatedly, causing recrystallization between particles.

Further, it is thought that when a dialysis agent is placed in an airtight container, the water having a high degree of freedom inside the airtight container may act as a catalyst for the reaction between citric acid and sodium chloride (or magnesium chloride hydrate or calcium chloride hydrate), causing the generation of hydrogen chloride. There is a possibility that this generated hydrogen chloride may cause dehydration of the anhydrous glucose, producing 5-hydroxymethylfurfural (5-HMF).

On the other hand, the water contained in the magnesium chloride hexahydrate generally used as the magnesium chloride hydrate is water of crystallization of a deliquescent material. This water of crystallization of magnesium chloride hexahydrate is chemically bonded water which is difficult to remove. As a result, dehydration of water of crystallization requires more energy than dehydration of free water. Accordingly, comprehending how much water needs to be removed from the magnesium chloride hexahydrate or the like can be achieved by increasing the efficiency of, and optimizing, the magnesium chloride hydrate drying step for the dialysis agent prepared by the method for providing a dialysis agent which includes a drying treatment step of drying the magnesium chloride hydrate.

Magnesium chloride hydrate has a water absorption ability, and for example when 32.0 g of magnesium chloride hexahydrate (water content: 53.2%) is dried to prepare a dried magnesium chloride hydrate having a water content of 43%, 5.6 g of water is lost. This lost water can be regarded as the amount of water that the dried magnesium chloride hydrate having a water content of 43% is able to absorb.

In the present description, the water absorption ability of magnesium chloride hydrate is represented by the difference between the initial amount of retained water (g) in the magnesium chloride hydrate ([weight of magnesium chloride hexahydrate] $\times$ [theoretical water content of magnesium chloride hexahydrate (53.2%)]) and the amount of retained water (g) following drying of the magnesium chloride hydrate ([weight of dried magnesium chloride hydrate] $\times$ [measured water content (%) of dried magnesium chloride hydrate]), and is referred to as "the amount of water removed from the magnesium chloride hydrate".

On the other hand, the water contained within each raw material of the dialysis agent may be broadly classified as two types, namely "water of adhesion" and "water of crystallization". Raw materials having water of crystallization include "deliquescent" raw materials which absorb water from the air and spontaneously become aqueous solutions, and "efflorescent" raw materials which release water of crystallization in air. The water of crystallization of efflorescent raw materials and the water of adhesion of raw materials represent water of a high degree of freedom which can be readily released into the air.

In the present description, the sum of the weight of the water of adhesion from raw materials and the weight of the water of crystallization of efflorescent components, which represents the water having a high degree of freedom, is referred to as "the water content of the entire dialysis agent".

First, the total amount of water of adhesion (amount of water of adhesion) is calculated from the measured water content, the theoretical water content, and the weight of each component except for the dried magnesium chloride hydrate.

$$\text{Measured water content of each component (\%)} -$$

$$\text{theoretical water content of each component (\%)} = \text{water}$$

$$\text{content due to water of adhesion of each component (\%)}$$

$$\text{Weight of each component (g)} \times \text{water content due to}$$

$$\text{water of adhesion of each component (\%)} = \text{amount of water of}$$

$$\text{adhesion for each component (g)}$$

$$\text{Total of amounts of water of adhesion for all components}$$

$$\text{(g)} = \text{total amount of water of adhesion (amount of water of}$$

$$\text{adhesion) (g).}$$

Alternatively, the amount of water adhesion may also be determined as follows:

$$\text{Amount of water of adhesion (g)} = \text{(total weight of all}$$

$$\text{components (g)} - \text{weight of dried magnesium chloride hydrate}$$

$$\text{(g))} \times \text{water content of total amount of water of adhesion}$$

$$\text{(\%).}$$

Then, the weight of the water of crystallization (amount of water of crystallization) within the efflorescent components is calculated from the theoretical water content of the efflorescent components and the weight of the efflorescent components.

$$\text{Amount of water of crystallization (g)} = \text{weight of}$$

$$\text{efflorescent components (g)} \times \text{theoretical water content of}$$

$$\text{efflorescent components (\%)}$$

The "water content of the entire dialysis agent" is determined from the sum of the "amount of water of adhesion" within the raw materials and the "weight of water of crystallization within the efflorescent components (amount of water of crystallization)" calculated in the manner described above. The ratio between the "water content of the entire dialysis agent" and the "amount of water removed from the magnesium chloride hydrate" is referred to as M.

$$\text{Water content of entire dialysis agent (g)} = \text{amount of}$$

$$\text{water of adhesion (g)} + \text{amount of water of crystallization}$$

$$\text{(g)}$$

$$M\ (\%) = [(\text{water content of entire dialysis agent (g)}) /$$

$$(\text{amount of water removed from magnesium chloride hydrate}$$

$$(\text{g}))] \times 100$$

The inventors of the present invention discovered that provided M, which represents the ratio between the "water content of the entire dialysis agent" and the "amount of water removed from the magnesium chloride hydrate" is not more than a prescribed value, consolidation and aggregation could be suppressed for a dialysis agent in which not granules, but rather single solid components of each of the various constituent components are placed in a container. Further, they also discovered that provided M, which represents the ratio between the "water content of the entire dialysis agent" and the "amount of water removed from the magnesium chloride hydrate" is not more than the prescribed value, the amount of water having a high degree of freedom inside an airtight container can be reduced, generation of hydrogen chloride can be suppressed, and the production of 5-HMF can be inhibited. In other words, the magnesium chloride hydrate, which is a component generally included in a small amount among the raw materials included in the dialysis agent, is subjected to a drying treatment to remove not only the water of adhesion, but also some of the water of crystallization, thus imparting the dried magnesium chloride hydrate with a function as a "desiccant". In the present invention, in the dialysis agent in which each of the constituent components is placed in the container as a single solid component, the discovery of this relationship between the parameter represented by the ratio M between the "water content of the entire dialysis agent" and the "amount of water removed from the magnesium chloride hydrate", and suppression of consolidation and aggregation has great significance.

The ratio M between the "water content of the entire dialysis agent" and the "amount of water removed from the magnesium chloride hydrate" is less than 118%, but is preferably 115% or lower. Further, the ratio M between the "water content of the entire dialysis agent" and the "amount of water removed from the magnesium chloride hydrate" is more preferably less than 97%, and still more preferably 93% or lower. If the ratio M between the "water content of the entire dialysis agent" and the "amount of water removed from the magnesium chloride hydrate" is less than 97%, then even in those cases when the dialysis agent contains anhydrous glucose as a carbohydrate component, degradation of the anhydrous glucose can be inhibited. If consideration is given to moisture and the like entering from outside the airtight container, then the ratio M between the "water content of the entire dialysis agent" and the "amount of water removed from the magnesium chloride hydrate" is still more preferably 75% or lower, and in terms of long-term storage and the like, is more preferably 60% or lower, still more preferably 50% or lower, and most preferably 40% or lower. The ratio M is preferably as low as possible, but if the ratio M is too low, then further reduction in the ratio M may sometimes require excessive drying treatment of the magnesium chloride hydrate, and therefore in order to achieve satisfactory results with an appropriate drying treatment, the ratio M is typically at least 7%, preferably at least 15%, and more preferably 20% or higher.

It is thought that provided the ratio M is less than 118%, water having a high degree of freedom, which can cause consolidation and aggregation of the dialysis agent, can be absorbed by the dried magnesium chloride hydrate, thereby suppressing consolidation and aggregation of the dialysis agent. It is thought that if the ratio M is less than 97%, then water having a high degree of freedom, which can cause degradation of anhydrous glucose, can adequately be absorbed by the dried magnesium chloride hydrate, meaning even in those cases when the dialysis agent contains anhydrous glucose as a carbohydrate component, degradation of the anhydrous glucose can be inhibited. The amount of water which must be removed from the magnesium chloride hydrate to suppress consolidation and aggregation of the dialysis agent and degradation of the anhydrous glucose may be restricted to the minimum amount required for each formulation (namely, the amounts of the various components in the A agent), and therefore the drying step can be made more efficient. Further, compared with a method for providing a dialysis agent in which the components are formed into granules, the unit operations can be shortened and simplified, thereby improving the productivity and reducing the chances of foreign matter contamination. Further, the establishment of production conditions when the variety of agent is altered (alteration of the concentration of each component) can be easier. Moreover, raw material remaining in the production equipment need not be removed when the variety of agent is altered (alteration of the concentration of each component), thus improving productivity. Accordingly, a dialysis agent of low cost and high quality can be achieved.

Further, it is thought that including, inside the airtight container, dried magnesium chloride hydrate that absorbs the water having a high degree of freedom inside the airtight container enables the suppression of consolidation, which occurs upon repeated aggregation, caused when the water having a high degree of freedom inside the airtight container forms liquid bridging to the crystals of each of the components, and subsequent drying of the resulting aggregates, resulting in recrystallization between particles.

Furthermore, it is thought that by including, inside the airtight container, sufficient dried magnesium chloride hydrate to absorb the water having a high degree of freedom inside the airtight container and any moisture entering from outside the airtight container, the water having a high degree of freedom that can cause degradation of the anhydrous glucose is absorbed by the dried magnesium chloride hydrate, meaning degradation of the anhydrous glucose can be inhibited.

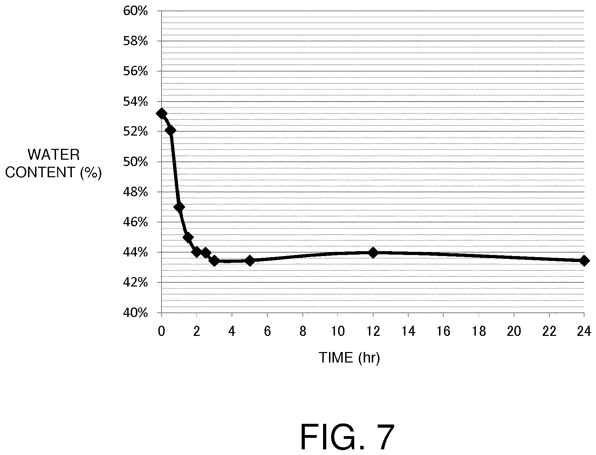

In the dialysis agent according to the first embodiment, the water content of the magnesium chloride hydrate is within a range from 43% to 51%, and is preferably within a range from 43% to 45%. The "water content" indicates the proportion (mass %) of water contained in the substance. By ensuring that the water content of the magnesium chloride hydrate is within a range from 43% to 51%, the production process can be simplified, the productivity can improve, and the problem of foreign matter contamination can be minimized. For example, when magnesium chloride hexahydrate is dried in an atmosphere at 100°C, the dehydration rate moderates when the water content of the magnesium chloride hydrate reaches a range from 43% to 45%, and the dehydration essentially stops proceeding at about 43% (see FIG. 7). Accordingly, magnesium chloride hydrate having a water content within a range from 43% to 45% can offer the advantage that the water content can be readily controlled. Further, by using magnesium chloride hydrate having a water content within the range from 43% to 45%, the dialysis agent can be produced using a magnesium chloride hydrate for which the water content is stable, which can offer another advantage in that the precision of the magnesium content of the dialysis agent can be stabilized. By using a magnesium chloride hydrate prepared by drying magnesium chloride hexahydrate until the water content is within a range from 43% to 45%, handling can be easier and the costs can be reduced compared with the case where an anhydrous magnesium chloride having a faster water absorption rate is used.

In this manner, by using the method of the present invention for providing a dialysis agent according to the first embodiment, a problem that occurs as a result of forming the agent into granules, wherein complex production conditions must be established when the variety of agent is altered (alteration of the concentration of each component), can be resolved. Further, a problem that occurs when forming the agent into granules results in more complex unit operations, leading to a loss in productivity, and the resulting increase in unit operations increases the chances of foreign matter contamination, can also be resolved. Furthermore, another problem that occurs as a result of forming the agent into granules, wherein establishment of the production conditions upon alteration of the variety of agent (alteration of the concentration of each component) becomes difficult, can also be resolved. Moreover, another problem that occurs when the variety of agent is altered (alteration of the concentration of each component), wherein the raw material remaining in the production equipment must be removed, resulting in product loss and time loss, and causing a deterioration in the productivity, cab also be resolved. Accordingly, a dialysis agent of low cost and high quality can be achieved.

In the dialysis agent according to the first embodiment, the magnesium chloride hydrate has preferably been subjected to a drying treatment, and the magnesium chloride hydrate has more preferably been subjected to a heated drying treatment in an atmosphere of 75°C or higher. Provided the atmosphere in the heated drying treatment of the magnesium chloride hydrate is at least 75°C, the water content within the magnesium chloride hydrate can be readily removed, making it easier to achieve a ratio between the water content of the entire dialysis agent and the amount of water removed from the magnesium chloride hydrate of less than 118% but at least 7%, and a water content for the magnesium chloride hydrate within a range from 43% to 51%.

The dialysis agent according to the first embodiment preferably contains a carbohydrate component. An example of the carbohydrate component is anhydrous glucose.

Examples of the electrolytes, besides the magnesium chloride hydrate, include sodium chloride, potassium chloride and calcium chloride hydrate. Preferred electrolytes, besides the magnesium chloride hydrate, are sodium chloride, potassium chloride and calcium chloride hydrate.

The dialysis agent according to the first embodiment preferably contains an alkaline component. Examples of the alkaline component include sodium hydrogen carbonate (sodium bicarbonate) and the like.

The dialysis agent according to the first embodiment contains a pH modifying component, and the pH modifying component is preferably at least one of an acetic acid mixture composed of a mixture of sodium acetate and glacial acetic acid, and a combination of this acetic acid mixture and sodium acetate. Examples of the sodium acetate include anhydrous sodium acetate and sodium acetate trihydrate. The mixture of sodium acetate and glacial acetic acid is prepared by mixing and powdering the sodium acetate and the glacial acetic acid. In this acetic acid mixture, it is thought that the glacial acetic acid and at least a portion of the sodium acetate form a complex. The acetic acid mixture may also include unreacted sodium acetate that has not formed a complex. The formation of a complex between the sodium acetate and the glacial acetic acid can be confirmed by X-ray diffraction methods.

In those cases where the pH modifier used in the dialysis agent is not prepared as an acetic acid mixture by mixing sodium acetate and glacial acetic acid and then powdering the resulting mixture, but rather simply involves adding sodium acetate and glacial acetic acid as pH modifiers to the dialysis agent in the airtight container, the glacial acetic acid may dehydrate the anhydrous glucose to produce 5-hydroxymethylfurfural (5-HMF), and the liquid glacial acetic acid may cause consolidation and aggregation of the chemical agent.

The molar ratio between sodium acetate and glacial acetic acid in the acetic acid mixture is preferably within a range from 1:1 to 1:5, and is more preferably within a range from 3:1 to 4:1. If the molar ratio of glacial acetic acid relative to sodium acetate is 1 or greater, then the glacial acetic acid does not bind completely to the sodium acetate, the resulting mixture cannot be powdered, and there is a possibility that the stability of the chemical agent may be impaired. In an A agent in which the molar ratio of sodium acetate relative to glacial acetic acid is 5 or greater, the pH and the concentration of alkaline agents of the dialysis agent prepared when the A agent is combined with the B agent comprising sodium bicarbonate may be unsuitable for dialysis treatment. Examples of the acetic acid mixture include a 1:1 mixture of sodium acetate and glacial acetic acid (sodium diacetate (powder acetic acid)), a 3:1 mixture of sodium acetate and glacial acetic acid, a 8:2.2 mixture of sodium acetate and glacial acetic acid, and a 10:2 mixture of sodium acetate and glacial acetic acid. Among these, by using a mixture for which the ratio between sodium acetate and glacial acetic acid is from 1:1 to 5:1, the total amount of sodium acetate and glacial acetic acid used in the dialysis agent can be added as a powdered mixture, and additional sodium acetate need not be added separately, which can offer the advantage of a shortened production process. Furthermore, mixtures for which the ratio between anhydrous sodium acetate and glacial acetic acid is from 3:1 to 4:1 enable an acetic acid mixture having a stable particle size to be produced even if glacial acetic acid is added to anhydrous sodium acetate.

Examples of other pH modifying components include organic solid acids such as citric acid, malic acid, lactic acid, fumaric acid, succinic acid and malonic acid, and organic acid salts, including citrate salts such as sodium citrate, lactate salts such as sodium lactate, malate salts such as sodium malate, fumarate salts such as sodium fumarate, succinate salts such as sodium succinate, and malonate salts such as sodium malonate. Further examples of pH modifying components that may be used include citric acid anhydride (anhydrous citric acid), as well as citric acid monohydrate, sodium citrate anhydride (anhydrous sodium citrate), sodium citrate dihydrate, a combination of citric acid anhydride and sodium citrate anhydride, a combination of citric acid anhydride and sodium citrate dihydrate, a combination of citric acid monohydrate and sodium citrate anhydride, a combination of citric acid monohydrate and sodium citrate dihydrate, a combination of sodium diacetate (powder acetic acid) and anhydrous sodium acetate, and a combination of sodium diacetate (powder acetic acid) and sodium acetate trihydrate.

The blend amounts of the various components in the dialysis agent according to the first embodiment, in those cases where the components are diluted to appropriate concentrations and mixed, preferably result in a dialysis fluid having the concentrations listed below. As shown below, in the dialysis agent according to the first embodiment, even though the blend amount of the magnesium chloride hydrate in the dialysis agent is small, the magnesium chloride hydrate can still be added to the container with good precision.

$Na^+$: 140.6 to 135.2 mEq/L

$K^+$: 2.0 mEq/L

$Ca^{2+}$: 3.5 to 2.5 mEq/L

$Mg^{2+}$: 1.5 to 1 mEq/L

$Cl^-$: 113 to 110.5 mEq/L

$HCO_3^-$: 35 to 25 mEq/L

anhydrous glucose: 150 to 100 mg/dL

citrate ions: 2.4 to 0 mEq/L

or acetate ions: 12 to 0 mEq/L

In the dialysis agent according to the first embodiment, there are no particular limitations on the method used for filling the container, provided the filling is performed as "single solid components". The dialysis agent usually has a two-part composition composed of an "A agent" comprising the electrolytes containing the magnesium chloride hydrate, the carbohydrate component such as anhydrous glucose and the pH modifying component, and a "B agent" comprising the alkaline component such as sodium bicarbonate. The components of the A agent are placed in a container as "single solid components" without forming into granules. Further, there are no particular limitations on the order in which each of the components is added to the container. Moreover, the "sodium chloride" which represents the main electrolytes of the "A agent" may be packaged separately, resulting in a three-part composition. In the first embodiment, the term "A agent" means a formulation comprising electrolytes containing at least magnesium chloride hydrate, anhydrous glucose, and a pH modifier.

<Second Embodiment of Dialysis Agent>

A dialysis agent according to a second embodiment of the present invention is a dialysis agent comprising electrolytes containing at least magnesium chloride hydrate and calcium chloride hydrate, and a pH modifier, wherein each of the constituent components is placed in a container as a single solid component. In the dialysis agent according to the second embodiment, the ratio between the water content of the entire dialysis agent and the sum of the amount of water removed from the magnesium chloride hydrate and the amount of water removed from the calcium chloride hydrate is not more than 210% but at least 2%, the water content of the magnesium chloride hydrate is within a range from 43% to 51%, and the water content of the calcium chloride hydrate is within a range from 1% to 22%.

Consolidation and aggregation, which are one major cause of deterioration in the solubility of dialysis agents, are thought to occur when aggregation, caused when the water having a high degree of freedom inside an airtight container forms liquid bridging to the crystals of each of the components of the dialysis agent, and drying of the resulting aggregates occur repeatedly, causing recrystallization between particles.

Further, it is thought that when a dialysis agent is placed in an airtight container, the water having a high degree of freedom inside the airtight container may act as a catalyst for the reaction between citric acid and sodium chloride (or magnesium chloride hydrate or calcium chloride hydrate), causing the generation of hydrogen chloride. There is a possibility that this generated hydrogen chloride may cause dehydration of the anhydrous glucose, producing 5-hydroxymethylfurfural (5-HMF).

On the other hand, the water contained in the magnesium chloride hexahydrate generally used as the magnesium chloride hydrate and the water contained in the calcium chloride dihydrate generally used as the calcium chloride hydrate is water of crystallization of deliquescent materials. This water of crystallization of magnesium chloride hexahydrate and calcium chloride dihydrate is chemically bonded water which is difficult to remove. As a result, dehydration of water of crystallization requires more energy than dehydration of free water. Accordingly, comprehending how much water needs to be removed from the magnesium chloride hexahydrate and the calcium chloride dihydrate and the like can be achieved by increasing the efficiency of, and optimizing, the magnesium chloride hydrate drying step and the calcium chloride hydrate drying step for the dialysis agent prepared by the method for providing a dialysis agent which includes a drying treatment step of drying the magnesium chloride hydrate and the calcium chloride hydrate.

Magnesium chloride hydrate has a water absorption ability, and for example when 203.31 g of magnesium chloride hexahydrate (water content: 53.2%) is dried to prepare a dried magnesium chloride hydrate having a water content of 44%, 33.3 g of water is lost. This lost water can be regarded as the amount of water that the dried magnesium chloride hydrate having a water content of 44% is able to absorb.

Similarly, calcium chloride hydrate has a water absorption ability, and for example when 147.0 g of calcium chloride dihydrate (water content: 24.5%) is dried to prepare a dried calcium chloride hydrate having a water content of 1%, 34.91 g of water is lost. This lost water can be regarded as the amount of water that the dried calcium chloride hydrate having a water content of 1% is able to absorb.

In the present description, the water absorption ability of magnesium chloride hydrate is represented by the difference between the initial amount of retained water (g) in the magnesium chloride hydrate ([weight of magnesium chloride hexahydrate] × [theoretical water content of magnesium chloride hexahydrate (53.2%)]) and the amount of retained water (g) following drying of the magnesium chloride hydrate ([weight of dried magnesium chloride hydrate] × [measured water content (%) of dried magnesium chloride hydrate]), and is referred to as "the amount of water removed from the magnesium chloride hydrate".

Further, the water absorption ability of dried calcium chloride hydrate is represented by the difference between the initial amount of retained water (g) in the calcium chloride hydrate ([weight of calcium chloride dihydrate] × [theoretical water content of calcium chloride dihydrate (24.5%)]) and the amount of retained water (g) following drying of the calcium chloride hydrate ([weight of dried calcium chloride hydrate] × [measured water content (%) of dried calcium chloride hydrate]), and is referred to as "the amount of water removed from the calcium chloride hydrate".

On the other hand, the water contained within each raw material of the dialysis agent may be broadly classified as two types, namely "water of adhesion" and "water of crystallization". Raw materials having water of crystallization include "deliquescent" raw materials which absorb water from the air and spontaneously become aqueous solutions, and "efflorescent" raw materials which release water of crystallization in air. The water of crystallization of efflorescent raw materials and the water of adhesion of raw materials represent water of a high degree of freedom which can be readily released into the air.

In the present description, the sum of the weight of the water of adhesion from raw materials and the weight of the water of crystallization of efflorescent components, which represents the water having a high degree of freedom, is referred to as "the water content of the entire dialysis agent".

First, the total amount of water of adhesion (amount of water of adhesion) is calculated from the measured water content, the theoretical water content, and the weight of each component except for the dried magnesium chloride hydrate and the dried calcium chloride hydrate.

```
Measured water content of each component (%) –

theoretical water content of each component (%) = water

content due to water of adhesion of each component (%)
```

$$\text{Weight of each component (g)} \times \text{water content due to}$$

$$\text{water of adhesion of each component (\%)} = \text{amount of water of}$$

$$\text{adhesion for each component (g)}$$

$$\text{Total of amounts of water of adhesion for all components}$$

$$\text{(g)} = \text{total amount of water of adhesion (amount of water of}$$

$$\text{adhesion) (g).}$$

Alternatively, the amount of water adhesion may also be determined as follows:

$$\text{Amount of water of adhesion (g)} = \text{(total weight of all}$$

$$\text{components (g)} - \text{weight of dried magnesium chloride hydrate}$$

$$\text{(g)} - \text{weight of dried calcium chloride hydrate (g))} \times \text{water}$$

$$\text{content of total amount of water of adhesion (\%).}$$

Then, the weight of the water of crystallization (amount of water of crystallization) within the efflorescent components is calculated from the theoretical water content of the efflorescent components and the weight of the efflorescent components.

$$\text{Amount of water of crystallization (g)} = \text{weight of}$$

$$\text{efflorescent components (g)} \times \text{theoretical water content of}$$

$$\text{efflorescent components (\%)}$$

The "water content of the entire dialysis agent" is determined from the sum of the "amount of water of adhesion" within the raw materials and the "weight of water of crystallization within the efflorescent components (amount of water of crystallization)" calculated in the manner described above. The ratio between the "water content of the entire dialysis agent" and the sum of the "amount of water removed from the magnesium chloride hydrate" and the "amount of water removed from the calcium chloride hydrate" is referred to as CM.

$$\text{Water content of entire dialysis agent (g)} = \text{amount of}$$

$$\text{water of adhesion (g)} + \text{amount of water of crystallization}$$

$$\text{(g)}$$

$$CM\ (\%) = [(\text{water content of entire dialysis agent (g)}) /$$

$$(\text{amount of water removed from magnesium chloride hydrate (g)}$$

$$+ \text{amount of water removed from calcium chloride hydrate (g)})]$$

$$\times\ 100$$

The inventors of the present invention discovered that provided CM, which represents the ratio between the "water content of the entire dialysis agent" and the sum of the "amount of water removed from the magnesium chloride hydrate" and the "amount of water removed from the calcium chloride hydrate" is not more than a prescribed value, consolidation and aggregation could be suppressed for a dialysis agent in which not granules, but rather single solid components of each of the various constituent components are placed in a container. Further, they also discovered that provided CM, which represents the ratio between the "water content of the entire dialysis agent" and the sum of the "amount of water removed from the magnesium chloride hydrate" and the "amount of water removed from the calcium chloride hydrate" is not more than the prescribed value, the amount of water having a high degree of freedom inside an airtight container can be reduced, generation of hydrogen chloride can be suppressed, and the production of 5-HMF can be inhibited. In other words, the magnesium chloride hydrate and calcium chloride hydrate, which are components that are generally included in small amounts among the raw materials included in the dialysis agent, are subjected to a drying treatment to remove not only the water of adhesion, but also some of the water of crystallization, thus imparting both the dried magnesium chloride hydrate and the dried calcium chloride hydrate with a function as "desiccants". In the dialysis agent in which each of the constituent components is placed in the container as a single solid component, the discovery of this relationship between the parameter represented by the ratio CM between the "water content of the entire dialysis agent" and the sum of the "amount of water removed from the magnesium chloride hydrate" and the "amount of water removed from the calcium chloride hydrate", and suppression of consolidation and aggregation has great significance.

The ratio CM between the "water content of the entire dialysis agent" and the sum of the "amount of water removed from the magnesium chloride hydrate" and the "amount of water removed from the calcium chloride hydrate" is not more than 210%. Further, the ratio CM between the "water content of the entire dialysis agent" and the sum of the "amount of water removed from the magnesium chloride hydrate" and the "amount of water removed from the calcium chloride hydrate" is preferably less than 167%, and more preferably 135% or lower. If the ratio CM between the "water content of the entire dialysis agent" and the sum of the "amount of water removed from the magnesium chloride hydrate" and the "amount of water removed from the calcium chloride hydrate" is less than 167%, then even in those cases when the dialysis agent contains anhydrous glucose as a carbohydrate component, degradation of the anhydrous glucose can be inhibited. If consideration is given to moisture and the like entering from outside the airtight container, then the ratio CM between the "water content of the entire dialysis agent" and the sum of the "amount of water removed from the magnesium chloride hydrate" and the "amount of water removed from the calcium chloride hydrate" is still more preferably 75% or lower, and in terms of long-term storage and the like, is most preferably 60% or lower. The ratio CM is preferably as low as possible, but if the ratio CM is too low, then further reduction in the ratio CM may sometimes require excessive drying treatment of the magnesium chloride hydrate and the calcium chloride hydrate, and therefore in order to achieve satisfactory results with an appropriate drying treatment, the ratio CM is typically at least 2%, preferably at least 4%, and more preferably 7% or higher.

It is thought that provided the ratio CM is not more than 210%, water having a high degree of freedom, which can cause consolidation and aggregation of the dialysis agent, can be absorbed by the dried magnesium chloride hydrate and the dried calcium chloride hydrate, thereby suppressing consolidation and aggregation of the dialysis agent. It is thought that if the ratio CM is less than 167%, then water having a high degree of freedom, which can cause degradation of anhydrous glucose, can adequately be absorbed by the dried magnesium chloride hydrate and the dried calcium chloride hydrate, meaning even in those cases when the dialysis agent contains anhydrous glucose as a carbohydrate component, degradation of the anhydrous glucose can be inhibited. The amount of water which must be removed from the magnesium chloride hydrate and the calcium chloride hydrate to suppress consolidation and aggregation of the dialysis agent and degradation of the anhydrous glucose may be restricted to the minimum amount required for each formulation (namely, the amounts of the various components in the A agent), and therefore the drying step can be made more efficient. Further, compared with a method for providing a dialysis agent in which the components are formed into granules, the unit operations can be shortened and simplified, thereby improving the productivity and reducing the chances of foreign matter contamination. Further, the establishment of production conditions when the variety of agent is altered (alteration of the concentration of each component) can be easier. Moreover, raw material remaining in the production equipment need not be removed when the variety of agent is altered (alteration of the concentration of each component), thus improving productivity. Accordingly, a dialysis agent of low cost and high quality can be achieved.

Further, it is thought that including, inside the airtight container, dried magnesium chloride hydrate and dried calcium chloride hydrate that absorb the water having a high degree of freedom inside the airtight container enables the suppression of consolidation, which occurs upon repeated aggregation, caused when the water having a high degree of freedom inside the airtight container forms liquid bridging to the crystals of each of the components, and subsequent drying of the resulting aggregates, resulting in recrystallization between particles.

Furthermore, it is thought that by including, inside the airtight container, sufficient dried magnesium chloride hydrate and dried calcium chloride hydrate to absorb the water having a high degree of freedom inside the airtight container and any moisture entering from outside the airtight container, the water having a high degree of freedom that can cause degradation of the anhydrous glucose is absorbed by the dried magnesium chloride hydrate and the dried calcium chloride hydrate, meaning degradation of the anhydrous glucose can be inhibited.

In the dialysis agent according to the second embodiment, the water content of the magnesium chloride hydrate is within a range from 43% to 51%, and is preferably within a range from 43% to 45%. The "water content" indicates the proportion (mass %) of water contained in the substance. By ensuring that the water content of the magnesium chloride hydrate is within a range from 43% to 51%, the production process can be simplified, the productivity can improve, and the problem of foreign matter contamination can be minimized. For example, when magnesium chloride hexahydrate is dried in an atmosphere at 100°C, the dehydration rate moderates when the water content of the magnesium chloride hydrate reaches a range from 43% to 45%, and the dehydration essentially stops proceeding at about 43% (see FIG. 7). Accordingly, magnesium chloride hydrate having a water content within a range from 43% to 45% can offer the advantage that the water content can be readily controlled. Further, by using magnesium chloride hydrate having a water content within the range from 43% to 45%, the dialysis agent can be produced using a magnesium chloride hydrate for which the water content is stable, which can offer another advantage in that the precision of the magnesium content of the dialysis agent can be stabilized. By using a magnesium chloride hydrate prepared by drying magnesium chloride hexahydrate until the water content is within a range from 43% to 45%, handling can be easier and the costs can be reduced compared with the case where an anhydrous magnesium chloride having a faster water absorption rate is used.

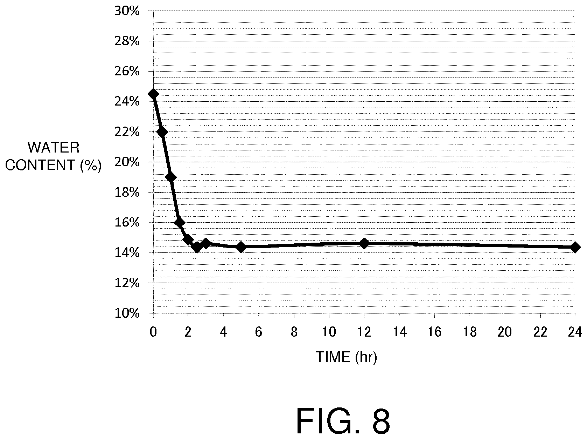

In the dialysis agent according to the second embodiment, the water content of the calcium chloride hydrate is within a range from 1% to 22%, and is preferably within a range from 14% to 22%, and more preferably within a range from 14% to 16%. The "water content" indicates the proportion (mass %) of water contained in the substance. By ensuring that the water content of the calcium chloride hydrate is within a range from 1% to 22%, the production process can be simplified, the productivity can improve, and the problem of foreign matter contamination can be minimized. Calcium chloride hydrate having a water content within a range from 14% to 22% can offer the advantage that, compared with a calcium chloride hydrate having a water content of less than 1%, the dialysis agent can be produced without requiring as much energy during production (drying). Provided the ratio CM is a value within the range described above, stability of the chemical agent can be achieved even when calcium chloride hydrate having a water content within a range from 14% to 22% is used. For example, when calcium chloride dihydrate is dried in an atmosphere at 100°C, the dehydration rate moderates when the water content of the calcium chloride hydrate reaches a range from 14% to 16%, and the dehydration essentially stops proceeding at about 14% (see FIG. 8). Accordingly, calcium chloride hydrate having a water content within a range from 14% to 16% can offer the advantage that the water content can be readily controlled. Further, by using calcium chloride hydrate having a water content within the range from 14% to 16%, the dialysis agent can be produced using a calcium chloride hydrate for which the water content is stable, which can offer another advantage in that the precision of the calcium content of the dialysis agent can be stabilized. By using a calcium chloride hydrate prepared by drying calcium chloride dihydrate until the water content is within a range from 14% to 16%, handling can be easier and the costs can be reduced compared with the case where a calcium chloride hydrate having a water content of less than 1% and having a faster water absorption rate is used.

Moreover, subjecting both the magnesium chloride hydrate and the calcium chloride hydrate to drying treatments can offer the advantage of improved stability of the chemical agent compared with the case where only one of the magnesium chloride hydrate and the calcium chloride hydrate is subjected to a drying treatment.

In this manner, by using the method for providing a dialysis agent according to the second embodiment, a problem that occurs as a result of forming the agent into granules, wherein complex production conditions must be established when the variety of agent is altered (alteration of the concentration of each component), can be resolved. Further, a problem that occurs when forming the agent into granules results in more complex unit operations, leading to a loss in productivity, and the resulting increase in unit operations increases the chances of foreign matter contamination, can also be resolved. Furthermore, another problem that occurs as a result of forming the agent into granules, wherein establishment of the production conditions upon alteration of the variety of agent (alteration of the concentration of each component) becomes difficult, can also be resolved. Moreover, another problem that occurs when the variety of agent is altered (alteration of the concentration of each component), wherein the raw material remaining in the production equipment must be removed, resulting in product loss and time loss, and causing a deterioration in the productivity, can also be resolved. Accordingly, a dialysis agent of low cost and high quality can be achieved.

In the dialysis agent according to the second embodiment, the magnesium chloride hydrate and the calcium chloride hydrate have preferably each been subjected to a drying treatment, and the magnesium chloride hydrate and the calcium chloride hydrate have more preferably each been subjected to a heated drying treatment in an atmosphere of 75°C or higher. Provided the atmosphere in the heated drying treatments of the magnesium chloride hydrate and the calcium chloride hydrate is at least 75°C, the water content within the magnesium chloride hydrate and the calcium chloride hydrate can be readily removed, making it easier to achieve a ratio between the water content of the entire dialysis agent and the sum of the amount of water removed from the magnesium chloride hydrate and the amount of water removed from the calcium chloride hydrate that is less than 210% but at least 2%, as well as a water content for the magnesium chloride hydrate within a range from 43% to 51% ,and a water content for the calcium chloride hydrate within a range from 1% to 22%.

The dialysis agent according to the second embodiment preferably contains a carbohydrate component. An example of the carbohydrate component is anhydrous glucose.

Examples of the electrolytes, besides the magnesium chloride hydrate and the calcium chloride hydrate, include sodium chloride and potassium chloride and the like. Preferred electrolytes, besides the magnesium chloride hydrate and the calcium chloride hydrate, are sodium chloride and potassium chloride.

The dialysis agent according to the second embodiment preferably contains an alkaline component. Examples of the alkaline component include sodium hydrogen carbonate (sodium bicarbonate) and the like.

The dialysis agent according to the second embodiment contains a pH modifying component, and the pH modifying component is preferably at least one of an acetic acid mixture composed of a mixture of sodium acetate and glacial acetic acid, and a combination of this acetic acid mixture and sodium acetate. Examples of the sodium acetate include anhydrous sodium acetate and sodium acetate trihydrate. The mixture of sodium acetate and glacial acetic acid is prepared by mixing and powdering the sodium acetate and the glacial acetic acid. In this acetic acid mixture, it is thought that the glacial acetic acid and at least a portion of the sodium acetate form a complex. The acetic acid mixture may also include unreacted sodium acetate that has not formed a complex. The formation of a complex between the sodium acetate and the glacial acetic acid can be confirmed by X-ray diffraction methods.

In those cases where the pH modifier used in the dialysis agent is not prepared as an acetic acid mixture by mixing sodium acetate and glacial acetic acid and then powdering the resulting mixture, but rather simply involves adding sodium acetate and glacial acetic acid as pH modifiers to the dialysis agent in the airtight container, the glacial acetic acid may dehydrate the anhydrous glucose to produce 5-hydroxymethylfurfural (5-HMF), and the liquid glacial acetic acid may cause consolidation and aggregation of the chemical agent.

The molar ratio between anhydrous sodium acetate and glacial acetic acid in the acetic acid mixture is preferably within a range from 1:1 to 1:5, and is more preferably within a range from 3:1 to 4:1. If the molar ratio of glacial acetic acid relative to sodium acetate is 1 or greater, then the glacial acetic acid does not bind completely to the sodium acetate, the resulting mixture cannot be powdered, and there is a possibility that the stability of the chemical agent may be impaired. In an A agent in which the molar ratio of sodium acetate relative to glacial acetic acid is 5 or greater, the pH and the concentration of alkaline agents of the dialysis agent prepared when the A agent is combined with the B agent comprising sodium bicarbonate may be unsuitable for dialysis treatment. Examples of the acetic acid mixture include a 1:1 mixture of sodium acetate and glacial acetic acid (sodium diacetate (powder acetic acid)), a 3:1 mixture of sodium acetate and glacial acetic acid, a 8:2.2 mixture of sodium acetate and glacial acetic acid, and a 10:2 mixture of sodium acetate and glacial acetic acid. Among these, by using a mixture for which the ratio between sodium acetate and glacial acetic acid is from 1:1 to 5:1, the total amount of sodium acetate and glacial acetic acid used in the dialysis agent can be added as a powdered mixture, and additional sodium acetate need not be added separately, which can offer the advantage of a shortened production process. Furthermore, mixtures for which the ratio between anhydrous sodium acetate and glacial acetic acid is from 3:1 to 4:1 enable an acetic acid mixture having a stable particle size to be produced even if glacial acetic acid is added to anhydrous sodium acetate.

Examples of other pH modifying components include organic solid acids such as citric acid, malic acid, lactic acid, fumaric acid, succinic acid and malonic acid, and organic acid salts, including citrate salts such as sodium citrate, lactate salts such as sodium lactate, malate salts such as sodium malate, fumarate salts such as sodium fumarate, succinate salts such as sodium succinate, and malonate salts such as sodium malonate. Further examples of pH modifying components that may be used include citric acid anhydride (anhydrous citric acid), as well as citric acid monohydrate, sodium citrate anhydride (anhydrous sodium citrate), sodium citrate dihydrate, a combination of citric acid anhydride and sodium citrate anhydride, a combination of citric acid anhydride and sodium citrate dihydrate, a combination of citric acid monohydrate and sodium citrate anhydride, a combination of citric acid monohydrate and sodium citrate dihydrate, a combination of sodium diacetate (powder acetic acid) and anhydrous sodium acetate, and a combination of sodium diacetate (powder acetic acid) and sodium acetate trihydrate.

The blend amounts of the various components in the dialysis agent according to the second embodiment, in those cases where the components are diluted to appropriate concentrations and mixed, preferably result in a dialysis fluid having the concentrations listed below. As shown below, in the dialysis agent according to the second embodiment, even though the blend amounts of the magnesium chloride hydrate and the calcium chloride hydrate in the dialysis agent are small, they can still be added to the container with good precision.

$Na^+$: 140.6 to 135.2 mEq/L

$K^+$: 2.0 mEq/L

$Ca^{2+}$: 3.5 to 2.5 mEq/L

$Mg^{2+}$: 1.5 to 1 mEq/L

$Cl^-$: 113 to 110.5 mEq/L

$HCO_3^-$: 35 to 25 mEq/L

anhydrous glucose: 150 to 100 mg/dL

citrate ions: 2.4 to 0 mEq/L

or acetate ions: 12 to 0 mEq/L

In the dialysis agent according to the second embodiment, there are no particular limitations on the method used for filling the container, provided the filling is performed as "single solid components". The dialysis agent usually has a two-part composition composed of an "A agent" comprising the electrolytes containing the magnesium chloride hydrate and the calcium chloride hydrate, the carbohydrate component such as anhydrous glucose and the pH modifying component, and a "B agent" comprising the alkaline component such as sodium bicarbonate. The components of the A agent are placed in a container as "single solid components" without forming into granules. Further, there are no particular limitations on the order in which each of the components is added to the container. Moreover, the "sodium chloride" which represents the main electrolytes of the "A agent" may be packaged separately, resulting in a three-part composition. In the second embodiment, the term "A agent" means a formulation comprising electrolytes containing at least magnesium chloride hydrate, anhydrous glucose, and a pH modifier.

<Reference Embodiment 1 of Dialysis Agent>

An object of the reference embodiment 1 is to provide a dialysis agent which comprises at least calcium chloride hydrate as electrolytes, and in which each of the constituent components is placed in a container as a single solid component, wherein consolidation and aggregation of the chemical agent can be suppressed, and the dialysis agent can be obtained at low cost.

Further, another object of the reference embodiment 1 is to provide a method for providing a dialysis agent which comprises at least calcium chloride hydrate as electrolytes and in which each of the constituent components is placed in a container as a single solid component, the method enabling a dialysis agent for which consolidation and aggregation of the chemical agent can be suppressed to be obtained at low cost.

A dialysis agent according to the reference embodiment 1 is a dialysis agent comprising electrolytes containing at least calcium chloride hydrate, and an acetic acid mixture composed of a mixture of sodium acetate and glacial acetic acid, wherein the ratio between the water content of the entire dialysis agent and the amount of water removed from the calcium chloride hydrate is less than 163% but at least 2%, the water content of the calcium chloride hydrate is within a range from 1% to 22%, and each of the constituent components is placed in a container as a single solid component.

Further, in the above dialysis agent, the ratio between the water content of the entire dialysis agent and the amount of water removed from the calcium chloride hydrate is preferably less than 82% but at least 2%.

Furthermore, in the above dialysis agent, the molar ratio between sodium acetate and glacial acetic acid in the acetic acid mixture is preferably within a range from 1:1 to 5:1, and the molar ratio is more preferably within a range from 3:1 to 4:1.

Further, in the above dialysis agent, the water content of the calcium chloride hydrate is preferably within a range from 14% to 22%, and more preferably within a range from 14% to 16%.

Furthermore, a method for providing the dialysis agent according to the reference embodiment 1 is a method for providing a dialysis agent comprising electrolytes containing at least calcium chloride hydrate, and an acetic acid mixture composed of a mixture of sodium acetate and glacial acetic acid, the method comprising a drying treatment step of drying the calcium chloride hydrate so that the ratio between the water content of the entire dialysis agent and the amount of water removed from the calcium chloride hydrate becomes less than 163% but at least 2%, and the water content of the calcium chloride hydrate becomes a value within a range from 1% to 22%, and a filling step of placing each of the constituent components in a container as a single solid component.

Moreover, in the drying treatment step of the above method for providing a dialysis agent, the calcium chloride hydrate is preferably dried so that the ratio between the water content of the entire dialysis agent and the amount of water removed from the calcium chloride hydrate becomes less than 82% but at least 2%.

Further, in the above method for providing a dialysis agent, the molar ratio between sodium acetate and glacial acetic acid in the acetic acid mixture is preferably within a range from 1:1 to 5:1, and the molar ratio is more preferably within a range from 3:1 to 4:1.

Furthermore, in the drying treatment step of the above method for providing a dialysis agent, the calcium chloride hydrate is preferably dried so that the water content of the calcium chloride hydrate becomes a value within a range from 14% to 22%, and is more preferably dried so that the water content of the calcium chloride hydrate becomes a value within a range from 14% to 16%.

In the reference embodiment 1, in the dialysis agent comprising at least calcium chloride hydrate as electrolytes, wherein each of the constituent components is placed in a container as a single solid component, by ensuring that the ratio between the water content of the entire dialysis agent and the amount of water removed from the calcium chloride hydrate is less than 163% but at least 2%, and the water content of the calcium chloride hydrate is within a range from 1% to 22%, a dialysis agent is provided which can be obtained at low cost, and in which consolidation and aggregation of the chemical agent can be suppressed.

Further, in the reference embodiment 1, in the method for providing a dialysis agent comprising at least calcium chloride hydrate as electrolytes, wherein each of the constituent components is placed in a container as a single solid component, by including an acetic acid mixture composed of a mixture of sodium acetate and glacial acetic acid, and drying the calcium chloride hydrate so that the ratio between the water content of the entire dialysis agent and the amount of water removed from the calcium chloride hydrate becomes less than 163% but at least 2%, and the water content of the calcium chloride hydrate becomes a value within a range from 1% to 22%, a dialysis agent which can suppress consolidation and aggregation of the chemical agent can be obtained at low cost.

The dialysis agent according to the reference embodiment 1 is a dialysis agent comprising electrolytes containing at least calcium chloride hydrate, and an acetic acid mixture composed of a mixture of sodium acetate and glacial acetic acid, wherein each of the constituent components is placed in a container as a single solid component. In the dialysis agent according to the reference embodiment 1, the ratio between the water content of the entire dialysis agent and the amount of water removed from the calcium chloride hydrate is less than 163% but at least 2%, and the water content of the calcium chloride hydrate is within a range from 1% to 22%.

Consolidation and aggregation, which are one major cause of deterioration in the solubility of dialysis agents, are thought to occur when aggregation, caused when the water having a high degree of freedom inside an airtight container forms liquid bridging to the crystals of each of the components of the dialysis agent, and drying of the resulting aggregates occur repeatedly, causing recrystallization between particles.

Further, it is thought that when a dialysis agent is placed in an airtight container, the water having a high degree of freedom inside the airtight container may act as a catalyst for the reaction between citric acid and sodium chloride (or magnesium chloride hydrate or calcium chloride hydrate), causing the generation of hydrogen chloride. There is a possibility that this generated hydrogen chloride may cause dehydration of the anhydrous glucose, producing 5-hydroxymethylfurfural (5-HMF).

On the other hand, the water contained in the calcium chloride dihydrate generally used as the calcium chloride hydrate is water of crystallization of a deliquescent material. This water of crystallization of calcium chloride dihydrate is chemically bonded water which is difficult to remove. As a result, dehydration of water of crystallization requires more energy than dehydration of free water. Accordingly, comprehending how much water needs to be removed from the calcium chloride dihydrate and the like can be achieved by increasing the efficiency of, and optimizing, the calcium chloride hydrate drying step for the dialysis agent prepared by the method for providing a dialysis agent which includes a drying treatment step of drying the calcium chloride hydrate.

Calcium chloride hydrate has a water absorption ability, and for example when 147.0 g of calcium chloride dihydrate (water content: 24.5%) is dried to prepare a dried calcium chloride hydrate having a water content of 1%, 34.91 g of water is lost. This lost water can be regarded as the amount of water that the dried calcium chloride hydrate having a water content of 1% is able to absorb.

In the present description, the water absorption ability of dried calcium chloride hydrate is represented by the difference between the initial amount of retained water (g) in the calcium chloride hydrate ([weight of calcium chloride dihydrate] $\times$ [theoretical water content of calcium chloride dihydrate (24.5%)]) and the amount of retained water (g) following drying of the calcium chloride hydrate ([weight of dried calcium chloride hydrate] $\times$ [measured water content (%) of dried calcium chloride hydrate]), and is referred to as "the amount of water removed from the calcium chloride hydrate".

On the other hand, the water contained within each raw material of the dialysis agent may be broadly classified as two types, namely "water of adhesion" and "water of crystallization". Raw materials having water of crystallization include "deliquescent" raw materials which absorb water from the air and spontaneously become aqueous solutions, and "efflorescent" raw materials which release water of crystallization in air. The water of crystallization of efflorescent raw materials and the water of adhesion of raw materials represent water of a high degree of freedom which can be readily released into the air.

In the present description, the sum of the weight of the water of adhesion from raw materials and the weight of the water of crystallization of efflorescent components, which represents the water having a high degree of freedom, is referred to as "the water content of the entire dialysis agent".

First, the total amount of water of adhesion (amount of water of adhesion) is calculated from the measured water content, the theoretical water content, and the weight of each component except for the dried calcium chloride hydrate.

Measured water content of each component (%) −

theoretical water content of each component (%) = water

content due to water of adhesion of each component (%)

Weight of each component (g) × water content due to water of adhesion of each component (%) = amount of water of adhesion for each component (g)

Total of amounts of water of adhesion for all components

(g) = total amount of water of adhesion (amount of water of

adhesion) (g).

Alternatively, the amount of water adhesion may also be determined as follows:

Amount of water of adhesion (g) = (total weight of all

components (g) − weight of dried calcium chloride hydrate

(g)) × water content of total amount of water of adhesion

(%).

Then, the weight of the water of crystallization (amount of water of crystallization) within the efflorescent components is calculated from the theoretical water content of the efflorescent components and the weight of the efflorescent components.

Amount of water of crystallization (g) = weight of

efflorescent components (g) × theoretical water content of

efflorescent components (%)

The "water content of the entire dialysis agent" is determined from the sum of the "amount of water of adhesion" within the raw materials and the "weight of water of crystallization within the efflorescent components (amount of water of crystallization)" calculated in the manner described above. The ratio between the "water content of the entire dialysis agent" and the "amount of water removed from the calcium chloride hydrate" is referred to as C.

```
Water content of entire dialysis agent (g) = amount of

water of adhesion (g) + amount of water of crystallization

(g)

   C (%) = [(water content of entire dialysis agent (g)) /

(amount of water removed from calcium chloride hydrate (g))]

   × 100
```

The inventors of the present invention discovered that provided C, which represents the ratio between the "water content of the entire dialysis agent" and the "amount of water removed from the calcium chloride hydrate" is not more than a prescribed value, consolidation and aggregation could be suppressed for a dialysis agent in which not granules, but rather single solid components of each of the various constituent components are placed in a container. Further, they also discovered that provided C, which represents the ratio between the "water content of the entire dialysis agent" and the "amount of water removed from the calcium chloride hydrate" is not more than the prescribed value, the amount of water having a high degree of freedom inside an airtight container can be reduced, generation of hydrogen chloride can be suppressed, and the production of 5-HMF can be inhibited. In other words, the calcium chloride hydrate, which is a component generally included in a small amount among the raw materials included in the dialysis agent, is subjected to a drying treatment to remove not only the water of adhesion, but also some of the water of crystallization, thus imparting the dried calcium chloride hydrate with a function as a "desiccant". In the dialysis agent in which each of the constituent components is placed in the container as a single solid component, the discovery of this relationship between the parameter represented by the ratio C between the "water content of the entire dialysis agent" and the "amount of water removed from the calcium chloride hydrate", and suppression of consolidation and aggregation has great significance.

The ratio C between the "water content of the entire dialysis agent" and the "amount of water removed from the calcium chloride hydrate" is less than 163%, but is preferably 113% or lower. Further, the ratio C between the "water content of the entire dialysis agent" and the "amount of water removed from the calcium chloride hydrate" is more preferably less than 82%, and still more preferably 77% or lower. If the ratio C between the "water content of the entire dialysis agent" and the "amount of water removed from the calcium chloride hydrate" is less than 82%, then even in those cases when the dialysis agent contains anhydrous glucose as a carbohydrate component, degradation of the anhydrous glucose can be inhibited. If consideration is given to moisture and the like entering from outside the airtight container, then the ratio C between the "water content of the entire dialysis agent" and the "amount of water removed from the calcium chloride hydrate" is still more preferably 55% or lower, and in terms of long-term storage and the like, is most preferably 40% or lower. The ratio C is preferably as low as possible, but if the ratio C is too low, then further reduction in the ratio C may sometimes require excessive drying treatment of the calcium chloride hydrate, and therefore in order to achieve satisfactory results with an appropriate drying treatment, the ratio C is typically at least 2%, preferably at least 4%, and more preferably 7% or higher.

It is thought that provided the ratio C is less than 163%, water having a high degree of freedom, which can cause consolidation and aggregation of the dialysis agent, can be absorbed by the dried calcium chloride hydrate, thereby suppressing consolidation and aggregation of the dialysis agent. It is thought that if the ratio C is less than 82%, then water having a high degree of freedom, which can cause degradation of anhydrous glucose, can adequately be absorbed by the dried calcium chloride hydrate, meaning even in those cases when the dialysis agent contains anhydrous glucose as a carbohydrate component, degradation of the anhydrous glucose can be inhibited. The amount of water which must be removed from the calcium chloride hydrate to suppress consolidation and aggregation of the dialysis agent and degradation of the anhydrous glucose may be restricted to the minimum amount required for each formulation (namely, the amounts of the various components in the A agent), and therefore the drying step can be made more efficient. Further, compared with a method for providing a dialysis agent in which the components are formed into granules, the unit operations can be shortened and simplified, thereby improving the productivity and reducing the chances of foreign matter contamination. Further, the establishment of production conditions when the variety of agent is altered (alteration of the concentration of each component) can be easier. Moreover, raw material remaining in the production equipment need not be removed when the variety of agent is altered (alteration of the concentration of each component), thus improving productivity. Accordingly, a dialysis agent of low cost and high quality can be achieved.

Further, it is thought that including, inside the airtight container, dried calcium chloride hydrate that absorbs the water having a high degree of freedom inside the airtight container enables the suppression of consolidation, which occurs upon repeated aggregation, caused when the water having a high degree of freedom inside the airtight container forms liquid bridging to the crystals of each of the components, and subsequent drying of the resulting aggregates, resulting in recrystallization between particles.

Furthermore, it is thought that by including, inside the airtight container, sufficient dried calcium chloride hydrate to absorb the water having a high degree of freedom inside the airtight container and any moisture entering from outside the airtight container, the water having a high degree of freedom that can cause degradation of the anhydrous glucose is absorbed by the dried calcium chloride hydrate, meaning degradation of the anhydrous glucose can be inhibited.

In the dialysis agent according to the reference embodiment 1, the water content of the calcium chloride hydrate is within a range from 1% to 22%, and is preferably within a range from 14% to 22%, and more preferably within a range from 14% to 16%. The "water content" indicates the proportion (mass %) of water contained in the substance. By ensuring that the water content of the calcium chloride hydrate is within a range from 1% to 22%, the production process can be simplified, the productivity can improve, and the problem of foreign matter contamination can be minimized. Calcium chloride hydrate having a water content within a range from 14% to 22% can offer the advantage that, compared with a calcium chloride hydrate having a water content of less than 1%, the dialysis agent can be produced without requiring as much energy during production (drying). Provided the ratio C is a value within the range described above, stability of the chemical agent can be achieved even when calcium chloride hydrate having a water content within a range from 14% to 22% is used. For example, when calcium chloride dihydrate is dried in an atmosphere at 100°C, the dehydration rate moderates when the water content of the calcium chloride hydrate reaches a range from 14% to 16%, and the dehydration essentially stops proceeding at about 14% (see FIG. 8). Accordingly, calcium chloride hydrate having a water content within a range from 14% to 16% can offer the advantage that the water content can be readily controlled. Further, by using calcium chloride hydrate having a water content within the range from 14% to 16%, the dialysis agent can be produced using a calcium chloride hydrate for which the water content is stable, which can offer another advantage in that the precision of the calcium content of the dialysis agent can be stabilized. By using a calcium chloride hydrate prepared by drying calcium chloride dihydrate until the water content is within a range from 14% to 16%, handling can be easier and the costs can be reduced compared with the case where a calcium chloride hydrate having a water content of less than 1% and having a faster water absorption rate is used.

In this manner, by using the method for providing a dialysis agent according to the reference embodiment 1, a problem that occurs as a result of forming the agent into granules, wherein complex production conditions must be established when the variety of agent is altered (alteration of the concentration of each component), can be resolved. Further, a problem that occurs when forming the agent into granules results in more complex unit operations, leading to a loss in productivity, and the resulting increase in unit operations increases the chances of foreign matter contamination, can also be resolved. Furthermore, another problem that occurs as a result of forming the agent into granules, wherein establishment of the production conditions upon alteration of the variety of agent (alteration of the concentration of each component) becomes difficult, can also be resolved. Moreover, another problem that occurs when the variety of agent is altered (alteration of the concentration of each component), wherein the raw material remaining in the production equipment must be removed, resulting in product loss and time loss, and causing a deterioration in the productivity, can also be resolved. Accordingly, a dialysis agent of low cost and high quality can be achieved.

In the dialysis agent according to the reference embodiment 1, the calcium chloride hydrate has preferably been subjected to a drying treatment, and the calcium chloride hydrate has more preferably been subjected to a heated drying treatment in an atmosphere of 75°C or higher. Provided the atmosphere in the heated drying treatment of the calcium chloride hydrate is at least 75°C, the water content within the calcium chloride hydrate can be readily removed, making it easier to achieve a ratio between the water content of the entire dialysis agent and the amount of water removed from the calcium chloride hydrate that is less than 163% but at least 2%, and a water content for the calcium chloride hydrate within a range from 1% to 22%.

The dialysis agent according to the reference embodiment 1 preferably contains a carbohydrate component. An example of the carbohydrate component is anhydrous glucose.

Examples of the electrolytes, besides the calcium chloride hydrate, include sodium chloride, potassium chloride, and magnesium chloride hydrate and the like. Preferred electrolytes, besides the calcium chloride hydrate, are sodium chloride, potassium chloride and magnesium chloride hydrate.

The dialysis agent according to the reference embodiment 1 preferably contains an alkaline component. Examples of the alkaline component include sodium hydrogen carbonate (sodium bicarbonate) and the like.

The dialysis agent according to the reference embodiment 1 contains an acetic acid mixture composed of a mixture of sodium acetate and glacial acetic acid as a pH modifying component. Examples of the sodium acetate include anhydrous sodium acetate and sodium acetate trihydrate. The mixture of sodium acetate and glacial acetic acid is prepared by mixing and powdering the sodium acetate and the glacial acetic acid. In this acetic acid mixture, it is thought that the glacial acetic acid and at least a portion of the sodium acetate form a complex. The acetic acid mixture may also include unreacted sodium acetate that has not formed a complex. The formation of a complex between the sodium acetate and the glacial acetic acid can be confirmed by X-ray diffraction methods.

In those cases where the pH modifier used in the dialysis agent is not prepared as an acetic acid mixture by mixing sodium acetate and glacial acetic acid and then powdering the resulting mixture, but rather simply involves adding sodium acetate and glacial acetic acid as pH modifiers to the dialysis agent in the airtight container, the glacial acetic acid may dehydrate the anhydrous glucose to produce 5-hydroxymethylfurfural (5-HMF), and the liquid glacial acetic acid may cause consolidation and aggregation of the chemical agent.

The molar ratio between sodium acetate and glacial acetic acid in the acetic acid mixture is preferably within a range from 1:1 to 1:5, and is more preferably within a range from 3:1 to 4:1. If the molar ratio of glacial acetic acid relative to sodium acetate is 1 or greater, then the glacial acetic acid does not bind completely to the sodium acetate, the resulting mixture cannot be powdered, and there is a possibility that the stability of the chemical agent may be impaired. In an A agent in which the molar ratio of sodium acetate relative to glacial acetic acid is 5 or greater, the pH and the concentration of alkaline agents of the dialysis agent prepared when the A agent is combined with the B agent comprising sodium bicarbonate may be unsuitable for dialysis treatment. Examples of the acetic acid mixture include a 1:1 mixture of sodium acetate and glacial acetic acid (sodium diacetate (powder acetic acid)), a 3:1 mixture of sodium acetate and glacial acetic acid, a 8:2.2 mixture of sodium acetate and glacial acetic acid, and a 10:2 mixture of sodium acetate and glacial acetic acid. Among these, by using a mixture for which the ratio between sodium acetate and glacial acetic acid is from 1:1 to 5:1, the total amount of sodium acetate and glacial acetic acid used in the dialysis agent can be added as a powdered mixture, and additional sodium acetate need not be added separately, which can offer the advantage of a shortened production process. Furthermore, mixtures for which the ratio between anhydrous sodium acetate and glacial acetic acid is from 3:1 to 4:1 enable an acetic acid mixture having a stable particle size to be produced even if glacial acetic acid is added to anhydrous sodium acetate.

Examples of other pH modifying components include organic solid acids such as citric acid, malic acid, lactic acid, fumaric acid, succinic acid and malonic acid, and organic acid salts, including citrate salts such as sodium citrate, lactate salts such as sodium lactate, malate salts such as sodium malate, fumarate salts such as sodium fumarate, succinate salts such as sodium succinate, and malonate salts such as sodium malonate.

The blend amounts of the various components in the dialysis agent according to the reference embodiment 1, in those cases where the components are diluted to appropriate concentrations and mixed, preferably result in a dialysis fluid having the concentrations listed below. As shown below, in the dialysis agent according to the reference embodiment 1, even though the blend amount of the calcium chloride hydrate in the dialysis agent is small, the calcium chloride hydrate can still be added to the container with good precision.

$Na^+$: 140.6 to 135.2 mEq/L
$K^+$: 2.0 mEq/L
$Ca^{2+}$: 3.5 to 2.5 mEq/L
$Mg^{2+}$: 1.5 to 1 mEq/L
$Cl^-$: 113 to 110.5 mEq/L
$HCO_3^-$: 35 to 25 mEq/L
anhydrous glucose: 150 to 100 mg/dL
citrate ions: 2.4 to 0 mEq/L
or acetate ions: 12 to 0 mEq/L

In the dialysis agent according to the reference embodiment 1, there are no particular limitations on the method used for filling the container, provided the filling is performed as "single solid components". The dialysis agent usually has a two-part composition composed of an "A agent" comprising the electrolytes containing the calcium chloride hydrate, the carbohydrate component such as anhydrous glucose and the pH modifying component, and a "B agent" comprising the alkaline component such as sodium bicarbonate. The components of the A agent are placed in a container as "single solid components" without forming into granules. Further, there are no particular limitations on the order in which each of the components is added to the container. Moreover, the "sodium chloride" which represents the main electrolytes of the "A agent" may be packaged separately, resulting in a three-part composition. In the reference embodiment 1, the term "A agent" means a formulation comprising electrolytes containing at least magnesium chloride hydrate, anhydrous glucose, and a pH modifier.

<Method of the present invention for Providing Dialysis Agent according to First Embodiment>

A method of the present invention for providing the dialysis agent of the first embodiment comprising electrolytes containing at least magnesium chloride hydrate, and a pH modifier is a method which comprises a drying treatment step of drying the magnesium chloride hydrate so that the ratio between the water content of the entire dialysis agent and the amount of water removed from the magnesium chloride hydrate becomes less than 118% but at least 7%, and the water content of the magnesium chloride hydrate becomes a value within a range from 43% to 51%, and a filling step of placing each of the constituent components in a container as a single solid component. In the drying treatment step, the magnesium chloride hydrate is preferably dried so that the ratio between the water content of the entire dialysis agent and the amount of water removed from the magnesium chloride hydrate becomes less than 97% but at least 7%. The method for providing the dialysis agent according to the first embodiment may also include a mixing step of mixing sodium acetate and glacial acetic acid to obtain an acetic acid mixture.

In the first embodiment, examples of the method used for the drying treatment for the magnesium chloride hydrate include one of, or a combination of, a heated drying method using heat, a reduced-pressure drying method performed in a state of reduced pressure, and a drying method using a desiccant or the like. From the viewpoint of the efficiency of the drying of the magnesium chloride hydrate, either a heated drying method using heat, or a combination of a heated drying method using heat and a reduced-pressure drying method performed in a state of reduced pressure is preferable. Further, from the viewpoint of improving the efficiency of the drying treatment, the magnesium chloride hydrate is preferably dried separately from the other components.

In the case of the heated drying method, the magnesium chloride hydrate drying treatment temperature is, for example, from 75°C to 110°C, and the drying time is, for example, from 15 minutes to 5 hours. In order to achieve a ratio between the water content of the entire dialysis agent and the amount of water removed from the magnesium chloride hydrate that is less than 118% but at least 7%, and a water content of the magnesium chloride hydrate that is within a range from 43% to 51%, it is preferable that the drying treatment temperature is 75°C or higher and the drying time is 15 minutes or longer, and it is more preferable that the drying treatment temperature is 100°C or higher and the drying time is 30 minutes or longer.

In order to prepare a dialysis fluid from the dialysis agent according to the first embodiment produced in the manner described above, the dialysis agent according to the first embodiment is treated as the "A agent" and dissolved in a prescribed amount of purified water to prepare an "A concentrate", the "B agent" is dissolved in a prescribed amount of purified water to prepare a "B concentrate", and the A concentrate and the B concentrate are then mixed together in a prescribed ratio and diluted. For example, the A agent for a dialysis agent of sample 7-1 of Example 7 is dissolved in sufficient purified water to prepare an A concentrate of 9 L, and 661.6 g of sodium bicarbonate as the B agent is dissolved in sufficient purified water to prepare a B concentrate of 11.34 L. By subsequently performing mixing in a ratio of A concentrate : B concentrate : purified water = 1:1.26:32.74, 315 L of a dialysis fluid can be prepared.

<Method for Providing Dialysis Agent according to Second Embodiment>

A method for providing the dialysis agent of the second embodiment comprising electrolytes containing at least magnesium chloride hydrate and calcium chloride hydrate, and a pH modifier is a method which comprises a drying treatment step of drying the magnesium chloride hydrate and the calcium chloride hydrate so that the ratio between the water content of the entire dialysis agent and the sum of the amount of water removed from the magnesium chloride hydrate and the amount of water removed from the calcium chloride hydrate becomes not more than 210% but at least 2%, the water content of the magnesium chloride hydrate becomes a value within a range from 43% to 51%, and the water content of the calcium chloride hydrate becomes a value within a range from 1% to 22%, and a filling step of placing each of the constituent components in a container as a single solid component. In the drying treatment step, the magnesium chloride hydrate and the calcium chloride hydrate are preferably dried so that the ratio between the water content of the entire dialysis agent and the sum of the amount of water removed from the magnesium chloride hydrate and the amount of water removed from the calcium chloride hydrate becomes less than 167% but at least 2%. The method for providing the dialysis agent according to the second embodiment may also include a mixing step of mixing sodium acetate and glacial acetic acid to obtain an acetic acid mixture.

In the second embodiment, examples of the method used for the drying treatment for the magnesium chloride hydrate and the calcium chloride hydrate include one of, or a combination of, a heated drying method using heat, a reduced-pressure drying method performed in a state of reduced pressure, and a drying method using a desiccant or the like. From the viewpoint of the efficiency of the drying of the magnesium chloride hydrate and the calcium chloride hydrate, either a heated drying method using heat, or a combination of a heated drying method using heat and a reduced-pressure drying method performed in a state of reduced pressure is preferable. Further, from the viewpoint of improving the efficiency of the drying treatment, the magnesium chloride hydrate and the calcium chloride hydrate are preferably dried separately from the other components. The magnesium chloride hydrate and the calcium chloride hydrate may be dried together or dried separately, but in terms of factors such as requiring a high degree of mixing of the calcium chloride hydrate and the magnesium chloride hydrate when the components are placed in the container, the magnesium chloride hydrate and the calcium chloride hydrate are preferably dried separately.

In the case of the heated drying method, the magnesium chloride hydrate and calcium chloride hydrate drying treatment temperature is, for example, from 75°C to 110°C, and the drying time is, for example, from 15 minutes to 5 hours. In order to achieve a ratio between the water content of the entire dialysis agent and the sum of the amount of water removed from the magnesium chloride hydrate and the amount of water removed from the calcium chloride hydrate that is not more than 210% but at least 2%, a water content of the magnesium chloride hydrate that is within a range

EP 2 939 694 B1

from 43% to 51%, and a water content of the calcium chloride hydrate that is within a range from 1% to 22%, it is preferable that the drying treatment temperature is 75°C or higher and the drying time is 15 minutes or longer, and it is more preferable that the drying treatment temperature is 100°C or higher and the drying time is 30 minutes or longer.

In order to prepare a dialysis fluid from the dialysis agent according to the second embodiment produced in the manner described above, the dialysis agent according to the second embodiment is treated as the "A agent" and dissolved in a prescribed amount of purified water to prepare an "A concentrate", the "B agent" is dissolved in a prescribed amount of purified water to prepare a "B concentrate", and the A concentrate and the B concentrate are then mixed together in a prescribed ratio and diluted. For example, the A agent for a dialysis agent of sample 16-1 of Example 16 is dissolved in sufficient purified water to prepare an A concentrate of 9 L, and 661.6 g of sodium bicarbonate as the B agent is dissolved in sufficient purified water to prepare a B concentrate of 11.34 L. By subsequently performing mixing in a ratio of A concentrate : B concentrate : purified water = 1:1.26:32.74, 315 L of a dialysis fluid can be prepared.

<Method for Providing Dialysis Agent according to Reference Embodiment 1>

A method for providing the dialysis agent of the reference embodiment 1 comprising electrolytes containing at least calcium chloride hydrate, and an acetic acid mixture composed of a mixture of sodium acetate and glacial acetic acid is a method which comprises a drying treatment step of drying the calcium chloride hydrate so that the ratio between the water content of the entire dialysis agent and the amount of water removed from the calcium chloride hydrate becomes less than 163% but at least 2%, and the water content of the calcium chloride hydrate becomes a value within a range from 1% to 22%, and a filling step of placing each of the constituent components in a container as a single solid component. In the drying treatment step, the calcium chloride hydrate is preferably dried so that the ratio between the water content of the entire dialysis agent and the amount of water removed from the calcium chloride hydrate becomes less than 82% but at least 2%. The method for providing the dialysis agent according to the reference embodiment 1 may also include a mixing step of mixing sodium acetate and glacial acetic acid to obtain the acetic acid mixture.

In the reference embodiment 1, examples of the method used for the drying treatment for the calcium chloride hydrate include one of, or a combination of, a heated drying method using heat, a reduced-pressure drying method performed in a state of reduced pressure, and a drying method using a desiccant or the like. From the viewpoint of the efficiency of the drying of the calcium chloride hydrate, either a heated drying method using heat, or a combination of a heated drying method using heat and a reduced-pressure drying method performed in a state of reduced pressure is preferable. Further, from the viewpoint of improving the efficiency of the drying treatment, the calcium chloride hydrate is preferably dried separately from the other components.

In the case of the heated drying method, the calcium chloride hydrate drying treatment temperature is, for example, from 75°C to 110°C, and the drying time is, for example, from 15 minutes to 5 hours. In order to achieve a ratio between the water content of the entire dialysis agent and the amount of water removed from the calcium chloride hydrate that is less than 163% but at least 2%, and a water content of the calcium chloride hydrate that is within a range from 1% to 22%, it is preferable that the drying treatment temperature is 75°C or higher and the drying time is 15 minutes or longer, and it is more preferable that the drying treatment temperature is 100°C or higher and the drying time is 30 minutes or longer.

In order to prepare a dialysis fluid from the dialysis agent according to the reference embodiment 1 produced in the manner described above, the dialysis agent according to the reference embodiment 1 is treated as the "A agent" and dissolved in a prescribed amount of purified water to prepare an "A concentrate", the "B agent" is dissolved in a prescribed amount of purified water to prepare a "B concentrate", and the A concentrate and the B concentrate are then mixed together in a prescribed ratio and diluted. For example, the A agent for a dialysis agent of sample 25-1 of Reference Example 7 is dissolved in sufficient purified water to prepare an A concentrate of 9 L, and 661.6 g of sodium bicarbonate as the B agent is dissolved in sufficient purified water to prepare a B concentrate of 11.34 L. By subsequently performing mixing in a ratio of A concentrate : B concentrate : purified water = 1:1.26:32.74, 315 L of a dialysis fluid can be prepared.

EXAMPLES

The present invention is described below in further detail using a series of examples and comparative examples, but the present invention is in no way limited by the following examples.

<Various Measurement Methods>

The various measurements in the examples were performed as follows.

[Water Content (%)]

The proportion (mass %) of water contained in a substance is defined as the water content. A more detailed description of the measurement method is provided below.

Measurements of the water content of sodium chloride, anhydrous glucose, potassium chloride, citric acid, sodium citrate and sodium acetate were performed in accordance with the water content measurement method disclosed in the 16th edition of The Japanese Pharmacopoeia.

Measurements of the water content of calcium chloride hydrate, magnesium chloride hydrate and powder acetic acid were performed in an atmosphere at a room temperature of 25°C±2.5°C and a relative humidity of 40%±2.5% in accordance with the Karl Fischer titration method, volumetric titration method and direct titration of JIS K0068. Using a Karl Fischer measuring device (manufactured by Kyoto Electronics Manufacturing Co., Ltd., model number: MKA-510), measurement was performed using 30 ml of methanol (manufactured by Kanto Chemical Co., Inc.) as the solvent. A Karl Fischer reagent (manufactured by Sigma Aldrich Japan K.K., product name: HYDRANAL-Composite 5) was used as the reagent for measuring the water content (reference: JIS K0068).

[Consolidation Rate]

The consolidation rate is defined as the ratio of the weight of consolidated matter relative to the total weight of all the components of the dialysis agent. The weight of consolidated matter is defined as the weight of the sample that does not pass through a test sieve (mesh size: 5.6 mm). A more detailed description of the measurement method is provided below.

A dialysis agent using magnesium chloride hydrate having a prescribed water content, a dialysis agent using calcium chloride hydrate having a prescribed water content, and a dialysis agent using both the magnesium chloride hydrate and the calcium chloride hydrate were each placed in a sealed container, and a 14-day stability test was performed in a constant temperature and humidity chamber (manufactured by Tabai Espec Corporation, model number: PR-3KP) under conditions of 40°C and a relative humidity of 75%. Following storage for 14 days, each dialysis agent was opened and poured onto a test sieve (mesh size: 5.6 mm) to confirm the amount (g) of consolidation.

[5-HMF Test]

A dialysis agent using magnesium chloride hydrate having a prescribed water content, a dialysis agent using calcium chloride hydrate having a prescribed water content, and a dialysis agent using both the magnesium chloride hydrate and the calcium chloride hydrate were each placed in a sealed container, and a 14-day stability test was performed in a constant temperature and humidity chamber (manufactured by Tabai Espec Corporation, model number: PR-3KP) under conditions of 40°C and a relative humidity of 75%. Following storage for 14 days, each dialysis agent was opened, and the dialysis agent was dissolved in RO water. In order to measure the rate of degradation of the anhydrous glucose in the dissolved dialysis agent A concentrate, the absorbance at a wavelength of 284 nm was measured based on the UV-VIS absorbance measurement method for testing the purity of glucose injection solutions described in the 16th edition of The Japanese Pharmacopoeia.

<Drying of Magnesium chloride hydrate>

The drying treatment for magnesium chloride hydrate was performed in the manner described below.

[Heated Drying]

Fifty grams of magnesium chloride hexahydrate ($MgCl_2 \cdot 6H_2O$) was placed in a stainless steel rectangular vat (external dimensions (mm): 210×170×31, bottom dimensions (mm): 170×130, SUS304), smoothed out to a uniform level, and then dried by heating at a prescribed temperature for a prescribed time. Following drying, the magnesium chloride hydrate was passed through sieves of 2,000 μm and 212 μm, and only the sample retained on the 212 μm sieve was used. The drying temperature was set to 100°C. The drying time was set to 30 minutes, 1 hour, 1.5 hours or 3.5 hours. The dryer used was a model DK600T manufactured by Yamato Scientific Co., Ltd.

The water content was measured for each of the obtained dried samples ($MgCl_2$), and for the magnesium chloride hexahydrate that had not been subjected to a drying treatment. The results are shown in Table 1.

[Table 1]

Table 1 (Magnesium chloride hydrate water content when magnesium chloride hexahydrate was dried at 100°C for a prescribed time)

| | Temperature | Time | Water content |
|---|---|---|---|
| Magnesium chloride hydrate (43%) | 100°C | 3.5hr | 43% |
| Magnesium chloride hydrate (45%) | 100°C | 1.5hr | 45% |
| Magnesium chloride hydrate (47%) | 100°C | 1.0hr | 47% |
| Magnesium chloride hydrate (51%) | 100°C | 0.5hr | 51% |
| Magnesium chloride hexahydrate | - | - | 53.2% |

<Drying of Calcium chloride hydrate>

The drying treatment for calcium chloride hydrate was performed in the manner described below.

[Heated Drying]

Fifty grams of calcium chloride dihydrate ($CaCl_2 \cdot 2H_2O$) was placed in a stainless steel rectangular vat (external dimensions (mm): 210×170×31, bottom dimensions (mm): 170×130, SUS304), smoothed out to a uniform level, and then dried by heating at a prescribed temperature for a prescribed time. Following drying, the calcium chloride hydrate was passed through sieves of 2,000 $\mu$m and 212 $\mu$m, and only the sample retained on the 212 $\mu$m sieve was used. The drying temperature was set to 100°C or 180°C. The drying time was set to 30 minutes, 1 hour or 3.5 hours at 100°C, and set to 2 hours at 180°C. The dryer used was a model DK600T manufactured by Yamato Scientific Co., Ltd.
The water content was measured for each of the obtained dried samples ($CaCl_2$), and for the calcium chloride dihydrate that had not been subjected to a drying treatment. The results are shown in Table 2.

[Table 2]

Table 2 (Calcium chloride hydrate water content when calcium chloride dihydrate was dried at a prescribed temperature for a prescribed time)

| | Temperature | Time | Water content |
|---|---|---|---|
| Calcium chloride hydrate (1%) | 180°C | 2.0hr | 1% |
| Calcium chloride hydrate (15%) | 100°C | 3.5hr | 15% |
| Calcium chloride hydrate (19%) | 100°C | 1.0hr | 19% |
| Calcium chloride hydrate (22%) | 100°C | 0.5hr | 22% |
| Calcium chloride dihydrate | - | - | 24.5% |

<Example 1>

Using magnesium chloride hydrate ($MgCl_2$) (water content: 43%, 45%, 47%, 51%) that had been subjected to a drying step to provide improved functionality as a desiccant, sodium chloride, potassium chloride, calcium chloride dihydrate, anhydrous citric acid, anhydrous sodium citrate, anhydrous glucose, and the magnesium chloride hydrate were placed in a container in a ratio shown in Table 3, and the container was sealed to complete preparation of a series of dialysis agents (samples 1-1 to 1-4).
The amount of water of adhesion in each of the sodium chloride, the potassium chloride, the calcium chloride dihydrate, the anhydrous citric acid, the anhydrous sodium citrate and the anhydrous glucose was measured using the water content measurement method described above to determine the amount of water of adhesion. The results are shown in Table 3.
A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 5. Further, the results of calculating the amount of water removed from the magnesium chloride hydrate and the water content of the entire dialysis agent, and then calculating the ratio M are shown in Table 5.

[Table 3]

Table 3 (Blend amounts in Example 1)

|  | Sample 1-1 | Sample 1-2 | Sample 1-3 | Sample 1-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Anhydrous citric acid | 34.3g | 34.3g | 34.3g | 34.3g |
| Anhydrous sodium citrate | 8.1g | 8.1g | 8.1g | 8.1g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 4]

Table 4 (Amounts of water of adhesion in Example 1)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Anhydrous citric acid | 0.03% | 0.00% | 0.03% | 34.3g | 0.01g |
| Anhydrous sodium citrate | 0.56% | 0.00% | 0.56% | 8.1g | 0.05g |
| Anhydrous glucose | 0.05% | 0.00% | 0.05% | 472.5g | 0.24g |
|  |  |  |  | Water content due to water of adhesion | Total amount of water of adhesion |
|  |  |  |  | 0.04% | 0.93g |

[Table 5]

Table 5 (M value, consolidation, and 5-HMF in Example 1)

| | Sample 1-1 | Sample 1-2 | Sample 1-3 | Sample 1-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2591.0g | 2591.8g | 2592.9g | 2595.2g |
| Amount of water of adhesion | 0.93g | 0.93g | 0.93g | 0.93g |
| Weight of efflorescent components | - | - | - | - |
| Theoretical water content of efflorescent components | - | - | - | - |
| Amount of water of crystallization | 0.00g | 0.00g | 0.00g | 0.00g |
| Water content of powder dialysis agent | 0.93g | 0.93g | 0.93g | 0.93g |
| M value | 16% | 19% | 25% | 65% |
| Weight of consolidation | 0.0g | 0.0g | 0.0g | 0.0g |
| Consolidation rate | 0.0% | 0.0% | 0.0% | 0.0% |
| 5-HMF | 0.007 | 0.009 | 0.009 | 0.010 |

<Example 2>

As shown in Table 6, with the exception of using a combination of anhydrous citric acid and sodium citrate dihydrate as the pH modifying component instead of the combination of anhydrous citric acid and anhydrous sodium citrate, evaluations were performed in the same manner as Example 1. The results are shown in Tables 7 and 8.

[Table 6]

Table 6 (Blend amounts in Example 2)

| | Sample 2-1 | Sample 2-2 | Sample 2-3 | Sample 2-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Anhydrous citric acid | 34.3g | 34.3g | 34.3g | 34.3g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 7]

Table 7 (Amounts of water of adhesion in Example 2)

| | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Anhydrous citric acid | 0.03% | 0.00% | 0.03% | 34.3g | 0.01g |
| Sodium citrate dihydrate | 12.26% | 12.25% | 0.01% | 9.3g | 0.00g |
| Anhydrous glucose | 0.05% | 0.00% | 0.05% | 472.5g | 0.24g |
| | | | | Water content due to water of adhesion | Total amount of water of adhesion |
| | | | | 0.03% | 0.88g |

[Table 8]

Table 8 (M value, consolidation, and 5-HMF in Example 2)

| | Sample 2-1 | Sample 2-2 | Sample 2-3 | Sample 2-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2592.2g | 2593.0g | 2594.1g | 2596.4g |
| Amount of water of adhesion | 0.88g | 0.88g | 0.88g | 0.88g |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 1.14g | 1.14g | 1.14g | 1.14g |
| Water content of powder dialysis agent | 2.02g | 2.02g | 2.02g | 2.02g |
| M value | 36% | 42% | 54% | 142% |

(continued)

|  | Sample 2-1 | Sample 2-2 | Sample 2-3 | Sample 2-4 |
|---|---|---|---|---|
| Weight of consolidation | 0.0g | 0.0g | 0.0g | 397.8g |
| Consolidation rate | 0.0% | 0.0% | 0.0% | 15.3% |
| 5-HMF | 0.005 | 0.006 | 0.005 | 0.079 |

<Example 3>

As shown in Table 9, with the exception of using a combination of citric acid monohydrate and anhydrous sodium citrate as the pH modifying component instead of the combination of anhydrous citric acid and anhydrous sodium citrate, evaluations were performed in the same manner as Example 1. The results are shown in Tables 10 and 11.

[Table 9]

Table 9 (Blend amounts in Example 3)

|  | Sample 3-1 | Sample 3-2 | Sample 3-3 | Sample 3-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Citric acid monohydrate | 37.5g | 37.5g | 37.5g | 37.5g |
| Anhydrous sodium citrate | 8.1g | 8.1g | 8.1g | 8.1g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 10]

Table 10 (Amounts of water of adhesion in Example 3)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Citric acid monohydrate | 9.26% | 8.58% | 0.68% | 37.5g | 0.26g |

(continued)

| | Measured water content | Theoretical water content | Water content due to water of adhesion | Weight of each component | Amount of water of adhesion in each component |
|---|---|---|---|---|---|
| Anhydrous sodium citrate | 0.56% | 0.00% | 0.56% | 8.1g | 0.05g |
| Anhydrous glucose | 0.05% | 0.00% | 0.05% | 472.5g | 0.24g |
| | | | | Water content due to water of adhesion | Total amount of water of adhesion |
| | | | | 0.05% | 1.17g |

[Table 11]

Table 11 (M value, consolidation, and 5-HMF in Example 3)

| | Sample 3-1 | Sample 3-2 | Sample 3-3 | Sample 3-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2594.2g | 2595.0g | 2596.1g | 2598.4g |
| Amount of water of adhesion | 1.17g | 1.17g | 1.17g | 1.17g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Amount of water of crystallization | 3.23g | 3.23g | 3.23g | 3.23g |
| Water content of powder dialysis agent | 4.40g | 4.40g | 4.40g | 4.40g |
| M value | 78% | 92% | 118% | 310% |
| Weight of consolidation | 0.0g | 0.0g | 226.5g | 345.8g |
| Consolidation rate | 0.0% | 0.0% | 8.7% | 13.3% |
| 5-HMF | 0.006 | 0.007 | 0.078 | 0.077 |

<Example 4>

As shown in Table 12, with the exception of using a combination of citric acid monohydrate and sodium citrate dihydrate as the pH modifying component instead of the combination of anhydrous citric acid and anhydrous sodium citrate, evaluations were performed in the same manner as Example 1. The results are shown in Tables 13 and 14.

[Table 12]

Table 12 (Blend amounts in Example 4)

|  | Sample 4-1 | Sample 4-2 | Sample 4-3 | Sample 4-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 13]

Table 13 (Amounts of water of adhesion in Example 4)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Citric acid monohydrate | 9.26% | 8.58% | 0.68% | 37.5g | 0.26g |
| Sodium citrate dihydrate | 12.26% | 12.25% | 0.01% | 9.3g | 0.00g |
| Anhydrous glucose | 0.05% | 0.00% | 0.05% | 472.5g | 0.24g |
|  |  |  |  | Water content due to water of adhesion | Total amount of water of adhesion |
|  |  |  |  | 0.04% | 1.13g |

[Table 14]

Table 14 (M value, consolidation, and 5-HMF in Example 4)

| | Sample 4-1 | Sample 4-2 | Sample 4-3 | Sample 4-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2595.4g | 2596.2g | 2597.3g | 2599.6g |
| Amount of water of adhesion | 1.13g | 1.13g | 1.13g | 1.13g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 5.50g | 5.50g | 5.50g | 5.50g |
| M value | 97% | 115% | 148% | 387% |
| Weight of consolidation | 3.6g | 4.2g | 190.9g | 207.9g |
| Consolidation rate | 0.1% | 0.2% | 7.3% | 8.0% |
| 5-HMF | 0.074 | 0.077 | 0.080 | 0.081 |

<Example 5>

With the exception of altering the blend amount of magnesium chloride hydrate as shown in Table 15, evaluations were performed in the same manner as Example 4. The results are shown in Tables 16 and 17.

[Table 15]

Table 15 (Blend amounts in Example 5)

| | Sample 5-1 | Sample 5-2 | Sample 5-3 | Sample 5-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 6.6g | - | - | - |

(continued)

|  | Sample 5-1 | Sample 5-2 | Sample 5-3 | Sample 5-4 |
|---|---|---|---|---|
| Magnesium chloride hydrate (45%) | - | 6.8g | - | - |
| Magnesium chloride hydrate (47%) | - | - | 7.1g | - |
| Magnesium chloride hydrate (51%) | - | - | - | 7.7g |

[Table 16]

Table 16 (Amounts of water of adhesion in Example 5)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Citric acid monohydrate | 9.26% | 8.58% | 0.68% | 37.5g | 0.26g |
| Sodium citrate dihydrate | 12.26% | 12.25% | 0.01% | 9.3g | 0.00g |
| Anhydrous glucose | 0.05% | 0.00% | 0.05% | 472.5g | 0.24g |
|  |  |  |  | Water content due to water of adhesion | Total amount of water of adhesion |
|  |  |  |  | 0.04% | 1.13g |

[Table 17]

Table 17 (M value, consolidation, and 5-HMF in Example 5)

|  | Sample 5-1 | Sample 5-2 | Sample 5-3 | Sample 5-4 |
|---|---|---|---|---|
| Weight of magnesium chloride hexahydrate | 8.0g | 8.0g | 8.0g | 8.0g |
| Theoretical water content of magnesium chloride hexahydrate | 53.2% | 53.2% | 53.2% | 53.2% |
| Weight of dried magnesium chloride hydrate | 6.6g | 6.8g | 7.1g | 7.7g |
| Measured water content of dried magnesium chloride hydrate | 43% | 45% | 47% | 51% |
| Amount of water removed from magnesium chloride hydrate | 1.42g | 1.20g | 0.92g | 0.33g |

(continued)

|  | Sample 5-1 | Sample 5-2 | Sample 5-3 | Sample 5-4 |
|---|---|---|---|---|
| Total weight of all components | 2575.6g | 2575.8g | 2576.1g | 2576.7g |
| Amount of water of adhesion | 1.13g | 1.13g | 1.13g | 1.13g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 5.50g | 5.50g | 5.50g | 5.50g |
| M value | 387% | 458% | 598% | 1667% |
| Weight of consolidation | 216.8g | 240.7g | 233.7g | 245.8g |
| Consolidation rate | 8.4% | 9.3% | 9.1% | 9.5% |
| 5-HMF | 0.098 | 0.103 | 0.092 | 0.098 |

<Example 6>

With the exception of altering the blend amount of magnesium chloride hydrate as shown in Table 18, evaluations were performed in the same manner as Example 4. The results are shown in Tables 19 and 20.

[Table 18]

Table 18 (Blend amounts in Example 6)

|  | Sample 6-1 | Sample 6-2 | Sample 6-3 | Sample 6-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Calcium chloride dihydrate | 69.5g | 69.5g | 69.5g | 69.5g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 39.6g | - | - | - |
| Magnesium chloride hydrate (45%) | - | 40.8g | - | - |
| Magnesium chloride hydrate (47%) | - | - | 42.5g | - |
| Magnesium chloride hydrate (51%) | - | - | - | 45.9g |

[Table 19]

Table 19 (Amounts of water of adhesion in Example 6)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.2g | 0.19g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Citric acid monohydrate | 9.26% | 8.58% | 0.68% | 37.5g | 0.26g |
| Sodium citrate dihydrate | 12.26% | 12.25% | 0.01% | 9.3g | 0.00g |
| Anhydrous glucose | 0.05% | 0.00% | 0.05% | 472.5g | 0.24g |
|  |  |  |  | Water content due to water of adhesion | Total amount of water of adhesion |
|  |  |  |  | 0.04% | 1.13g |

[Table 20]

Table 20 (M value, consolidation, and 5-HMF in Example 6)

|  | Sample 6-1 | Sample 6-2 | Sample 6-3 | Sample 6-4 |
|---|---|---|---|---|
| Weight of magnesium chloride hexahydrate | 48.0g | 48.0g | 48.0g | 48.0g |
| Theoretical water content of magnesium chloride hexahydrate | 53.2% | 53.2% | 53.2% | 53.2% |
| Weight of dried magnesium chloride hydrate | 39.6g | 40.8g | 42.5g | 45.9g |
| Measured water content of dried magnesium chloride hydrate | 43% | 45% | 47% | 51% |
| Amount of water removed from magnesium chloride hydrate | 8.51g | 7.18g | 5.56g | 2.13g |
| Total weight of all components | 2608.6g | 2609.8g | 2611.5g | 2614.9g |
| Amount of water of adhesion | 1.13g | 1.13g | 1.13g | 1.13g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |

(continued)

|  | Sample 6-1 | Sample 6-2 | Sample 6-3 | Sample 6-4 |
|---|---|---|---|---|
| Amount of water of crystallization | 4.37g | 4.37g | 4.37g | 4.37g |
| Water content of powder dialysis agent | 5.50g | 5.50g | 5.50g | 5.50g |
| M value | 65% | 77% | 99% | 258% |
| Weight of consolidation | 0.0g | 0.0g | 0.0g | 220.9g |
| Consolidation rate | 0.0% | 0.0% | 0.0% | 8.4% |
| 5-HMF | 0.007 | 0.007 | 0.077 | 0.079 |

<Example 7>

As shown in Table 21, with the exception of using a combination of powder acetic acid and anhydrous sodium acetate as the pH modifying component instead of the combination of anhydrous citric acid and anhydrous sodium citrate, evaluations were performed in the same manner as Example 1. The results are shown in Tables 22 and 23. The powder acetic acid was obtained by mixing anhydrous sodium acetate and glacial acetic acid in a molar ratio of 1:1.

[Table 21]

Table 21 (Blend amounts in Example 7)

|  | Sample 7-1 | Sample 7-2 | Sample 7-3 | Sample 7-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Calcium chloride dihydrate | 69.5g | 69.5g | 69.5g | 69.5g |
| Powder acetic acid | 99.4g | 99.4g | 99.4g | 99.4g |
| Anhydrous sodium acetate | 149.4g | 149.4g | 149.4g | 149.4g |
| Anhydrous glucose | 315.Og | 315.Og | 315.Og | 315.Og |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 22]

Table 22 (Amounts of water of adhesion in Example 7)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.8g | 0.20g |
| Potassium chloride | 0.02% | 0.00% | 0.02% | 47.0g | 0.01g |
| Calcium chloride dihydrate | 25.13% | 24.51% | 0.62% | 69.5g | 0.43g |

(continued)

| | Measured water content | Theoretical water content | Water content due to water of adhesion | Weight of each component | Amount of water of adhesion in each component |
|---|---|---|---|---|---|
| Powder acetic Acid | 0.21% | 0.00% | 0.21% | 99.4g | 0.21g |
| Anhydrous sodium acetate | 0.21% | 0.00% | 0.21% | 149.4g | 0.31g |
| Anhydrous glucose | 0.05% | 0.00% | 0.05% | 315.Og | 0.16g |
| | | | | Water content due to water of adhesion | Total amount of water of adhesion |
| | | | | 0.05% | 1.32g |

[Table 23]

Table 23 (M value, consolidation, and 5-HMF in Example 7)

| | Sample 7-1 | Sample 7-2 | Sample 7-3 | Sample 7-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2676.5g | 2677.3g | 2678.4g | 2680.7g |
| Amount of water of adhesion | 1.32g | 1.32g | 1.32g | 1.32g |
| Weight of efflorescent components | - | - | - | - |
| Theoretical water content of efflorescent components | - | - | - | - |
| Amount of water of crystallization | 0.00g | 0.00g | 0.00g | 0.00g |
| Water content of powder dialysis agent | 1.32g | 1.32g | 1.32g | 1.32g |
| M value | 23% | 28% | 36% | 93% |
| Weight of consolidation | 0.0g | 0.0g | 0.0g | 0.0g |
| Consolidation rate | 0.0% | 0.0% | 0.0% | 0.0% |
| 5-HMF | 0.004 | 0.004 | 0.004 | 0.004 |

<Example 8>

As shown in Table 24, with the exception of using a combination of powder acetic acid and sodium acetate trihydrate as the pH modifying component instead of the combination of anhydrous citric acid and anhydrous sodium citrate, evaluations were performed in the same manner as Example 1. The results are shown in Tables 25 and 26.

[Table 24]

Table 24 (Blend amounts in Example 8)

| | Sample 8-1 | Sample 8-2 | Sample 8-3 | Sample 8-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Calcium chloride dihydrate | 69.5g | 69.5g | 69.5g | 69.5g |
| Powder acetic acid | 99.4g | 99.4g | 99.4g | 99.4g |
| Sodium acetate trihydrate | 247.8g | 247.8g | 247.8g | 247.8g |
| Anhydrous glucose | 315.0g | 315.0g | 315.0g | 315.0g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |

[Table 25]

Table 25 (Amounts of water of adhesion in Example 8)

| | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.8g | 0.20g |
| Potassium chloride | 0.02% | 0.00% | 0.02% | 47.0g | 0.01g |
| Calcium chloride dihydrate | 25.13% | 24.51% | 0.62% | 69.5g | 0.43g |
| Powder acetic Acid | 0.21% | 0.00% | 0.21% | 99.4g | 0.21g |
| Sodium acetate trihydrate | 39.89% | 39.72% | 0.17% | 247.8g | 0.42g |
| Anhydrous glucose | 0.05% | 0.00% | 0.05% | 315.0g | 0.16g |
| | | | | Water content due to water of adhesion | Total amount of water of adhesion |
| | | | | 0.05% | 1.42g |

[Table 26]

Table 26 (M value, consolidation, and 5-HMF in Example 8)

| | Sample 8-1 | Sample 8-2 | Sample 8-3 | Sample 8-4 |
|---|---|---|---|---|
| Weight of magnesium chloride hexahydrate | 32.0g | 32.0g | 32.0g | 32.0g |

(continued)

|  | Sample 8-1 | Sample 8-2 | Sample 8-3 | Sample 8-4 |
|---|---|---|---|---|
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Total weight of all components | 2774.9g | 2775.7g | 2776.8g | 2779.1g |
| Amount of water of adhesion | 1.42g | 1.42g | 1.42g | 1.42g |
| Weight of efflorescent component (sodium acetate trihydrate) | 247.8g | 247.8g | 247.8g | 247.8g |
| Theoretical water content of efflorescent component (sodium acetate trihydrate) | 39.7% | 39.7% | 39.7% | 39.7% |
| Amount of water of crystallization | 98.38g | 98.38g | 98.38g | 98.38g |
| Water content of powder dialysis agent | 99.80g | 99.80g | 99.80g | 99.80g |
| M value | 1760% | 2088% | 2683% | 7028% |
| Weight of consolidation | 783.8g | 800.9g | 784.5g | 792.6g |
| Consolidation rate | 28.2% | 28.9% | 28.3% | 28.5% |
| 5-HMF | 0.005 | 0.004 | 0.002 | 0.005 |

The concentration of each component when each sample was dissolved in RO water and made up to 315 L is shown in Table 27.

[Table 27]

Table 27 (Concentration when each sample was dissolved in RO water and made up to 315 L)

|  | Examples 1 to 4 | Example 5 | Example 6 | Examples 7 and 8 |
|---|---|---|---|---|
| Na+ | 105.3mEq/L | 105.3mEq/L | 105.3mEq/L | 115.0mEq/L |
| K+ | 2.0mEq/L | 2.0mEq/L | 2.0mEq/L | 2.0mEq/L |
| Ca++ | 3.0mEq/L | 3.0mEq/L | 3.0mEq/L | 3.0mEq/L |
| Mg++ | 1.0mEq/L | 0.25mEq/L | 1.5mEq/L | 1.0mEq/L |
| Cl- | 111.0mEq/L | 110.3mEq/L | 111.5mEq/L | 113.0mEq/L |
| Citrate ion | 2.0mEq/L | 2.0mEq/L | 2.0mEq/L | 0.0mEq/L |
| Acetate ion | 0.0mEq/L | 0.0mEq/L | 0.0mEq/L | 10.2mEq/L |
| Anhydrous glucose | 150.0mg/dl | 150.0mg/dl | 150.0mg/dl | 100.0mg/dl |

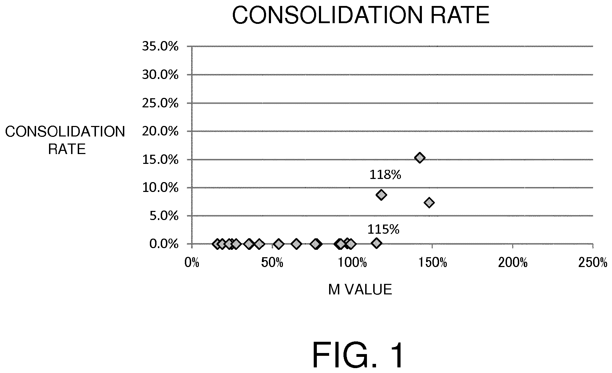

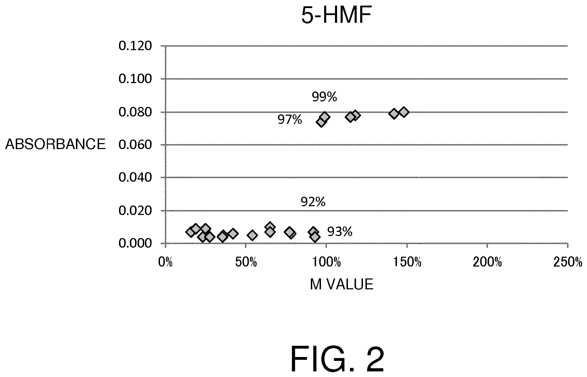

Combining the above results, the relationship between the ratio M and the consolidation rate is shown in FIG. 1, and the relationship between the ratio M and the 5-HMF absorbance is shown in FIG. 2.

In this manner, by ensuring that the ratio M between the water content of the entire dialysis agent and the amount of water removed from the magnesium chloride hydrate was less than 118% but at least 7%, consolidation and aggregation of the chemical agent were suppressed in the stability test. Further, by ensuring that the ratio between the water content of the entire dialysis agent and the amount of water removed from the magnesium chloride hydrate was less than 97% but at least 7%, production of 5-HMF was inhibited in the stability test.

<Example 9>

[Preparation of Acetic Acid Mixtures]

Anhydrous sodium acetate (38.8 g) was placed in a container, and glacial acetic acid (28.4 g) was added and mixed at a rate of addition of 6 mL/minute, thus obtaining an acetic acid mixture (1.5:1.5) composed of a mixture of anhydrous sodium acetate and glacial acetic acid (anhydrous sodium acetate : glacial acetic acid (molar ratio) = 1.5:1.5).
Anhydrous sodium acetate (77.5 g) was placed in a container, and glacial acetic acid (56.7 g) was added and mixed at a rate of addition of 6 mL/minute, thus obtaining an acetic acid mixture (3:3) composed of a mixture of anhydrous sodium acetate and glacial acetic acid (anhydrous sodium acetate : glacial acetic acid (molar ratio) = 3:3).
Anhydrous sodium acetate (154.8 g) was placed in a container, and glacial acetic acid (37.8 g) was added and mixed at a rate of addition of 6 mL/minute, thus obtaining an acetic acid mixture (6:2) composed of a mixture of anhydrous sodium acetate and glacial acetic acid (anhydrous sodium acetate : glacial acetic acid (molar ratio) = 6:2).
Anhydrous sodium acetate (206.7 g) was placed in a container, and glacial acetic acid (42.0 g) was added and mixed at a rate of addition of 6 mL/minute, thus obtaining an acetic acid mixture (8:2.2) composed of a mixture of anhydrous sodium acetate and glacial acetic acid (anhydrous sodium acetate : glacial acetic acid (molar ratio) = 8:2.2).
Anhydrous sodium acetate (258.4 g) was placed in a container, and glacial acetic acid (37.8 g) was added and mixed at a rate of addition of 6 mL/minute, thus obtaining an acetic acid mixture (10:2) composed of a mixture of anhydrous sodium acetate and glacial acetic acid (anhydrous sodium acetate : glacial acetic acid (molar ratio) = 10:2).
An X-ray diffraction method confirmed that the anhydrous sodium acetate and all or a portion of the glacial acetic acid had formed a complex.
Using magnesium chloride hydrate ($MgCl_2$) (water content: 45%) that had been subjected to a drying step to provide improved functionality as a desiccant, sodium chloride, potassium chloride, calcium chloride dihydrate, anhydrous glucose, the magnesium chloride hydrate, and a pH modifier (any one of the acetic acid mixture (1.5:1.5), the acetic acid mixture (3:3), the acetic acid mixture (6:2), the acetic acid mixture (8:2.2), the acetic acid mixture (10:2), or a combination of anhydrous sodium acetate and glacial acetic acid) were placed in a container in a ratio shown in Table 28, the container was sealed, and the container contents were dispersed to complete preparation of a series of dialysis agents (samples 9-1 to 9-6).
The amount of water of adhesion in each of the sodium chloride, the potassium chloride, the calcium chloride dihydrate, the anhydrous glucose and the pH modifier was measured using the water content measurement method described above to determine the amount of water of adhesion. The results are shown in Table 29.
A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 30. Further, the results of calculating the amount of water removed from the magnesium chloride hydrate and the water content of the entire dialysis agent, and then calculating the ratio M are shown in Table 30.

[Table 28]

Table 28 (Blend amounts in Example 9)

|  | Sample 9-1 | Sample 9-2 | Sample 9-3 | Sample 9-4 | Sample 9-5 | Sample 9-6 |
|---|---|---|---|---|---|---|
| Sodium chloride | 1969.8g | 1969.8g | 1969.8g | 1969.8g | 1969.8g | 1969.8g |
| Potassium chloride | 47.0g | 47.0g | 47.0g | 47.0g | 47.0g | 47.0g |
| Calcium chloride dihydrate | 69.5g | 69.5g | 69.5g | 69.5g | 69.5g | 69.5g |
| Acetic acid mixture (1.5:1.5) | 67.1g | - | - | - | - | - |
| Acetic acid mixture (3:3) | - | 134.2g | - | - | - | - |
| Acetic acid mixture (6:2) |  | - | 192.6g | - | - | - |
| Acetic acid mixture (8:2.2) | - | - | - | 248.7g |  | - |

(continued)

| | Sample 9-1 | Sample 9-2 | Sample 9-3 | Sample 9-4 | Sample 9-5 | Sample 9-6 |
|---|---|---|---|---|---|---|
| Acetic acid mixture (10:2) | | - | - | - | 296.2g | - |
| Glacial acetic acid | - | - | - | - | - | 42.0g |
| Anhydrous sodium acetate | - | - | - | - | - | 206.7g |
| Anhydrous glucose | 315.0g | 315.0g | 315.0g | 315.0g | 315.0g | 315.0g |
| Magnesium chloride hydrate (45%) | 27.2g | 27.2g | 27.2g | 27.2g | 27.2g | 27.2g |

[Table 29]

Table 29 (Amounts of water of adhesion in Example 9)

| | Measured water content | Theoretical water content | Water content due to water of adhesion | Weight of each component | Amount of water of adhesion in each component |
|---|---|---|---|---|---|
| Sodium chloride | 0.03% | 0.00% | 0.03% | 1969.80g | 0.59g |
| Potassium chloride | 0.02% | 0.00% | 0.02% | 47.00g | 0.01g |
| Calcium chloride dihydrate | 25.13% | 24.51% | 0.62% | 69.5g | 0.43g |
| Acetic acid mixture (1.5:1.5) | 0.21% | 0.00% | 0.21% | 67.1g | 0.14g |
| Acetic acid mixture (3:3) | 0.21% | 0.00% | 0.21% | 134.2g | 0.28g |
| Acetic acid mixture (6:2) | 0.19% | 0.00% | 0.19% | 192.6g | 0.37g |
| Acetic acid mixture (8:2.2) | 0.18% | 0.00% | 0.18% | 248.7g | 0.45g |
| Acetic acid mixture (10:2) | 0.16% | 0.00% | 0.16% | 296.2g | 0.47g |
| Glacial acetic acid | 0.07% | 0.00% | 0.07% | 42.0g | 0.03g |
| Anhydrous sodium acetate | 0.21% | 0.00% | 0.21% | 206.7g | 0.43g |
| Anhydrous glucose | 0.05% | 0.00% | 0.05% | 315.00g | 0.16g |

[Table 30]

Table 30 (M value, consolidation, and 5-HMF in Example 9)

| | Sample 9-1 | Sample 9-2 | Sample 9-3 | Sample 9-4 | Sample 9-5 | Sample 9-6 |
|---|---|---|---|---|---|---|
| Weight of magnesium chloride hexahydrate | 32.0g | 32.0g | 32.0g | 32.0g | 32.0g | 32.0g |

(continued)

| | Sample 9-1 | Sample 9-2 | Sample 9-3 | Sample 9-4 | Sample 9-5 | Sample 9-6 |
|---|---|---|---|---|---|---|
| Theoretical water content of magnesium chloride hexahydrate | 53.2% | 53.2% | 53.2% | 53.2% | 53.2% | 53.2% |
| Weight of dried magnesium chloride hydrate | 27.2g | 27.2g | 27.2g | 27.2g | 27.2g | 27.2g |
| Measured water content of dried magnesium chloride hydrate | 45% | 45% | 45% | 45% | 45% | 45% |
| Amount of water removed from magnesium chloride hydrate | 4.78g | 4.78g | 4.78g | 4.78g | 4.78g | 4.78g |
| Total weight of all components | 2495.6g | 2562.7g | 2621.1g | 2677.2g | 2724.7g | 2677.2g |
| Amount of water of adhesion | 1.33g | 1.47g | 1.55g | 1.64g | 1.66g | 1.65g |
| Weight of efflorescent components | - | - | - | - | - | - |
| Theoretical water content of efflorescent components | - | - | - | - | - | - |
| Amount of water of crystallization | 0.00g | 0.00g | 0.00g | 0.00g | 0.00g | 0.00g |
| Water content of powder dialysis agent | 1.33g | 1.47g | 1.55g | 1.64g | 1.66g | 1.65g |
| M value | 28% | 31% | 32% | 34% | 35% | 35% |
| Weight of consolidation | 1.2g | 1.1g | 1.3g | 1.0g | 1.3g | 429.7g |
| Consolidation rate | 0.0% | 0.0% | 0.0% | 0.0% | 0.0% | 16.1% |
| 5-HMF | 0.005 | 0.005 | 0.005 | 0.005 | 0.005 | 0.080 |

In this manner, by using an acetic acid mixture composed of a mixture of anhydrous sodium acetate and glacial acetic acid as the pH modifier, rather than a simple combination of anhydrous sodium acetate and glacial acetic, consolidation and aggregation of the chemical agent were suppressed in the stability test. Further, production of 5-HMF was inhibited in the stability test. In Sample 9-6, in which a dialysis agent was produced using anhydrous sodium acetate and glacial acetic acid as pH modifiers, significantly more consolidation of the chemical agent and degradation of the anhydrous glucose occurred compared with the case where an acetic acid mixture was used.

The concentration of each component when Samples 9-1 to 9-6 were each dissolved in RO water and made up to 315 L is shown in Table 31.

[Table 31]

Table 31 (Concentration when each sample was dissolved in RO water and made up to 315 L)

| | Sample 9-1 | Sample 9-2 | Sample 9-3 | Sample 9-4 | Sample 9-5 | Sample 9-6 |
|---|---|---|---|---|---|---|
| Na+ | 108.5mEq/L | 110.0mEq/L | 113.0mEq/L | 115.0mEq/L | 117.0mEq/L | 115.0mEq/L |
| K+ | 2.0mEq/L | 2.0mEq/L | 2.0mEq/L | 2.0mEq/L | 2.0mEq/L | 2.0mEq/L |
| Ca++ | 3.0mEq/L | 3.0mEq/L | 3.0mEq/L | 3.0mEq/L | 3.0mEq/L | 3.0mEq/L |
| Mg++ | 1.0mEq/L | 1.0mEq/L | 1.0mEq/L | 1.0mEq/L | 1.0mEq/L | 1.0mEq/L |

(continued)

|  | Sample 9-1 | Sample 9-2 | Sample 9-3 | Sample 9-4 | Sample 9-5 | Sample 9-6 |
|---|---|---|---|---|---|---|
| Cl- | 113.0mEq/ L | 113.0mEq/ L | 113.0mEq/ L | 113.0mEq/ L | 113.0mEq/ L | 113.0mEq/ L |
| Acetate ion | 3.0mEq/L | 6.0mEq/L | 8.0mEq/L | 10.2mEq/L | 12.0mEq/L | 10.2mEq/L |
| Anhydrous glucose | 100.0mg/dl | 100.0mg/dl | 100.0mg/dl | 100.0mg/dl | 100.0mg/dl | 100.0mg/dl |

<Example 10>

Using magnesium chloride hydrate ($MgCl_2$) (water content: 43%, 45%, 47%, 51%) and calcium chloride hydrate ($CaCl_2$) (water content: 1%, 15%, 19%, 22%) that had been subjected to drying steps to provide improved functionality as desiccants, sodium chloride, potassium chloride, anhydrous citric acid, anhydrous sodium citrate, anhydrous glucose and the calcium chloride hydrate were placed in a container in a ratio shown in Table 32, and the container was sealed to complete preparation of a series of dialysis agents (samples 10-1 to 10-4).

The amount of water of adhesion in each of the sodium chloride, the potassium chloride, the anhydrous citric acid, the anhydrous sodium citrate and the anhydrous glucose was measured using the water content measurement method described above to determine the amount of water of adhesion. The results are shown in Table 33.

A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 34. Further, the results of calculating the amount of water removed from the magnesium chloride hydrate, the amount of water removed from the calcium chloride hydrate and the water content of the entire dialysis agent, and then calculating the ratio CM are shown in Table 34.

[Table 32]

Table 32 (Blend amounts in Example 10)

|  | Sample 10-1 | Sample 10-2 | Sample 10-3 | Sample 10-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Anhydrous citric acid | 34.3g | 34.3g | 34.3g | 34.3g |
| Anhydrous sodium citrate | 8.1g | 8.1g | 8.1g | 8.1g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 33]

Table 33 (Amounts of water of adhesion in Example 10)

| | Measured water content | Theoretical water content | Water content due to water of adhesion | Weight of each component | Amount of water of adhesion in each component |
|---|---|---|---|---|---|
| Sodium chloride | 0.05% | 0.00% | 0.05% | 1933.20g | 0.97g |
| Potassium chloride | 0.07% | 0.00% | 0.07% | 47.00g | 0.03g |
| Anhydrous citric acid | 0.03% | 0.00% | 0.03% | 34.30g | 0.01g |
| Anhydrous sodium citrate | 0.92% | 0.00% | 0.92% | 8.10g | 0.07g |
| Anhydrous glucose | 0.10% | 0.00% | 0.10% | 472.50g | 0.47g |
| | | | | Water content due to water of adhesion | Total amount of water of adhesion |
| | | | | 0.06% | 1.56g |

[Table 34]

Table 34 (CM value, consolidation, and 5-HMF in Example 10)

| | Sample 10-1 | Sample 10-2 | Sample 10-3 | Sample 10-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2574.5g | 2584.0g | 2588.2g | 2592.9g |
| Amount of water of adhesion | 1.56g | 1.56g | 1.56g | 1.56g |
| Weight of efflorescent components | - | - | - | - |

(continued)

|  | Sample 10-1 | Sample 10-2 | Sample 10-3 | Sample 10-4 |
|---|---|---|---|---|
| Theoretical water content of efflorescent components | - | - | - | - |
| Amount of water of crystallization | 0.00g | 0.00g | 0.00g | 0.00g |
| Water content of powder dialysis agent | 1.56g | 1.56g | 1.56g | 1.56g |
| CM value | 7% | 12% | 18% | 43% |
| Weight of consolidation | 0.0g | 0.0g | 0.0g | 0.0g |
| Consolidation rate | 0.0% | 0.0% | 0.0% | 0.0% |
| 5-HMF | 0.005 | 0.007 | 0.007 | 0.008 |

<Example 11>

As shown in Table 35, with the exception of using a combination of anhydrous citric acid and sodium citrate dihydrate as the pH modifying component instead of the combination of anhydrous citric acid and anhydrous sodium citrate, evaluations were performed in the same manner as Example 10. The results are shown in Tables 36 and 37.

[Table 35]

Table 35 (Blend amounts in Example 11)

|  | Sample 11-1 | Sample 11-2 | Sample 11-3 | Sample 11-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Anhydrous citric acid | 34.3g | 34.3g | 34.3g | 34.3g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 36]

Table 36 (Amounts of water of adhesion in Example 11)

| | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Anhydrous citric acid | 0.03% | 0.00% | 0.03% | 34.30g | 0.01g |
| Sodium citrate dihydrate | 12.26% | 12.25% | 0.01% | 9.30g | 0.00g |
| Anhydrous glucose | 0.05% | 0.00% | 0.05% | 472.50g | 0.24g |
| | | | | Water content due to water of adhesion | Total amount of water of adhesion |
| | | | | 0.02% | 0.45g |

[Table 37]

Table 37 (CM value, consolidation, and 5-HMF in Example 11)

| | Sample 11-1 | Sample 11-2 | Sample 11-3 | Sample 11-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2575.7g | 2585.2g | 2589.4g | 2594.1g |
| Amount of water of adhesion | 0.45g | 0.45g | 0.45g | 0.45g |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |

(continued)

|  | Sample 11-1 | Sample 11-2 | Sample 11-3 | Sample 11-4 |
|---|---|---|---|---|
| Amount of water of crystallization | 1.14g | 1.14g | 1.14g | 1.14g |
| Water content of powder dialysis agent | 1.59g | 1.59g | 1.59g | 1.59g |
| CM value | 7% | 13% | 19% | 43% |
| Weight of consolidation | 0.0g | 0.0g | 0.0g | 0.0g |
| Consolidation rate | 0.0% | 0.0% | 0.0% | 0.0% |
| 5-HMF | 0.006 | 0.007 | 0.006 | 0.008 |

<Example 12>

As shown in Table 38, with the exception of using a combination of citric acid monohydrate and anhydrous sodium citrate as the pH modifying component instead of the combination of anhydrous citric acid and anhydrous sodium citrate, evaluations were performed in the same manner as Example 10. The results are shown in Tables 39 and 40.

[Table 38]

Table 38 (Blend amounts in Example 12)

|  | Sample 12-1 | Sample 12-2 | Sample 12-3 | Sample 12-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Citric acid monohydrate | 37.5g | 37.5g | 37.5g | 37.5g |
| Anhydrous sodium citrate | 8.1g | 8.1g | 8.1g | 8.1g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 39]

Table 39 (Amounts of water of adhesion in Example 12)

|  | Measured water content | Theoretical water content | Watercontent due to water of adhesion | Weight of each component | Amount of water of adhesion in each component |
|---|---|---|---|---|---|
| Sodium chloride | 0.05% | 0.00% | 0.05% | 1933.20g | 0.97g |

(continued)

|  | Measured water content | Theoretical water content | Water content due to water of adhesion | Weight of each component | Amount of water of adhesion in each component |
|---|---|---|---|---|---|
| Potassium chloride | 0.07% | 0.00% | 0.07% | 47.00g | 0.03g |
| Citric acid monohydrate | 9.26% | 8.58% | 0.68% | 37.50g | 0.26g |
| Anhydrous sodium citrate | 0.92% | 0.00% | 0.92% | 8.10g | 0.07g |
| Anhydrous glucose | 0.10% | 0.00% | 0.10% | 472.50g | 0.47g |
|  |  |  |  | Water content due to water of adhesion | Total amount of water of adhesion |
|  |  |  |  | 0.07% | 1.80g |

[Table 40]

Table 40 (CM value, consolidation, and 5-HMF in Example 12)

|  | Sample 12-1 | Sample 12-2 | Sample 12-3 | Sample 12-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2577.7g | 2587.2g | 2591.4g | 2596.1g |
| Amount of water of adhesion | 1.80g | 1.80g | 1.80g | 1.80g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Amount of water of crystallization | 3.23g | 3.23g | 3.23g | 3.23g |

(continued)

|  | Sample 12-1 | Sample 12-2 | Sample 12-3 | Sample 12-4 |
|---|---|---|---|---|
| Water content of powder dialysis agent | 5.03g | 5.03g | 5.03g | 5.03g |
| CM value | 23% | 40% | 60% | 137% |
| Weight of consolidation | 0.0g | 0.0g | 0.0g | 1.0g |
| Consolidation rate | 0.0% | 0.0% | 0.0% | 0.0% |
| 5-HMF | 0.006 | 0.007 | 0.006 | 0.008 |

<Example 13>

As shown in Table 41, with the exception of using a combination of citric acid monohydrate and sodium citrate dihydrate as the pH modifying component instead of the combination of anhydrous citric acid and anhydrous sodium citrate, evaluations were performed in the same manner as Example 10. The results are shown in Tables 42 and 43.

[Table 41]

Table 41 (Blend amounts in Example 13)

|  | Sample 13-1 | Sample 13-2 | Sample 13-3 | Sample 13-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 42]

Table 42 (Amounts of water of adhesion in Example 13)

|  | Measured water content | Theoretical water content | Watercontent due to water of adhesion | Weight of each component | Amount of water of adhesion in each component |
|---|---|---|---|---|---|
| Sodium chloride | 0.05% | 0.00% | 0.05% | 1933.20g | 0.97g |
| Potassium chloride | 0.07% | 0.00% | 0.07% | 47.00g | 0.03g |

(continued)

|  | Measured water content | Theoretical water content | Water content due to water of adhesion | Weight of each component | Amount of water of adhesion in each component |
|---|---|---|---|---|---|
| Citric acid monohydrate | 9.26% | 8.58% | 0.68% | 37.50g | 0.26g |
| Sodium citrate dihydrate | 12.28% | 12.25% | 0.03% | 9.30g | 0.00g |
| Anhydrous glucose | 0.10% | 0.00% | 0.10% | 472.50g | 0.47g |
|  |  |  |  | Water content due to water of adhesion | Total amount of water of adhesion |
|  |  |  |  | 0.07% | 1.73g |

[Table 43]

Table 43 (CM value, consolidation, and 5-HMF in Example 13)

|  | Sample 13-1 | Sample 13-2 | Sample 13-3 | Sample 13-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2578.9g | 2588.4g | 2592.6g | 2597.3g |
| Amount of water of adhesion | 1.73g | 1.73g | 1.73g | 1.73g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |

(continued)

|  | Sample 13-1 | Sample 13-2 | Sample 13-3 | Sample 13-4 |
|---|---|---|---|---|
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 6.10g | 6.10g | 6.10g | 6.10g |
| CM value | 28% | 49% | 72% | 167% |
| Weight of consolidation | 0.0g | 0.0g | 0.8g | 1. 3g |
| Consolidation rate | 0.0% | 0.0% | 0.0% | 0.1% |
| 5-HMF | 0.008 | 0.008 | 0.007 | 0.077 |

<Example 14>

With the exception of altering the blend amounts of magnesium chloride hydrate and calcium chloride hydrate as shown in Table 44, evaluations were performed in the same manner as Example 13. The results are shown in Tables 45 and 46.

[Table 44]

Table 44 (Blend amounts in Example 14)

|  | Sample 14-1 | Sample 14-2 | Sample 14-3 | Sample 14-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 6.6g | - | - | - |
| Magnesium chloride hydrate (45%) | - | 6.8g | - | - |
| Magnesium chloride hydrate (47%) | - | - | 7.1g | - |
| Magnesium chloride hydrate (51%) | - | - | - | 7.7g |
| Calcium chloride hydrate (1%) | 22.1g | - | - | - |
| Calcium chloride hydrate (15%) | - | 25.7g | - | - |
| Calcium chloride hydrate (19%) | - | - | 27.0g | - |
| Calcium chloride hydrate (22%) | - | - | - | 28.0g |

[Table 45]

Table 45 (Amounts of water of adhesion in Example 14)

| | Measured water content | Theoretical water content | Water content due to water of adhesion | Weight of each component | Amount of water of adhesion in each component |
|---|---|---|---|---|---|
| Sodium chloride | 0.05% | 0.00% | 0.05% | 1933.20g | 0.97g |
| Potassium chloride | 0.07% | 0.00% | 0.07% | 47.00g | 0.03g |
| Citric acid monohydrate | 9.26% | 8.58% | 0.68% | 37.50g | 0.26g |
| Sodium citrate dihydrate | 12.28% | 12.25% | 0.03% | 9.30g | 0.00g |
| Anhydrous glucose | 0.10% | 0.00% | 0.10% | 472.50g | 0.47g |
| | | | | Water content due to water of adhesion | Total amount of water of adhesion |
| | | | | 0.07% | 1.73g |

[Table 46]

Table 46 (CM value, consolidation, and 5-HMF in Example 14)

| | Sample 14-1 | Sample 14-2 | Sample 14-3 | Sample 14-4 |
|---|---|---|---|---|
| Weight of magnesium chloride hexahydrate | 8.0g | 8.0g | 8.0g | 8.0g |
| Theoretical water content of magnesium chloride hexahydrate | 53.2% | 53.2% | 53.2% | 53.2% |
| Weight of dried magnesium chloride hydrate | 6.6g | 6.8g | 7.1g | 7.7g |
| Measured water content of dried magnesium chloride hydrate | 43% | 45% | 47% | 51% |
| Amount of water removed from magnesium chloride hydrate | 1.42g | 1.20g | 0.92g | 0.33g |
| Weight of calcium chloride dihydrate | 29.0g | 29.0g | 29.0g | 29.0g |
| Theoretical water content of calcium chloride dihydrate | 24.5% | 24.5% | 24.5% | 24.5% |
| Weight of dried calcium chloride hydrate | 22.1g | 25.7g | 27.0g | 28.0g |
| Measured water content of dried calcium chloride hydrate | 1% | 15% | 19% | 22% |
| Amount of water removed from calcium chloride hydrate | 6.88g | 3.25g | 1.98g | 0.94g |
| Total weight of all components | 2528.2g | 2532.0g | 2533.6g | 2535.2g |
| Amount of water of adhesion | 1.73g | 1.73g | 1.73g | 1.73g |
| Weight of efflorescent component (citric acid monohydrate) | 37.5g | 37.5g | 37.5g | 37.5g |

(continued)

|  | Sample 14-1 | Sample 14-2 | Sample 14-3 | Sample 14-4 |
|---|---|---|---|---|
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 6.10g | 6.10g | 6.10g | 6.10g |
| CM value | 73% | 137% | 210% | 480% |
| Weight of consolidation | 0.0g | 0.9g | 8.4g | 128.7g |
| Consolidation rate | 0.0% | 0.0% | 0.3% | 5.1% |
| 5-HMF | 0.008 | 0.008 | 0.080 | 0.095 |

<Example 15>

With the exception of altering the blend amounts of magnesium chloride hydrate and calcium chloride hydrate as shown in Table 47, evaluations were performed in the same manner as Example 13. The results are shown in Tables 48 and 49.

[Table 47]

Table 47 (Blend amounts in Example 15)

|  | Sample 15-1 | Sample 15-2 | Sample 15-3 | Sample 15-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Magnesium chloride hydrate (43%) | 39.6g | - | - | - |
| Magnesium chloride hydrate (45%) | - | 40.8g | - | - |
| Magnesium chloride hydrate (47%) | - | - | 42.5g | - |
| Magnesium chloride hydrate (51%) | - | - | - | 45.9g |
| Calcium chloride hydrate (1%) | 61.8g | - | - | - |
| Calcium chloride hydrate (15%) | - | 72.0g | - | - |
| Calcium chloride hydrate (19%) | - | - | 75.6g | - |
| Calcium chloride hydrate (22%) | - | - | - | 78.4g |

[Table 48]

Table 48 (Amounts of water of adhesion in Example 15)

| | Measured water content | Theoretical water content | Water content due to water of adhesion | Weight of each component | Amount of water of adhesion in each component |
|---|---|---|---|---|---|
| Sodium chloride | 0.05% | 0.00% | 0.05% | 1933.20g | 0.97g |
| Potassium chloride | 0.07% | 0.00% | 0.07% | 47.00g | 0.03g |
| Citric acid monohydrate | 9.26% | 8.58% | 0.68% | 37.50g | 0.26g |
| Sodium citrate dihydrate | 12.28% | 12.25% | 0.03% | 9.30g | 0.00g |
| Anhydrous glucose | 0.10% | 0.00% | 0.10% | 472.50g | 0.47g |
| | | | | Water content due to water of adhesion | Total amount of water of adhesion |
| | | | | 0.07% | 1.73g |

[Table 49]

Table 49 (CM value, consolidation, and 5-HMF in Example 15)

| | Sample 15-1 | Sample 15-2 | Sample 15-3 | Sample 15-4 |
|---|---|---|---|---|
| Weight of magnesium chloride hexahydrate | 48.0g | 48.0g | 48.0g | 48.0g |
| Theoretical water content of magnesium chloride hexahydrate | 53.2% | 53.2% | 53.2% | 53.2% |
| Weight of dried magnesium chloride hydrate | 39.6g | 40.8g | 42.5g | 45.9g |
| Measured water content of dried magnesium chloride hydrate | 43% | 45% | 47% | 51% |
| Amount of water removed from magnesium chloride hydrate | 8.51g | 7.18g | 5.56g | 2.13g |
| Weight of calcium chloride dihydrate | 81.1g | 81.1g | 81.1g | 81.1g |
| Theoretical water content of calcium chloride dihydrate | 24.5% | 24.5% | 24.5% | 24.5% |
| Weight of dried calcium chloride hydrate | 61.8g | 72.0g | 75.6g | 78.4g |
| Measured water content of dried calcium chloride hydrate | 1% | 15% | 19% | 22% |
| Amount of water removed from calcium chloride hydrate | 19.25g | 9.07g | 5.51g | 2.62g |
| Total weight of all components | 2600.9g | 2612.3g | 2617.6g | 2623.8g |
| Amount of water of adhesion | 1.73g | 1.73g | 1.73g | 1.73g |
| Weight of efflorescent component (citric acid monohydrate) | 37.5g | 37.5g | 37.5g | 37.5g |

(continued)

|  | Sample 15-1 | Sample 15-2 | Sample 15-3 | Sample 15-4 |
|---|---|---|---|---|
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 6.10g | 6.10g | 6.10g | 6.10g |
| CM value | 22% | 38% | 55% | 128% |
| Weight of consolidation | 0.0g | 0.3g | 0.0g | 0.5g |
| Consolidation rate | 0.0% | 0.0% | 0.0% | 0.0% |
| 5-HMF | 0.007 | 0.008 | 0.006 | 0.008 |

<Example 16>

As shown in Table 50, with the exception of using a combination of powder acetic acid and anhydrous sodium acetate as the pH modifying component instead of the combination of anhydrous citric acid and anhydrous sodium citrate, evaluations were performed in the same manner as Example 10. The results are shown in Tables 51 and 52.

[Table 50]

Table 50 (Blend amounts in Example 16)

|  | Sample 16-1 | Sample 16-2 | Sample 16-3 | Sample 16-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Powder acetic acid | 99.4g | 99.4g | 99.4g | 99.4g |
| Anhydrous sodium acetate | 149.4g | 149.4g | 149.4g | 149.4g |
| Anhydrous glucose | 315.Og | 315.Og | 315.Og | 315.Og |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 51]

Table 51 (Amounts of water of adhesion in Example 16)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.80g | 0.20g |
| Potassium chloride | 0.02% | 0.00% | 0.02% | 47.00g | 0.01g |
| Powder acetic acid | 0.21% | 0.00% | 0.21% | 99.40g | 0.21g |
| Anhydrous sodium acetate | 0.21% | 0.00% | 0.21% | 149.40g | 0.31g |
| Anhydrous glucose | 0.05% | 0.00% | 0.05% | 315.00g | 0.16g |
|  |  |  |  | Water content due to water of adhesion | Total amount of water of adhesion |
|  |  |  |  | 0.03% | 0.89g |

[Table 52]

Table 52 (CM value, consolidation, and 5-HMF in Example 16)

|  | Sample 16-1 | Sample 16-2 | Sample 16-3 | Sample 16-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2660.0g | 2669.5g | 2673.7g | 2678.4g |
| Amount of water of adhesion | 0.89g | 0.89g | 0.89g | 0.89g |
| Weight of efflorescent components | - | - | - | - |
| Theoretical water content of efflorescent components | - | - | - | - |
| Amount of water of crystallization | 0.00g | 0.00g | 0.00g | 0.00g |

(continued)

|  | Sample 16-1 | Sample 16-2 | Sample 16-3 | Sample 16-4 |
|---|---|---|---|---|
| Water content of powder dialysis agent | 0.89g | 0.89g | 0.89g | 0.89g |
| CM value | 4% | 7% | 11% | 24% |
| Weight of consolidation | 0.0g | 0.0g | 0.0g | 0.0g |
| Consolidation rate | 0.0% | 0.0% | 0.0% | 0.0% |
| 5-HMF | 0.003 | 0.004 | 0.004 | 0.004 |

<Example 17>

As shown in Table 53, with the exception of using a combination of powder acetic acid and sodium acetate trihydrate as the pH modifying component instead of the combination of anhydrous citric acid and anhydrous sodium citrate, evaluations were performed in the same manner as Example 10. The results are shown in Tables 54 and 55.

[Table 53]

Table 53 (Blend amounts in Example 17)

|  | Sample 17-1 | Sample 17-2 | Sample 17-3 | Sample 17-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Powder acetic acid | 99.4g | 99.4g | 99.4g | 99.4g |
| Sodium acetate trihydrate | 247.8g | 247.8g | 247.8g | 247.8g |
| Anhydrous glucose | 315.0g | 315.0g | 315.0g | 315.0g |
| Magnesium chloride hydrate (43%) | 26.4g | - | - | - |
| Magnesium chloride hydrate (45%) | - | 27.2g | - | - |
| Magnesium chloride hydrate (47%) | - | - | 28.3g | - |
| Magnesium chloride hydrate (51%) | - | - | - | 30.6g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 54]

Table 54 (Amounts of water of adhesion in Example 17)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.80g | 0.20g |
| Potassium chloride | 0.02% | 0.00% | 0.02% | 47.00g | 0.01g |

(continued)

| | Measured water content | Theoretical water content | Water content due to water of adhesion | Weight of each component | Amount of water of adhesion in each component |
|---|---|---|---|---|---|
| Powder acetic acid | 0.21% | 0.00% | 0.21% | 99.40g | 0.21g |
| Sodium acetate trihydrate | 39.89% | 39.72% | 0.17% | 247.80g | 0.42g |
| Anhydrous glucose | 0.05% | 0.00% | 0.05% | 315.00g | 0.16g |
| | | | | Water content due to water of adhesion | Total amount of water of adhesion |
| | | | | 0.04% | 0.99g |

[Table 55]

Table 55 (CM value, consolidation, and 5-HMF in Example 17)

| | Sample 17-1 | Sample 17-2 | Sample 17-3 | Sample 17-4 |
|---|---|---|---|---|
| Weight of magnesium chloride hexahydrate | 32.0g | 32.0g | 32.0g | 32.0g |
| Theoretical water content of magnesium chloride hexahydrate | 53.2% | 53.2% | 53.2% | 53.2% |
| Weight of dried magnesium chloride hydrate | 26.4g | 27.2g | 28.3g | 30.6g |
| Measured water content of dried magnesium chloride hydrate | 43% | 45% | 47% | 51% |
| Amount of water removed from magnesium chloride hydrate | 5.67g | 4.78g | 3.72g | 1.42g |
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2758.4g | 2767.9g | 2772.1g | 2776.8g |
| Amount of water of adhesion | 0.99g | 0.99g | 0.99g | 0.99g |
| Weight of efflorescent component (sodium acetate trihydrate) | 247.8g | 247.8g | 247.8g | 247.8g |
| Theoretical water content of efflorescent component (sodium acetate trihydrate) | 39.7% | 39.7% | 39.7% | 39.7% |
| Amount of water of crystallization | 98.38g | 98.38g | 98.38g | 98.38g |
| Water content of powder dialysis agent | 99.37g | 99.37g | 99.37g | 99.37g |

(continued)

|  | Sample 17-1 | Sample 17-2 | Sample 17-3 | Sample 17-4 |
|---|---|---|---|---|
| CM value | 448% | 792% | 1177% | 2715% |
| Weight of consolidation | 767.4g | 792.6g | 784.5g | 812.5g |
| Consolidation rate | 27.8% | 28.6% | 28.3% | 29.3% |
| 5-HMF | 0.004 | 0.004 | 0.004 | 0.004 |

The concentration of each component when each sample was dissolved in RO water and made up to 315 L is shown in Table 56.

[Table 56]

Table 56 (Concentration when each sample was dissolved in RO water and made up to 315 L)

|  | Examples 10 to 13 | Example 14 | Example 15 | Examples 16 and 17 |
|---|---|---|---|---|
| Na+ | 105.3mEq/L | 105.3mEq/L | 105.3mEq/L | 115.0mEq/L |
| K+ | 2.0mEq/L | 2.0mEq/L | 2.0mEq/L | 2.0mEq/L |
| Ca++ | 3.0mEq/L | 1.25mEq/L | 3.5mEq/L | 3.0mEq/L |
| Mg++ | 1.0mEq/L | 0.25mEq/L | 1.5mEq/L | 1.0mEq/L |
| Cl- | 111.mEq/L | 108.5mEq/L | 112.0mEq/L | 113.0mEq/L |
| Citrate ion | 2.0mEq/L | 2.0mEq/L | 2.0mEq/L | 0.0mEq/L |
| Acetate ion | 0.0mEq/L | 0.0mEq/L | 0.0mEq/L | 10.2mEq/L |
| Anhydrous glucose | 150.0mg/dl | 150.0mg/dl | 150.0mg/dl | 100.0mg/dl |

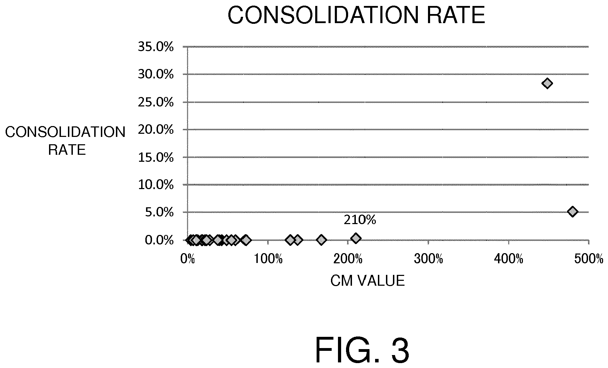

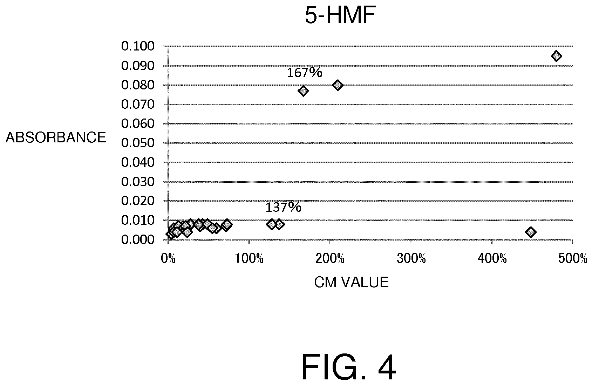

Combining the above results, the relationship between the ratio CM and the consolidation rate is shown in FIG. 3, and the relationship between the ratio CM and the 5-HMF absorbance is shown in FIG. 4.

In this manner, by ensuring that the ratio between the water content of the entire dialysis agent and the sum of the amount of water removed from the magnesium chloride hydrate and the amount of water removed from the calcium chloride hydrate was not more than 210% but at least 2%, consolidation and aggregation of the chemical agent were suppressed in the stability test. Further, by ensuring that the ratio between the water content of the entire dialysis agent and the sum of the amount of water removed from the magnesium chloride hydrate and the amount of water removed from the calcium chloride hydrate was not more than 167% but at least 2%, production of 5-HMF was inhibited in the stability test.

<Example 18>

Using magnesium chloride hydrate ($MgCl_2$) (water content: 45%) and calcium chloride hydrate ($CaCl_2$) (water content: 15%) that had been subjected to drying steps to provide improved functionality as desiccants, sodium chloride, potassium chloride, anhydrous glucose, the magnesium chloride hydrate, the calcium chloride hydrate and a pH modifier (any one of the acetic acid mixture (1.5:1.5), the acetic acid mixture (3:3), the acetic acid mixture (6:2), the acetic acid mixture (8:2.2), the acetic acid mixture (10:2), or a combination of anhydrous sodium acetate and glacial acetic acid) were placed in a container in a ratio shown in Table 57, the container was sealed, and the container contents were dispersed to complete preparation of a series of dialysis agents (samples 18-1 to 18-6).

The amount of water of adhesion in each of the sodium chloride, the potassium chloride, the anhydrous glucose and the pH modifier was measured using the water content measurement method described above to determine the amount of water of adhesion. The results are shown in Table 58.

A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 59. Further, the results of calculating the amount of water removed from the magnesium chloride hydrate, the amount of water removed from the calcium chloride hydrate and the water content of the entire dialysis agent, and then calculating the ratio CM are shown in Table 59.

[Table 57]

Table 57 (Blend amounts in Example 18)

|  | Sample 18-1 | Sample 18-2 | Sample 18-3 | Sample 18-4 | Sample 18-5 | Sample 18-6 |
|---|---|---|---|---|---|---|
| Sodium chloride | 1969.8g | 1969.8g | 1969.8g | 1969.8g | 1969.8g | 1969.8g |
| Potassium chloride | 47.0g | 47.0g | 47.0g | 47.0g | 47.0g | 47.0g |
| Acetic acid mixture (1.5:1.5) | 67.1g | - | - | - | - | - |
| Acetic acid mixture (3:3) | - | 134.2g | - | - | - | - |
| Acetic acid mixture (6:2) |  | - | 192.6g | - | - | - |
| Acetic acid mixture (8:2.2) | - | - | - | 248.7g |  | - |
| Acetic acid mixture (10:2) |  | - | - | - | 296.2g | - |
| Glacial acetic acid | - | - | - | - | - | 42.0g |
| Anhydrous sodium acetate | - | - | - | - | - | 206.7g |
| Anhydrous glucose | 315.0g | 315.0g | 315.0g | 315.0g | 315.0g | 315.0g |
| Magnesium chloride hydrate (45%) | 27.2g | 27.2g | 27.2g | 27.2g | 27.2g | 27.2g |
| Calcium chloride hydrate (15%) | 61.7g | 61.7g | 61.7g | 61.7g | 61.7g | 61.7g |

[Table 58]

Table 58 (Amounts of water of adhesion in Example 18)

|  | Measured water content | Theoretical water content | Water content due to water of adhesion | Weight of each component | Amount of water of adhesion in each component |
|---|---|---|---|---|---|
| Sodium chloride | 0.03% | 0.00% | 0.03% | 1969.8g | 0.59g |
| Potassium chloride | 0.02% | 0.00% | 0.02% | 47.0g | 0.01g |
| Acetic acid mixture (1.5:1.5) | 0.21% | 0.00% | 0.21% | 67.1g | 0.14g |
| Acetic acid mixture (3:3) | 0.21% | 0.00% | 0.21% | 134.2g | 0.28g |
| Acetic acid mixture (6:2) | 0.19% | 0.00% | 0.19% | 192.6g | 0.37g |

(continued)

|  | Measured water content | Theoretical water content | Water content due to water of adhesion | Weight of each component | Amount of water of adhesion in each component |
|---|---|---|---|---|---|
| Acetic acid mixture (8:2.2) | 0.18% | 0.00% | 0.18% | 248.7g | 0.45g |
| Acetic acid mixture (10:2) | 0.16% | 0.00% | 0.16% | 296.2g | 0.47g |
| Glacial acetic acid | 0.07% | 0.00% | 0.07% | 42.0g | 0.03g |
| Anhydrous sodium acetate | 0.21% | 0.00% | 0.21% | 206.7g | 0.43g |
| Anhydrous glucose | 0.05% | 0.00% | 0.05% | 315.Og | 0.16g |

[Table 59]

Table 59 (CM value, consolidation, and 5-HMF in Example 18)

|  | Sample 18-1 | Sample 18-2 | Sample 18-3 | Sample 18-4 | Sample 18-5 | Sample 18-6 |
|---|---|---|---|---|---|---|
| Weight of magnesium chloride hexahydrate | 32.0g | 32.0g | 32.0g | 32.0g | 32.0g | 32.0g |
| Theoretical water content of magnesium chloride hexahydrate | 53.2% | 53.2% | 53.2% | 53.2% | 53.2% | 53.2% |
| Weight of dried magnesium chloride hydrate | 27.2g | 27.2g | 27.2g | 27.2g | 27.2g | 27.2g |
| Measured water content of dried magnesium chloride hydrate | 45% | 45% | 45% | 45% | 45% | 45% |
| Amount of water removed from magnesium chloride hydrate | 4.78g | 4.78g | 4.78g | 4.78g | 4.78g | 4.78g |
| Weight of calcium chloride dihydrate | 69.5g | 69.5g | 69.5g | 69.5g | 69.5g | 69.5g |
| Theoretical water content of calcium chloride dihydrate | 24.5% | 24.5% | 24.5% | 24.5% | 24.5% | 24.5% |
| Weight of dried calcium chloride hydrate | 61.7g | 61.7g | 61.7g | 61.7g | 61.7g | 61.7g |
| Measured water content of dried calcium chloride hydrate | 15% | 15% | 15% | 15% | 15% | 15% |
| Amount of water removed from calcium chloride hydrate | 7.77g | 7.77g | 7.77g | 7.77g | 7.77g | 7.77g |
| Total weight of all components | 2487.8g | 2554.9g | 2613.3g | 2669.4g | 2716.9g | 2669.4g |
| Amount of water of adhesion | 0.90g | 1.04g | 1.12g | 1.21g | 1.23g | 1.22g |

(continued)

| | Sample 18-1 | Sample 18-2 | Sample 18-3 | Sample 18-4 | Sample 18-5 | Sample 18-6 |
|---|---|---|---|---|---|---|
| Weight of efflorescent components | - | - | - | - | - | - |
| Theoretical water content of efflorescent components | - | - | - | - | - | - |
| Amount of water of crystallization | 0.00g | 0.00g | 0.00g | 0.00g | 0.00g | 0.00g |
| Water content of powder dialysis agent | 0.90g | 1.04g | 1.12g | 1.21g | 1.23g | 1.22g |
| CM value | 7% | 8% | 9% | 10% | 10% | 10% |
| Weight of consolidation | 1.0g | 0.9g | 1.5g | 1.1g | 1.2g | 449.7g |
| Consolidation rate | 0.0% | 0.0% | 0.1% | 0.0% | 0.0% | 16.8% |
| 5-HMF | 0.005 | 0.005 | 0.005 | 0.005 | 0.005 | 0.081 |

In this manner, by using an acetic acid mixture composed of a mixture of anhydrous sodium acetate and glacial acetic acid as the pH modifier, rather than a simple combination of anhydrous sodium acetate and glacial acetic, consolidation and aggregation of the chemical agent were suppressed in the stability test. Further, production of 5-HMF was inhibited in the stability test. In Sample 18-6, in which a dialysis agent was produced using anhydrous sodium acetate and glacial acetic acid as pH modifiers, significantly more consolidation of the chemical agent and degradation of the anhydrous glucose occurred compared with the case where an acetic acid mixture was used.

The concentration of each component when Samples 18-1 to 18-6 were each dissolved in RO water and made up to 315 L is shown in Table 60.

[Table 60]

Table 60 (Concentration when each sample was dissolved in RO water and made up to 315 L)

| | Sample 18-1 | Sample 18-2 | Sample 18-3 | Sample 18-4 | Sample 18-5 | Sample 18-6 |
|---|---|---|---|---|---|---|
| Na+ | 108.5mEq/L | 110.0mEq/L | 113.0mEq/L | 115.0mEq/L | 117. OmEq/L | 115.0mEq/L |
| K+ | 2.0mEq/L | 2.0mEq/L | 2.0mEq/L | 2.0mEq/L | 2.0mEq/L | 2.0mEq/L |
| Ca++ | 3.0mEq/L | 3.0mEq/L | 3.0mEq/L | 3.0mEq/L | 3.0mEq/L | 3.0mEq/L |
| Mg++ | 1.0mEq/L | 1.0mEq/L | 1.0mEq/L | 1.0mEq/L | 1.0mEq/L | 1.0mEq/L |
| Cl- | 113.0mEq/L | 113.0mEq/L | 113.0mEq/L | 113.0mEq/L | 113.0mEq/L | 113.0mEq/L |
| Acetate ion | 3.0mEq/L | 6.0mEq/L | 8.0mEq/L | 10.2mEq/L | 12.0mEq/L | 10.2mEq/L |
| Anhydrous glucose | 100.0mg/dl | 100.0mg/dl | 100.0mg/dl | 100.0mg/dl | 100.0mg/dl | 100.0mg/dl |

<Reference Example 1>

Using calcium chloride hydrate ($CaCl_2$) (water content: 1%, 15%, 19%, 22%) that had been subjected to a drying step to provide improved functionality as a desiccant, sodium chloride, potassium chloride, magnesium chloride hexahydrate, anhydrous citric acid, anhydrous sodium citrate, anhydrous glucose, and the calcium chloride hydrate were placed in a container in a ratio shown in Table 61, and the container was sealed to complete preparation of a series of dialysis agents (samples 19-1 to 19-4).

The amount of water of adhesion in each of the sodium chloride, the potassium chloride, the magnesium chloride hexahydrate, the anhydrous citric acid, the anhydrous sodium citrate and the anhydrous glucose was measured using the water content measurement method described above to determine the amount of water of adhesion. The results are shown in Table 62.

A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 63. Further, the results of calculating the amount of water removed from the calcium chloride hydrate and the water content of the entire dialysis agent, and then calculating the ratio C are shown in Table 63.

[Table 61]

Table 61 (Blend amounts in Reference Example 1)

|  | Sample 19-1 | Sample 19-2 | Sample 19-3 | Sample 19-4 |
| --- | --- | --- | --- | --- |
| Sodium chloride | 1933.2g | 1933.2g | 1933.2g | 1933.2g |
| Potassium chloride | 47.0g | 47.0g | 47.0g | 47.0g |
| Magnesium chloride hexahydrate | 32.0g | 32.0g | 32.0g | 32.0g |
| Anhydrous citric acid | 34.3g | 34.3g | 34.3g | 34.3g |
| Anhydrous sodium citrate | 8.1g | 8.1g | 8.1g | 8.1g |
| Anhydrous glucose | 472.5g | 472.5g | 472.5g | 472.5g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 62]

Table 62 (Amounts of water of adhesion in Reference Example 1)

|  | Measured water content | Theoretical water content | Water content due to water of adhesion | Weight of each component | Amount of water of adhesion in each component |
| --- | --- | --- | --- | --- | --- |
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Anhydrous citric acid | 0.03% | 0.00% | 0.03% | 34.30g | 0.01g |
| Anhydrous sodium citrate | 0.56% | 0.00% | 0.56% | 8.10g | 0.05g |
| Anhydrous glucose | 0.05% | 0.00% | 0.05% | 472.50g | 0.24g |

(continued)

|  | Measured water content | Theoretical water content | Water content due to water of adhesion | Weight of each component | Amount of water of adhesion in each component |
|---|---|---|---|---|---|
|  |  |  |  | Water content due to water of adhesion | Total amount of water of adhesion |
|  |  |  |  | 0.03% | 0.74g |

[Table 63]

Table 63 (C value, consolidation, and 5-HMF in Reference Example 1)

|  | Sample 19-1 | Sample 19-2 | Sample 19-3 | Sample 19-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2580.1g | 2588.8g | 2591.9g | 2594.3g |
| Amount of water of adhesion | 0.74g | 0.74g | 0.74g | 0.74g |
| Weight of efflorescent components | - | - | - | - |
| Theoretical water content of efflorescent components | - | - | - | - |
| Amount of water of crystallization | 0.00g | 0.00g | 0.00g | 0.00g |
| Water content of powder dialysis agent | 0.74g | 0.74g | 0.74g | 0.74g |
| C value | 4% | 10% | 16% | 33% |
| Weight of consolidation | 0.0g | 0.8g | 1.2g | 1.8g |
| Consolidation rate | 0.0% | 0.0% | 0.0% | 0.1% |
| 5-HMF | 0.006 | 0.008 | 0.008 | 0.010 |

<Reference Example 2>

As shown in Table 64, with the exception of using a combination of anhydrous citric acid and sodium citrate dihydrate as the pH modifying component instead of the combination of anhydrous citric acid and anhydrous sodium citrate, evaluations were performed in the same manner as Reference Example 1. The results are shown in Tables 65 and 66.

[Table 64]

Table 64 (Blend amounts in Reference Example 2)

|  | Sample 20-1 | Sample 20-2 | Sample 20-3 | Sample 20-4 |
|---|---|---|---|---|
| Sodium chloride | 1933.2g | 1933.2g | 1933.2g | 1933.2g |
| Potassium chloride | 47.0g | 47.0g | 47.0g | 47.0g |

(continued)

|  | Sample 20-1 | Sample 20-2 | Sample 20-3 | Sample 20-4 |
|---|---|---|---|---|
| Magnesium chloride hexahydrate | 32.0g | 32.0g | 32.0g | 32.0g |
| Anhydrous citric acid | 34.3g | 34.3g | 34.3g | 34.3g |
| Sodium citrate dihydrate | 9.3g | 9.3g | 9.3g | 9.3g |
| Anhydrous glucose | 472.5g | 472.5g | 472.5g | 472.5g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 65]

Table 65 (Amounts of water of adhesion in Reference Example 2)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Anhydrous citric acid | 0.03% | 0.00% | 0.03% | 34.30g | 0.01g |
| Sodium citrate dihydrate | 12.26% | 12.25% | 0.01% | 9.30g | 0.00g |
| Anhydrous glucose | 0.05% | 0.00% | 0.05% | 472.50g | 0.24g |
|  |  |  |  | Water content due to water of adhesion | Total amount of water of adhesion |
|  |  |  |  | 0.03% | 0.69g |

[Table 66]

Table 66 (C value, consolidation, and 5-HMF in Reference Example 2)

|  | Sample 20-1 | Sample 20-2 | Sample 20-3 | Sample 20-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |

(continued)

|  | Sample 20-1 | Sample 20-2 | Sample 20-3 | Sample 20-4 |
|---|---|---|---|---|
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2581.3g | 2590.0g | 2593.1g | 2595.5g |
| Amount of water of adhesion | 0.69g | 0.69g | 0.69g | 0.69g |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 1.14g | 1.14g | 1.14g | 1.14g |
| Water content of powder dialysis agent | 1.83g | 1.83g | 1.83g | 1.84g |
| C value | 11% | 24% | 39% | 82% |
| Weight of consolidation | 0.0g | 0.0g | 0.0g | 0.0g |
| Consolidation rate | 0.0% | 0.0% | 0.0% | 0.0% |
| 5-HMF | 0.007 | 0.008 | 0.009 | 0.070 |

<Reference Example 3>

As shown in Table 67, with the exception of using a combination of citric acid monohydrate and anhydrous sodium citrate as the pH modifying component instead of the combination of anhydrous citric acid and anhydrous sodium citrate, evaluations were performed in the same manner as Reference Example 1. The results are shown in Tables 68 and 69.

[Table 67]

Table 67 (Blend amounts in Reference Example 3)

|  | Sample 21-1 | Sample 21-2 | Sample 21-3 | Sample 21-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Magnesium chloride hexahydrate | 32.0g | 32.0g | 32.0g | 32.0g |
| Citric acid monohydrate | 37.5g | 37.5g | 37.5g | 37.5g |
| Anhydrous sodium citrate | 8.1g | 8.1g | 8.1g | 8.1g |
| Anhydrous glucose | 472.5g | 472.5g | 472.5g | 472.5g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 68]

Table 68 (Amounts of water of adhesion in Reference Example 3)

| | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Citric acid monohydrate | 9.26% | 8.58% | 0.68% | 37.50g | 0.26g |
| Anhydrous sodium citrate | 0.56% | 0.00% | 0.56% | 8.10g | 0.05g |
| Anhydrous glucose | 0.05% | 0.00% | 0.05% | 472.50g | 0.24g |
| | | | | Water content due to water of adhesion | Total amount of water of adhesion |
| | | | | 0.04% | 0.98g |

[Table 69]

Table 69 (C value, consolidation, and 5-HMF in Reference Example 3)

| | Sample 21-1 | Sample 21-2 | Sample 21-3 | Sample 21-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2583.3g | 2592.0g | 2595.1g | 2597.5g |
| Amount of water of adhesion | 0.98g | 0.98g | 0.98g | 0.98g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Amount of water of crystallization | 3.23g | 3.23g | 3.23g | 3.23g |
| Water content of powder dialysis agent | 4.21g | 4.21g | 4.21g | 4.21g |
| C value | 26% | 54% | 89% | 188% |
| Weight of consolidation | 0.0g | 0.0g | 1.5g | 245.6g |

(continued)

|  | Sample 21-1 | Sample 21-2 | Sample 21-3 | Sample 21-4 |
|---|---|---|---|---|
| Consolidation rate | 0.0% | 0.0% | 0.1% | 9.5% |
| 5-HMF | 0.007 | 0.007 | 0.072 | 0.082 |

<Reference Example 4>

As shown in Table 70, with the exception of using a combination of citric acid monohydrate and sodium citrate dihydrate as the pH modifying component instead of the combination of anhydrous citric acid and anhydrous sodium citrate, evaluations were performed in the same manner as Reference Example 1. The results are shown in Tables 71 and 72.

[Table 70]

Table 70 (Blend amounts in Reference Example 4)

|  | Sample 22-1 | Sample 22-2 | Sample 22-3 | Sample 22-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Magnesium chloride hexahydrate | 32.0g | 32.0g | 32.0g | 32.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 71]

Table 71 (Amounts of water of adhesion in Reference Example 4)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Citric acid monohydrate | 9.26% | 8.58% | 0.68% | 37.50g | 0.26g |
| Sodium citrate dihydrate | 12.26% | 12.25% | 0.01% | 9.30g | 0.00g |

(continued)

|  | Measured water content | Theoretical water content | Water content due to water of adhesion | Weight of each component | Amount of water of adhesion in each component |
|---|---|---|---|---|---|
| Anhydrous glucose | 0.05% | 0.00% | 0.05% | 472.50g | 0.24g |
|  |  |  |  | Water content due to water of adhesion | Total amount of water of adhesion |
|  |  |  |  | 0.04% | 0.94g |

[Table 72]

Table 72 (C value, consolidation, and 5-HMF in Reference Example 4)

|  | Sample 22-1 | Sample 22-2 | Sample 22-3 | Sample 22-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2584.5g | 2593.2g | 2596.3g | 2598.7g |
| Amount of water of adhesion | 0.94g | 0.94g | 0.94g | 0.94g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 5.31g | 5.31g | 5.31g | 5.31g |
| C value | 32% | 68% | 113% | 237% |
| Weight of consolidation | 0.0g | 0.0g | 3.8g | 232.8g |
| Consolidation rate | 0.0% | 0.0% | 0.1% | 9.0% |
| 5-HMF | 0.006 | 0.006 | 0.044 | 0.078 |

<Reference Example 5>

With the exception of altering the blend amount of calcium chloride hydrate as shown in Table 73, evaluations were performed in the same manner as Reference Example 4. The results are shown in Tables 74 and 75.

[Table 73]

Table 73 (Blend amounts in Reference Example 5)

|  | Sample 23-1 | Sample 23-2 | Sample 23-3 | Sample 23-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Magnesium chloride hexahydrate | 32.0g | 32.0g | 32.0g | 32.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Calcium chloride hydrate (1%) | 22.1g | - | - | - |
| Calcium chloride hydrate (15%) | - | 25.7g | - | - |
| Calcium chloride hydrate (19%) | - | - | 27.0g | - |
| Calcium chloride hydrate (22%) | - | - | - | 28.0g |

[Table 74]

Table 74 (Amounts of water of adhesion in Reference Example 5)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Citric acid monohydrate | 9.26% | 8.58% | 0.68% | 37.50g | 0.26g |
| Sodium citrate dihydrate | 12.26% | 12.25% | 0.01% | 9.30g | 0.00g |
| Anhydrous glucose | 0.05% | 0.00% | 0.05% | 472.50g | 0.24g |
|  |  |  |  | Water content due to water of adhesion | Total amount of water of adhesion |
|  |  |  |  | 0.04% | 0.94g |

[Table 75]

Table 75 (C value, consolidation, and 5-HMF in Reference Example 5)

| | Sample 23-1 | Sample 23-2 | Sample 23-3 | Sample 23-4 |
|---|---|---|---|---|
| Weight of calcium chloride dihydrate | 29.0g | 29.0g | 29.0g | 29.0g |
| Theoretical water content of calcium chloride dihydrate | 24.5% | 24.5% | 24.5% | 24.5% |
| Weight of dried calcium chloride hydrate | 22.1g | 25.7g | 27.0g | 28.0g |
| Measured water content of dried calcium chloride hydrate | 1% | 15% | 19% | 22% |
| Amount of water removed from calcium chloride hydrate | 6.88g | 3.25g | 1.98g | 0.94g |
| Total weight of all components | 2553.6g | 2557.2g | 2558.5g | 2559.5g |
| Amount of water of adhesion | 0.94g | 0.94g | 0.94g | 0.94g |
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 5.31g | 5.31g | 5.31g | 5.31g |
| C value | 77% | 163% | 268% | 565% |
| Weight of consolidation | 0.0g | 147.0g | 267.8g | 279.5g |
| Consolidation rate | 0.0% | 5.7% | 10.5% | 10.9% |
| 5-HMF | 0.005 | 0.075 | 0.085 | 0.099 |

<Reference Example 6>

With the exception of altering the blend amount of calcium chloride hydrate as shown in Table 76, evaluations were performed in the same manner as Reference Example 4. The results are shown in Tables 77 and 78.

[Table 76]

Table 76 (Blend amounts in Reference Example 6)

| | Sample 24-1 | Sample 24-2 | Sample 24-3 | Sample 24-4 |
|---|---|---|---|---|
| Sodium chloride | 1933.2g | 1933.2g | 1933.2g | 1933.2g |
| Potassium chloride | 47.0g | 47.0g | 47.0g | 47.0g |
| Magnesium chloride hexahydrate | 32.0g | 32.0g | 32.0g | 32.0g |
| Citric acid monohydrate | 37.5g | 37.5g | 37.5g | 37.5g |
| Sodium citrate dihydrate | 9.3g | 9.3g | 9.3g | 9.3g |
| Anhydrous glucose | 472.5g | 472.5g | 472.5g | 472.5g |
| Calcium chloride hydrate (1%) | 61.8g | - | - | - |

(continued)

| | Sample 24-1 | Sample 24-2 | Sample 24-3 | Sample 24-4 |
|---|---|---|---|---|
| Calcium chloride hydrate (15%) | - | 72.0g | - | - |
| Calcium chloride hydrate (19%) | - | - | 75.6g | - |
| Calcium chloride hydrate (22%) | - | - | - | 78.4g |

[Table 77]

Table 77 (Amounts of water of adhesion in Reference Example 6)

| | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1933.20g | 0.19g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Citric acid monohydrate | 9.26% | 8.58% | 0.68% | 37.50g | 0.26g |
| Sodium citrate dihydrate | 12.26% | 12.25% | 0.01% | 9.30g | 0.00g |
| Anhydrous glucose | 0.05% | 0.00% | 0.05% | 472.50g | 0.24g |
| | | | | Water content due to water of adhesion | Total amount of water of adhesion |
| | | | | 0.04% | 0.94g |

[Table 78]

Table 78 (C value, consolidation, and 5-HMF in Reference Example 6)

| | Sample 24-1 | Sample 24-2 | Sample 24-3 | Sample 24-4 |
|---|---|---|---|---|
| Weight of calcium chloride dihydrate | 81.1g | 81.1g | 81.1g | 81.1g |
| Theoretical water content of calcium chloride dihydrate | 24.5% | 24.5% | 24.5% | 24.5% |
| Weight of dried calcium chloride hydrate | 61.8g | 72.0g | 75.6g | 78.4g |
| Measured water content of dried calcium chloride hydrate | 1% | 15% | 19% | 22% |
| Amount of water removed from calcium chloride hydrate | 19.25g | 9.07g | 5.51g | 2.62g |
| Total weight of all components | 2593.3g | 2603.5g | 2607.1g | 2609.9g |
| Amount of water of adhesion | 0.94g | 0.94g | 0.94g | 0.94g |

(continued)

| | Sample 24-1 | Sample 24-2 | Sample 24-3 | Sample 24-4 |
|---|---|---|---|---|
| Weight of efflorescent component (citric acid monohydrate) | 37.5g | 37.5g | 37.5g | 37.5g |
| Theoretical water content of efflorescent component (citric acid monohydrate) | 8.6% | 8.6% | 8.6% | 8.6% |
| Weight of efflorescent component (sodium citrate dihydrate) | 9.3g | 9.3g | 9.3g | 9.3g |
| Theoretical water content of efflorescent component (sodium citrate dihydrate) | 12.3% | 12.3% | 12.3% | 12.3% |
| Amount of water of crystallization | 4.37g | 4.37g | 4.37g | 4.37g |
| Water content of powder dialysis agent | 5.31g | 5.31g | 5.31g | 5.31g |
| C value | 28% | 59% | 96% | 203% |
| Weight of consolidation | 0.0g | 0.0g | 0.0g | 259.7g |
| Consolidation rate | 0.0% | 0.0% | 0.0% | 10.0% |
| 5-HMF | 0.010 | 0.008 | 0.038 | 0.079 |

<Reference Example 7>

As shown in Table 79, with the exception of using a combination of powder acetic acid and anhydrous sodium acetate as the pH modifying component instead of the combination of anhydrous citric acid and anhydrous sodium citrate, evaluations were performed in the same manner as Reference Example 1. The results are shown in Tables 80 and 81. The powder acetic acid was obtained by mixing anhydrous sodium acetate and glacial acetic acid in a molar ratio of 1:1.

[Table 79]

Table 79 (Blend amounts in Reference Example 7)

| | Sample 25-1 | Sample 25-2 | Sample 25-3 | Sample 25-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Magnesium chloride hexahydrate | 32.0g | 32.0g | 32.0g | 32.0g |
| Powder acetic acid | 99.4g | 99.4g | 99.4g | 99.4g |
| Anhydrous sodium acetate | 149.4g | 149.4g | 149.4g | 149.4g |
| Anhydrous glucose | 315.Og | 315.Og | 315.Og | 315.Og |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 80]

Table 80 (Amounts of water of adhesion in Reference Example 7)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.80g | 0.20g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Powder acetic acid | 0.21% | 0.00% | 0.21% | 99.40g | 0.21g |
| Anhydrous sodium acetate | 0.21% | 0.00% | 0.21% | 149.40g | 0.31g |
| Anhydrous glucose | 0.05% | 0.00% | 0.05% | 315.00g | 0.16g |
|  |  |  |  | Water content due to water of adhesion | Total amount of water of adhesion |
|  |  |  |  | 0.04% | 1.13g |

[Table 81]

Table 81 (C value, consolidation, and 5-HMF in Reference Example 7)

|  | Sample 25-1 | Sample 25-2 | Sample 25-3 | Sample 25-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2665.6g | 2674.3g | 2677.4g | 2679.8g |
| Amount of water of adhesion | 1.13g | 1.13g | 1.13g | 1.13g |
| Weight of efflorescent components |  |  |  |  |
| Theoretical water content of efflorescent components |  |  |  |  |
| Amount of water of crystallization | 0.00g | 0.00g | 0.00g | 0.00g |
| Water content of powder dialysis agent | 1.13g | 1.13g | 1.13g | 1.13g |
| C value | 7% | 15% | 24% | 50% |
| Weight of consolidation | 0.0g | 0.0g | 0.0g | 0.0g |
| Consolidation rate | 0.0% | 0.0% | 0.0% | 0.0% |
| 5-HMF | 0.004 | 0.003 | 0.004 | 0.004 |

<Reference Example 8>

As shown in Table 82, with the exception of using a combination of powder acetic acid and sodium acetate trihydrate as the pH modifying component instead of the combination of anhydrous citric acid and anhydrous sodium citrate, evaluations were performed in the same manner as Reference Example 1. The results are shown in Tables 83 and 84.

[Table 82]

Table 82 (Blend amounts in Reference Example 8)

|  | Sample 26-1 | Sample 26-2 | Sample 26-3 | Sample 26-4 |
|---|---|---|---|---|
| Sodium chloride | 1969.8g | 1969.8g | 1969.8g | 1969.8g |
| Potassium chloride | 47.0g | 47.0g | 47.0g | 47.0g |
| Magnesium chloride hexahydrate | 32.0g | 32.0g | 32.0g | 32.0g |
| Powder acetic acid | 99.4g | 99.4g | 99.4g | 99.4g |
| Sodium acetate trihydrate | 247.8g | 247.8g | 247.8g | 247.8g |
| Anhydrous glucose | 315.0g | 315.0g | 315.0g | 315.0g |
| Calcium chloride hydrate (1%) | 53.0g | - | - | - |
| Calcium chloride hydrate (15%) | - | 61.7g | - | - |
| Calcium chloride hydrate (19%) | - | - | 64.8g | - |
| Calcium chloride hydrate (22%) | - | - | - | 67.2g |

[Table 83]

Table 83 (Amounts of water of adhesion in Reference Example 8)

|  | Measured water content | Theoretical water content | Water content due to water of adhesion | Weight of each component | Amount of water of adhesion in each component |
|---|---|---|---|---|---|
| Sodium chloride | 0.01% | 0.00% | 0.01% | 1969.80g | 0.20g |
| Potassium chloride | 0.02% | 0.00% | 0.02% | 47.00g | 0.01g |
| Magnesium chloride hexahydrate | 53.96% | 53.20% | 0.76% | 32.00g | 0.24g |
| Powder acetic acid | 0.21% | 0.00% | 0.21% | 99.40g | 0.21g |
| Sodium acetate trihydrate | 39.89% | 39.72% | 0.17% | 247.80g | 0.42g |
| Anhydrous glucose | 0.05% | 0.00% | 0.05% | 315.00g | 0.16g |
|  |  |  |  | Water content due to water of adhesion | Total amount of water of adhesion |
|  |  |  |  | 0.05% | 1.24g |

[Table 84]

Table 84 (C value, consolidation, and 5-HMF in Reference Example 8)

|  | Sample 26-1 | Sample 26-2 | Sample 26-3 | Sample 26-4 |
|---|---|---|---|---|
| Weight of calcium chloride dihydrate | 69.5g | 69.5g | 69.5g | 69.5g |
| Theoretical water content of calcium chloride dihydrate | 24.5% | 24.5% | 24.5% | 24.5% |
| Weight of dried calcium chloride hydrate | 53.0g | 61.7g | 64.8g | 67.2g |
| Measured water content of dried calcium chloride hydrate | 1% | 15% | 19% | 22% |
| Amount of water removed from calcium chloride hydrate | 16.50g | 7.77g | 4.72g | 2.24g |
| Total weight of all components | 2764.0g | 2772.7g | 2775.8g | 2778.2g |
| Amount of water of adhesion | 1.24g | 1.24g | 1.24g | 1.24g |
| Weight of efflorescent component (sodium acetate trihydrate) | 247.8g | 247.8g | 247.8g | 247.8g |
| Theoretical water content of efflorescent component (sodium acetate trihydrate) | 39.7% | 39.7% | 39.7% | 39.7% |
| Amount of water of crystallization | 98.38g | 98.38g | 98.38g | 98.38g |
| Water content of powder dialysis agent | 99.61g | 99.61g | 99.61g | 99.61g |
| C value | 604% | 1282% | 2110% | 4447% |
| Weight of consolidation | 795.6g | 764.3g | 810.5g | 825.7g |
| Consolidation rate | 28.8% | 27.6% | 29.2% | 29.7% |
| 5-HMF | 0.004 | 0.003 | 0.004 | 0.003 |

The concentration of each component when each sample was dissolved in RO water and made up to 315 L is shown in Table 85.

[Table 85]

Table 85 (Concentration when each sample was dissolved in RO water and made up to 315 L)

|  | Reference Examples 1 to 4 | Reference Example 5 | Reference Example 6 | Reference Examples 7 and 8 |
|---|---|---|---|---|
| Na+ | 105.3mEq/L | 105.3mEq/L | 105.3mEq/L | 115.0mEq/L |
| K+ | 2.0mEq/L | 2.0mEq/L | 2.0mEq/L | 2.0mEq/L |
| Ca++ | 3.0mEq/L | 1.25mEq/L | 3.5mEq/L | 3.0mEq/L |
| Mg++ | 1.0mEq/L | 1.0mEq/L | 1.0mEq/L | 1.0mEq/L |
| Cl- | 111.0mEq/L | 109.3mEq/L | 111.5mEq/L | 113.0mEq/L |
| Citrate ion | 2.0mEq/L | 2.0mEq/L | 2.0mEq/L | 0.0mEq/L |
| Acetate ion | 0.0mEq/L | 0.0mEq/L | 0.0mEq/L | 10.2mEq/L |
| Anhydrous glucose | 150.0mg/dl | 150.0mg/dl | 150.0mg/dl | 100.0mg/dl |

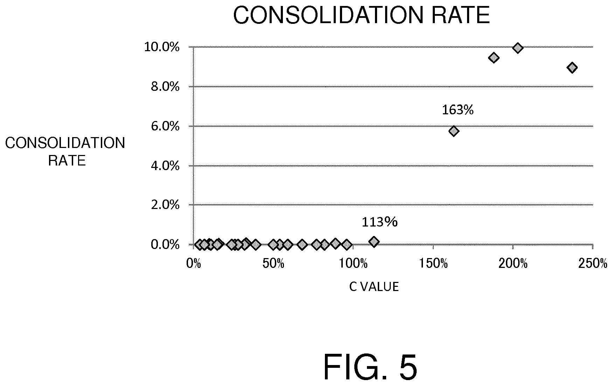

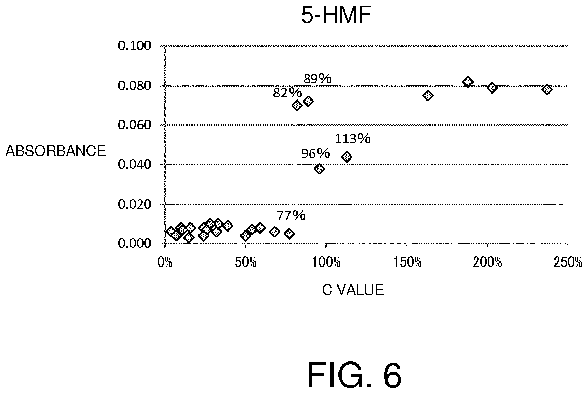

Combining the above results, the relationship between the ratio C and the consolidation rate is shown in FIG. 5, and the relationship between the ratio C and the 5-HMF absorbance is shown in FIG. 6.

In this manner, by ensuring that the ratio C between the water content of the entire dialysis agent and the amount of water removed from the calcium chloride hydrate was less than 163% but at least 2%, consolidation and aggregation of the chemical agent were suppressed in the stability test. Further, by ensuring that the ratio between the water content of the entire dialysis agent and the amount of water removed from the calcium chloride hydrate was less than 82% but at least 2%, production of 5-HMF was inhibited in the stability test.

<Reference Example 9>

Using calcium chloride hydrate ($CaCl_2$) (water content: 15%) that had been subjected to a drying step to provide improved functionality as a desiccant, sodium chloride, potassium chloride, magnesium chloride hexahydrate, anhydrous glucose, the calcium chloride hydrate, and a pH modifier (any one of an acetic acid mixture (1.5:1.5), an acetic acid mixture (3:3), an acetic acid mixture (6:2), an acetic acid mixture (8:2.2), an acetic acid mixture (10:2), or a combination of anhydrous sodium acetate and glacial acetic acid) were placed in a container in a ratio shown in Table 86, the container was sealed, and the container contents were dispersed to complete preparation of a series of dialysis agents (samples 27-1 to 27-6).

The amount of water of adhesion in each of the sodium chloride, the potassium chloride, the magnesium chloride hexahydrate, the anhydrous glucose and the pH modifier was measured using the water content measurement method described above to determine the amount of water of adhesion. The results are shown in Table 87.

A stability test (40°C, 75% RH, 14 days) was performed for each of the above dialysis agents, and the existence of consolidation was investigated and evaluated using the method described above. Further, following the stability test, each dialysis agent was dissolved in RO water to prepare a dialysis agent A concentrate. In order to measure the rate of degradation of the anhydrous glucose in the dialysis agent A concentrate, the 5-HMF absorbance was measured using the method described above. The results are shown in Table 88. Further, the results of calculating the amount of water removed from the calcium chloride hydrate and the water content of the entire dialysis agent, and then calculating the ratio C are shown in Table 88.

[Table 86]

Table 86 (Blend amounts in Reference Example 9)

| | Sample 27-1 | Sample 27-2 | Sample 27-3 | Sample 27-4 | Sample 27-5 | Sample 27-6 |
|---|---|---|---|---|---|---|
| Sodium chloride | 1969.8g | 1969.8g | 1969.8g | 1969.8g | 1969.8g | 1969.8g |
| Potassium chloride | 47.0g | 47.0g | 47.0g | 47.0g | 47.0g | 47.0g |
| Magnesium chloride hexahydrate | 32.0g | 32.0g | 32.0g | 32.0g | 32.0g | 32.0g |
| Acetic acid mixture (1.5:1.5) | 67.1g | - | - | - | - | - |
| Acetic acid mixture (3:3) | - | 134.2g | - | - | - | - |
| Acetic acid mixture (6:2) | - | - | 192.6g | - | - | - |
| Acetic acid mixture (8:2.2) | - | - | - | 248.7g | | - |
| Acetic acid mixture (10:2) | | - | - | - | 296.2g | - |
| Glacial acetic acid | - | - | - | - | - | 42.0g |
| Anhydrous sodium acetate | - | - | - | - | - | 206.7g |
| Anhydrous glucose | 315.0g | 315.0g | 315.0g | 315.0g | 315.0g | 315.0g |

(continued)

| | Sample 27-1 | Sample 27-2 | Sample 27-3 | Sample 27-4 | Sample 27-5 | Sample 27-6 |
|---|---|---|---|---|---|---|
| Calcium chloride hydrate (15%) | 61.7g | 61.7g | 61.7g | 61.7g | 61.7g | 61.7g |

[Table 87]

Table 87 (Amounts of water of adhesion in Reference Example 9)

| | Measured water content | Theoretical water content | Water content due to water of adhesion | Weight of each component | Amount of water of adhesion in each component |
|---|---|---|---|---|---|
| Sodium chloride | 0.03% | 0.00% | 0.03% | 1969.8g | 0.59g |
| Potassium chloride | 0.02% | 0.00% | 0.02% | 47.0g | 0.01g |
| Magnesium chloride hexahydrate | 53.96% | 53.20% | 0.76% | 32.00g | 0.24g |
| Acetic acid mixture (1.5:1.5) | 0.21% | 0.00% | 0.21% | 67.1g | 0.14g |
| Acetic acid mixture (3:3) | 0.21% | 0.00% | 0.21% | 134.2g | 0.28g |
| Acetic acid mixture (6:2) | 0.19% | 0.00% | 0.19% | 192.6g | 0.37g |
| Acetic acid mixture (8:2.2) | 0.18% | 0.00% | 0.18% | 248.7g | 0.45g |
| Acetic acid mixture (10:2) | 0.16% | 0.00% | 0.16% | 296.2g | 0.47g |
| Glacial acetic acid | 0.07% | 0.00% | 0.07% | 42.0g | 0.03g |
| Anhydrous sodium acetate | 0.21% | 0.00% | 0.21% | 206.7g | 0.43g |
| Anhydrous glucose | 0.05% | 0.00% | 0.05% | 315.Og | 0.16g |

[Table 88]

Table 88 (C value, consolidation, and 5-HMF in Reference Example 9)

| | Sample 27-1 | Sample 27-2 | Sample 27-3 | Sample 27-4 | Sample 27-5 | Sample 27-6 |
|---|---|---|---|---|---|---|
| Weight of calcium chloride dihydrate | 69.5g | 69.5g | 69.5g | 69.5g | 69.5g | 69.5g |
| Theoretical water content of calcium chloride dihydrate | 24.5% | 24.5% | 24.5% | 24.5% | 24.5% | 24.5% |

(continued)

|  | Sample 27-1 | Sample 27-2 | Sample 27-3 | Sample 27-4 | Sample 27-5 | Sample 27-6 |
|---|---|---|---|---|---|---|
| Weight of dried calcium chloride hydrate | 61.7g | 61.7g | 61.7g | 61.7g | 61.7g | 61.7g |
| Measured water content of dried calcium chloride hydrate | 15% | 15% | 15% | 15% | 15% | 15% |
| Amount of water removed from calcium chloride hydrate | 7.77g | 7.77g | 7.77g | 7.77g | 7.77g | 7.77g |
| Total weight of all components | 2460.6g | 2527.7g | 2586.1g | 2642.2g | 2689.7g | 2642.2g |
| Amount of water of adhesion | 1.14g | 1.28g | 1.37g | 1.45g | 1.47g | 1.46g |
| Weight of efflorescent components | - | - | - | - | - | - |
| Theoretical water content of efflorescent components | - | - | - | - | - | - |
| Amount of water of crystallization | 0.00g | 0.00g | 0.00g | 0.00g | 0.00g | 0.00g |
| Water content of powder dialysis agent | 1.14g | 1.28g | 1.37g | 1.45g | 1.47g | 1.46g |
| C value | 15% | 16% | 18% | 19% | 19% | 19% |
| Weight of consolidation | 1.0g | 1.1g | 1.3g | 1.3g | 1.2g | 453.6g |
| Consolidation rate | 0.0% | 0.0% | 0.0% | 0.0% | 0.0% | 17.2% |
| 5-HMF | 0.005 | 0.005 | 0.005 | 0.005 | 0.005 | 0.083 |

In this manner, by using an acetic acid mixture composed of a mixture of anhydrous sodium acetate and glacial acetic acid as the pH modifier, rather than a simple combination of anhydrous sodium acetate and glacial acetic, consolidation and aggregation of the chemical agent were suppressed in the stability test. Further, production of 5-HMF was inhibited in the stability test. In Sample 27-6, in which a dialysis agent was produced using anhydrous sodium acetate and glacial acetic acid as pH modifiers, significantly more consolidation of the chemical agent and degradation of the anhydrous glucose occurred compared with the case where an acetic acid mixture was used.

The concentration of each component when Samples 27-1 to 27-6 were each dissolved in RO water and made up to 315 L is shown in Table 89.

[Table 89]

Table 89 (Concentration when each sample was dissolved in RO water and made up to 315 L)

|  | Sample 27-1 | Sample 27-2 | Sample 27-3 | Sample 27-4 | Sample 27-5 | Sample 27-6 |
|---|---|---|---|---|---|---|
| Na+ | 108.5mEq/L | 110.0mEq/L | 113.0mEq/L | 115.0mEq/L | 117.0mEq/L | 115.0mEq/L |
| K+ | 2.0mEq/L | 2.0mEq/L | 2.0mEq/L | 2.0mEq/L | 2.0mEq/L | 2.0mEq/L |
| Ca++ | 3.0mEq/L | 3.0mEq/L | 3.0mEq/L | 3.0mEq/L | 3.0mEq/L | 3.0mEq/L |
| Mg++ | 1.0mEq/L | 1.0mEq/L | 1.0mEq/L | 1.0mEq/L | 1.0mEq/L | 1.0mEq/L |

(continued)

|  | Sample 27-1 | Sample 27-2 | Sample 27-3 | Sample 27-4 | Sample 27-5 | Sample 27-6 |
|---|---|---|---|---|---|---|
| Cl- | 113.0mEq/L | 113.0mEq/L | 113.0mEq/L | 113.0mEq/L | 113.0mEq/L | 113.0mEq/L |
| Acetate ion | 3.0mEq/L | 6.0mEq/L | 8.0mEq/L | 10.2mEq/L | 12.0mEq/L | 10.2mEq/L |
| Anhydrous glucose | 100.0mg/dl | 100.0mg/dl | 100.0mg/dl | 100.0mg/dl | 100.0mg/dl | 100.0mg/dl |

**Claims**

1. A method for providing a dialysis agent consisting of:

- electrolytes containing magnesium chloride hydrate and optionally comprising calcium chloride hydrate,
- at least one pH modifier selected from the group consisting of citric acid, sodium citrate, an acetic acid mixture composed of a mixture of sodium acetate and glacial acetic acid, and a combination of this acetic acid mixture and sodium acetate, wherein the citric acid can be anhydrous or a monohydrate and wherein the sodium citrate can be anhydrous or a dihydrate and
- optionally at least one compound selected from sodium chloride, anhydrous glucose and potassium chloride, said method comprising:

a drying treatment step of heat drying said magnesium chloride hydrate in an atmosphere of 75°C or higher separately from other components, until a ratio between water content of the entire dialysis agent and an amount of water removed from said magnesium chloride hydrate, expressed by the following formula:

$$M(\%)=[(\text{water content of entire dialysis agent (g))} / (\text{amount of water removed from magnesium chloride hydrate (g))}] * 100,$$

becomes less than 118% but at least 7%,
and water content of said magnesium chloride hydrate becomes a value within a range from 43 mass% to 51 mass%,
wherein the amount of water removed from the magnesium chloride hydrate is represented by the difference between the initial amount of retained water (g) in the magnesium chloride hydrate and the amount of retained water (g) following drying of the magnesium chloride hydrate, the difference between the initial amount of retained water (g) in the magnesium chloride hydrate, i.e. [weight of magnesium chloride hexahydrate] × [theoretical water content of magnesium chloride hexahydrate (53.2%)] and the amount of retained water (g) following drying of the magnesium chloride hydrate, i.e. the [weight of dried magnesium chloride hydrate] × [measured water content (%) of dried magnesium chloride hydrate], wherein the water content of the entire dialysis agent is determined from the sum of the "amount of water of adhesion" within the raw materials and the "amount of water of crystallization",
wherein the total amount of water of adhesion is calculated from the measured water content, the theoretical water content, and the weight of each component except for the dried magnesium chloride hydrate as follows:

$$\text{Measured water content of each component (\%)} - \text{theoretical water content of each component (\%)} = \text{water content due to water of adhesion of each component (\%)}$$

$$\text{Weight of each component (g)} \times \text{water content due to water of adhesion of each component (\%)} = \text{amount of water of adhesion for each component,}$$

$$\text{Total of amounts of water of adhesion for all components (g)} = \text{total amount of water of adhesion (amount of water of adhesion) (g),}$$

wherein the measurements of the water content of sodium chloride, anhydrous glucose, potassium chloride, citric acid, sodium citrate and sodium acetate are performed in accordance with the water content measurement method disclosed in the 16th edition of The Japanese Pharmacopoeia,

wherein the measurements of the water content of calcium chloride hydrate, magnesium chloride hydrate and powder acetic acid are performed in an atmosphere at a room temperature of 25°C±2.5°C and a relative humidity of 40%±2.5% in accordance with the Karl Fischer titration method, volumetric titration method and direct titration of JIS K0068 using a Karl Fischer measuring device, wherein the measurement is performed using 30 ml of methanol as the solvent, wherein a Karl Fischer reagent HYDRANAL-Composite 5 is used as the reagent for measuring the water content,

wherein the weight of the water of crystallization, which corresponds to the amount of water of crystallization within efflorescent components, is calculated from the theoretical water content of the efflorescent components and the weight of the efflorescent components as follows:

$$\text{Amount of water of crystallization (g)} = \text{weight of efflorescent components (g)} \times \text{theoretical water content of efflorescent components (\%)}$$

and a filling step of placing each constituent component in a container as a single solid component.

2. The method for providing a dialysis agent according to Claim 1, wherein in said drying treatment step, said magnesium chloride hydrate is dried in an atmosphere of 75°C or higher until said ratio between the water content of the entire dialysis agent and the amount of water removed from the magnesium chloride hydrate becomes less than 97% but at least 7%, wherein the water content is measured in an atmosphere at a room temperature of 25°C±2.5°C and a relative humidity of 40%±2.5% in accordance with the Karl Fischer titration method, volumetric titration method and direct titration of JIS K0068 using a Karl Fischer measuring device, wherein the measurement is performed using 30 ml of methanol as the solvent, wherein a Karl Fischer reagent HYDRANAL-Composite 5 is used as the reagent for measuring the water content.

3. The method for providing a dialysis agent according to Claim 2, wherein in said drying treatment step, said magnesium chloride hydrate is dried in an atmosphere of 75°C or higher until said water content of the magnesium chloride hydrate, measured in an atmosphere at a room temperature of 25°C±2.5°C and a relative humidity of 40%±2.5% in accordance with the Karl Fischer titration method, volumetric titration method and direct titration of JIS K0068 using a Karl Fischer measuring device, wherein the measurement is performed using 30 ml of methanol as the solvent, wherein a Karl Fischer reagent HYDRANAL-Composite 5 is used as the reagent for measuring the water content, becomes a value within a range from 43 mass% to 45 mass%.

4. The method for providing a dialysis agent according to Claim 2 or 3, wherein said pH modifier is at least one of an acetic acid mixture composed of a mixture of sodium acetate and glacial acetic acid, and a combination of the acetic acid mixture and sodium acetate.

5. The method for providing a dialysis agent according to Claim 4, wherein a molar ratio between sodium acetate and glacial acetic acid in said acetic acid mixture is within a range from 1:1 to 5:1.

**Patentansprüche**

1. Verfahren zum Bereitstellen eines Dialyseagens bestehend aus:

   - Elektrolyten, enthaltend Magnesiumchloridhydrat und optional umfassend Calciumchloridhydrat,
   - mindestens einem pH-Wert Modifizierer, ausgewählt aus der Gruppe bestehend aus Zitronensäure, Natriumcitrat, einer Essigsäuremischung gebildet aus einer Mischung aus Natriumacetat und Eisessigsäure, und einer Kombination aus dieser Essigsäuremischung und Natriumacetat, wobei die Zitronensäure wasserfrei oder ein Monohydrat sein kann und wobei das Natriumcitrat wasserfrei oder ein Dihydrat sein kann und
   - optional mindestens einer Verbindung, ausgewählt aus Natriumchlorid, wasserfreier Glucose und Kaliumchlorid, das Verfahren umfassend:
   einen Trocknungsbehandlungsschritt des Wärmetrocknens des Magnesiumchloridhydrats in einer Atmosphäre von 75°C oder höher, separat von anderen Komponenten, bis ein Verhältnis zwischen dem Wassergehalt des gesamten Dialyseagens und der Menge an Wasser, das von dem Magnesiumchloridhydrat entfernt wurde, durch folgende Formel ausgedrückt:

$$M(\%) = [(\text{Wassergehalt des gesamten Dialyseagens (g))} / (\text{Menge an Wasser, das von dem Magnesiumchloridhydrat entfernt wurde (g))}] * 100,$$

weniger als 118% aber mindestens 7% wird,
und der Wassergehalt des Magnesiumchloridhydrats einen Wert in einem Bereich von 43 Masse% bis 51 Masse% erreicht,
wobei die Menge an Wasser, das von dem Magnesiumchloridhydrat entfernt wurde, durch die Differenz zwischen der initial in dem Magnesiumchloridhydrat enthaltenen Menge an Wasser (g) und der Menge an enthaltenem Wasser (g) nach erfolgtem Trocknen des Magnesiumchloridhydrats, der Differenz zwischen der initial in dem Magnesiumchloridhydrat enthaltenen Menge an Wasser (g), d.h. [Gewicht des Magnesiumchloridhexahydrats] × [theoretischer Wassergehalt des Magnesiumchloridhexahydrats (53.2%)] und der Menge an enthaltenem Wasser (g) nach erfolgtem Trocknen des Magnesiumchloridhydrats, d.h. das [Gewicht des getrockneten Magnesiumchloridhydrats] x [gemessener Wassergehalt (%) des getrockneten Magnesiumchloridhydrats], dargestellt wird,
wobei der Wassergehalt des gesamten Dialyseagens aus der Summe der "Menge an adhäsiv gebundenem Wasser" in den Rohmaterialien und der "Menge an Kristallwasser" bestimmt wird,
wobei die Gesamtmenge an adhäsiv gebundenem Wasser aus dem gemessenen Wassergehalt, dem theoretischen Wassergehalt und dem Gewicht jeder Komponente mit Ausnahme des getrockneten Magnesiumchloridhydrats wie folgt bestimmt wird:

$$\text{Gemessener Wassergehalt jeder Komponente (\%) - theoretischer Wassergehalt jeder Komponente (\%)} = \text{Wassergehalt bedingt durch adhäsiv gebundenes Wasser einer jeden Komponente (\%)}$$

$$\text{Gewicht jeder Komponente (g)} \times \text{Wassergehalt bedingt durch adhäsiv gebundenes Wasser einer jeden Komponente (\%)} = \text{Menge an adhäsiv gebundenem Wasser einer jeden Komponente,}$$

$$\text{Gesamtmengen an adhäsiv gebundenem Wasser aller Komponenten (g)} = \text{Gesamtmenge an adhäsiv gebundenem Wasser (Menge an adhäsiv gebundenem Wasser) (g),}$$

wobei die Messungen des Wassergehalts des Natriumchlorids, der wasserfreien Glucose, des Kaliumchlorids, der Zitronensäure, des Natriumcitrats und des Natriumacetats im Einklang mit den Wassergehaltsmessmethoden, offenbart in der 16. Edition der Japanischen Pharmakopöe ausgeführt werden,
wobei die Messungen des Wassergehalts des Calciumchloridhydrats, des Magnesiumchloridhydrats und der pulverförmigen Essigsäure in einer Atmosphäre bei Raumtemperatur von 25°C±2,5°C und einer relativen Feuchte von 40%±2,5% im Einklang mit dem Karl Fischer Titrationsverfahren, einem volumetrischen Titrationsverfahren und einer direkten Titration gemäß JIS K0068 unter Verwendung einer Karl Fischer Messvorrichtung ausgeführt werden, wobei die Messung unter Verwendung von 30 ml Methanol, als Lösungsmittel ausgeführt wird, wobei ein Karl Fischer Reagens HYDRANAL-Komposit 5 als das Reagens zur Messung des Wassergehalts verwendet wird,
wobei das Gewicht des Kristallwassers, das der Menge an Kristallwasser in effloreszierenden Komponenten entspricht, aus dem theoretischen Wassergehalt der effloreszierenden Komponenten und dem Gewicht der effloreszierenden Komponenten wie folgt ermittelt wird:

$$\text{Menge an Kristallwasser (g)} = \text{Gewicht an effloreszierenden Komponenten (g)} \times$$

$$\text{theoretischer Wassergehalt der effloreszierenden Komponenten (\%)}$$

und einen Abfüllschritt des Platzierens jeder Bestandteilskomponente in einem Container als Einzelfeststoffkomponente.

**2.** Verfahren zur Bereitstellung eines Dialyseagens nach Anspruch 1, wobei in dem Trocknungsbehandlungsschritt das Magnesiumchloridhydrat in einer Atmosphäre von 75°C oder höher getrocknet wird, bis das Verhältnis zwischen dem Wassergehalt des gesamten Dialyseagens und der Menge an Wasser, die von dem Magnesiumchloridhydrat entfernt wurde, weniger als 97% aber mindestens 7% beträgt, wobei der Wassergehalt in einer Atmosphäre bei Raumtemperatur von 25°C±2,5°C und einer relativen Feuchte von 40%±2,5% im Einklang mit dem Karl Fischer Titrationsverfahren, einem volumetrischen Titrationsverfahren und einer direkten Titration gemäß JIS K0068 unter Verwendung einer Karl Fischer Messvorrichtung gemessen wird, wobei die Messung unter Verwendung von 30 ml Methanol, als Lösungsmittel ausgeführt wird, wobei ein Karl Fischer Reagens HYDRANAL-Komposit 5 als das Reagens zur Messung des Wassergehalts verwendet wird.

**3.** Verfahren zur Bereitstellung eines Dialyseagens nach Anspruch 2, wobei in dem Trocknungsbehandlungsschritt das Magnesiumchloridhydrat in einer Atmosphäre von 75°C oder höher getrocknet wird, bis der Wassergehalt des Magnesiumchloridhydrats, gemessen in einer Atmosphäre bei Raumtemperatur von 25°C±2,5°C und einer relativen Feuchte von 40%±2,5% im Einklang mit dem Karl Fischer Titrationsverfahren, einem volumetrischen Titrationsverfahren und einer direkten Titration gemäß JIS K0068 unter Verwendung einer Karl Fischer Messvorrichtung, wobei die Messung unter Verwendung von 30 ml Methanol, als Lösungsmittel ausgeführt wird, wobei ein Karl Fischer Reagens HYDRANAL-Komposit 5 als das Reagens zur Messung des Wassergehalts verwendet wird, einen Wert in einem Bereich von 43 Masse% bis 45 Masse% erlangt.

**4.** Verfahren zur Bereitstellung eines Dialyseagens nach Anspruch 2 oder 3, wobei der pH-Wert Modifizierer mindestens einer aus einer Essigsäuremischung, gebildet aus einer Mischung von Natriumacetat und Eisessigsäure, und einer Kombination der Essigsäuremischung und Natriumacetat ist.

**5.** Verfahren zur Bereitstellung eines Dialyseagens nach Anspruch 4, wobei ein molares Verhältnis zwischen dem Natriumacetat und der Eisessigsäure in der Essigsäuremischung in einem Bereich von 1:1 bis 5:1 liegt.

**Revendications**

**1.** Procédé pour produire un agent de dialyse qui est constitué par :

- des électrolytes qui contiennent de l'hydrate de chlorure de magnésium et en option, qui comprennent de l'hydrate de chlorure de calcium ;
- au moins un modificateur de pH qui est sélectionné parmi le groupe qui est constitué par l'acide citrique, le citrate de sodium, un mélange d'acide acétique qui est composé d'un mélange d'acétate de sodium et d'acide acétique glacial, et une combinaison de ce mélange d'acide acétique et d'acétate de sodium, dans lequel l'acide citrique peut être anhydre ou un monohydrate et dans lequel le citrate de sodium peut être anhydre ou un dihydrate ; et
- en option, au moins un composé qui est sélectionné parmi le chlorure de sodium, le glucose anhydre et le chlorure de potassium, ledit procédé comprenant :

une étape de traitement par séchage qui consiste à sécher à la chaleur ledit hydrate de chlorure de magnésium dans une atmosphère de 75 °C ou au-delà séparément des autres composants, jusqu'à ce qu'un rapport entre la teneur en eau de l'agent de dialyse complet et une quantité d'eau enlevée dudit hydrate de chlorure de magnésium, lequel rapport est exprimé au moyen de la formule qui suit :

$$M(\%) = [(\text{teneur en eau de l'agent de dialyse complet (g)})/(\text{quantité d'eau enlevée de l'hydrate de chlorure de magnésium (g)})] * 100$$

devienne inférieur à 118 % mais au moins égal à 7 %,
et jusqu'à ce que la teneur en eau dudit hydrate de chlorure de magnésium devienne égale à une valeur à l'intérieur d'une plage qui va de 43 % en masse à 51 % en masse,
dans lequel la quantité d'eau enlevée de l'hydrate de chlorure de magnésium est représentée par la différence entre la quantité initiale d'eau retenue (g) dans l'hydrate de chlorure de magnésium et la quantité d'eau retenue (g) suite au séchage de l'hydrate de chlorure de magnésium, la différence entre la quantité initiale d'eau retenue (g) dans l'hydrate de chlorure de magnésium étant [poids d'hexahydrate de chlorure de magnésium] x [teneur en eau théorique de l'hexahydrate de chlorure de magnésium (53,2 %)] et la quantité d'eau retenue (g) suite au séchage de l'hydrate de chlorure de magnésium étant [poids d'hydrate de chlorure de magnésium séché] x [teneur en eau mesurée (%) de l'hydrate de chlorure de magnésium séché] ; dans lequel
la teneur en eau de l'agent de dialyse complet est déterminée à partir de la somme de la "quantité d'eau d'adhérence" à l'intérieur des matériaux bruts et de la "quantité d'eau de cristallisation" ; dans lequel
la quantité totale d'eau d'adhérence est calculée à partir de la teneur en eau mesurée, de la teneur en eau théorique et du poids de chaque composant à l'exception de l'hydrate de chlorure de magnésium séché comme suit :

$$\text{teneur en eau mesurée de chaque composant (\%) - teneur en eau théorique de chaque composant (\%) = teneur en eau due à l'eau d'adhérence de chaque composant (\%)}$$

$$\text{poids de chaque composant (g) x teneur en eau due à l'eau d'adhérence de chaque composant (\%) = quantité d'eau d'adhérence pour chaque composant,}$$

$$\text{total des quantités d'eau d'adhérence pour tous les composants (g) = quantité totale d'eau d'adhérence (quantité d'eau d'adhérence) (g) ;}$$

dans lequel les mesures de la teneur en eau du chlorure de sodium, du glucose anhydre, du chlorure de potassium, de l'acide citrique, du citrate de sodium et de l'acétate de sodium sont réalisées conformément au procédé de mesure de teneur en eau décrit dans la 16ème édition de The Japanese Pharmacopoeia ;
dans lequel les mesures de la teneur en eau de l'hydrate de chlorure de calcium, de l'hydrate de chlorure de magnésium et de l'acide acétique en poudre sont réalisées dans une atmosphère à une température ambiante de 25 °C ± 2,5 °C et à une humidité relative de 40 % ± 2,5 % conformément au procédé de titrage de Karl Fischer, au procédé de titrage volumétrique et au titrage direct de JIS K0068 en utilisant un dispositif de mesure de Karl Fischer;
dans lequel la mesure est réalisée en utilisant 30 ml de méthanol en tant que solvant, dans lequel un réactif de Karl Fischer HYDRANAL- Composite 5 est utilisé en tant que réactif pour mesurer la teneur en eau ; et
dans lequel le poids de l'eau de cristallisation, qui correspond à la quantité d'eau de cristallisation à l'intérieur de composants efflorescents, est calculé à partir de la teneur en eau théorique des composants efflorescents et du poids des composants efflorescents comme suit :

$$\text{quantité d'eau de cristallisation (g) = poids des composants efflorescents (g) x teneur en eau théorique des composants efflorescents (\%)}$$

et une étape de remplissage qui consiste à placer chaque composant constitutif dans un contenant en tant qu'unique composant solide.

2. Procédé pour produire un agent de dialyse selon la revendication 1, dans lequel, au niveau de ladite étape de traitement par séchage, ledit hydrate de chlorure de magnésium est séché dans une atmosphère de 75 °C ou au-delà jusqu'à ce que ledit rapport entre la teneur en eau de l'agent de dialyse complet et la quantité d'eau enlevée de l'hydrate de chlorure de magnésium devienne inférieur à 97 % mais égal à au moins 7 %, dans lequel la teneur en eau est mesurée dans une atmosphère à une température ambiante de 25 °C $\pm$ 2,5 °C et à une humidité relative de 40 % $\pm$ 2,5 % conformément au procédé de titrage de Karl Fischer, au procédé de titrage volumétrique et au titrage direct de JIS K0068 en utilisant un dispositif de mesure de Karl Fischer, et dans lequel la mesure est réalisée en utilisant 30 ml de méthanol en tant que solvant, dans lequel un réactif de Karl Fischer HYDRANAL-Composite 5 est utilisé en tant que réactif pour mesurer la teneur en eau.

3. Procédé pour produire un agent de dialyse selon la revendication 2, dans lequel, au niveau de ladite étape de traitement par séchage, ledit hydrate de chlorure de magnésium est séché dans une atmosphère de 75 °C ou au-delà jusqu'à ce que ladite teneur en eau de l'hydrate de chlorure de magnésium, mesurée dans une atmosphère à une température ambiante de 25 °C $\pm$ 2,5 °C et à une humidité relative de 40 % $\pm$ 2,5 % conformément au procédé de titrage de Karl Fischer, au procédé de titrage volumétrique et au titrage direct de JIS K0068 en utilisant un dispositif de mesure de Karl Fischer, dans lequel la mesure est réalisée en utilisant 30 ml de méthanol en tant que solvant, dans lequel un réactif de Karl Fischer HYDRANAL-Composite 5 est utilisé en tant que réactif pour mesurer la teneur en eau, devienne égale à une valeur à l'intérieur d'une plage qui va de 43 % en masse à 45 % en masse.

4. Procédé pour produire un agent de dialyse selon la revendication 2 ou 3, dans lequel ledit modificateur de pH est au moins un modificateur pris parmi un mélange d'acide acétique qui est composé d'un mélange d'acétate de sodium et d'acide acétique glacial, et une combinaison du mélange d'acide acétique et d'acétate de sodium.

5. Procédé pour produire un agent de dialyse selon la revendication 4, dans lequel un rapport molaire entre l'acétate de sodium et l'acide acétique glacial dans ledit mélange d'acide acétique est à l'intérieur d'une plage qui va de 1:1 à 5:1.

CONSOLIDATION RATE

FIG. 1

FIG. 2

FIG. 3

FIG. 4

FIG. 5

FIG. 6

FIG. 7

FIG. 8

## REFERENCES CITED IN THE DESCRIPTION

**Patent documents cited in the description**

- JP H08080345 A
- JP 2001340448 A
- WO 2011161055 A
- JP 2010029654 A